# EUROPÄISCHE PATENTSCHRIFT

(11) **EP 0 934 288 B1**
(45) Veröffentlichungstag und Bekanntmachung des Hinweises auf die Patenterteilung: **04.01.2006**
(21) Anmeldenummer: 97911227.3
(22) Anmeldetag: 15.10.1997
(51) Int. Cl.: C07D 271/06, C07D 413/04, C07D 413/12, C07D 451/06, C07D 471/08, A61K 31/41, C07D 221/00, C07D 209/00

(54) **OXADIAZOLE, VERFAHREN ZU IHRER HERSTELLUNG SOWIE IHRE VERWENDUNG ALS ARZNEIMITTEL**
OXADIAZOLES, PROCESSES FOR THEIR PREPARATION AND THEIR USE AS MEDICAMENTS
OXADIAZOLES, PROCEDES PERMETTANT DE LES PREPARER ET LEUR UTILISATION COMME MEDICAMENTS

(30) Priorität: 18.10.1996 DE 19643037
(43) Veröffentlichungstag der Anmeldung: 11.08.1999
(73) Patentinhaber: Boehringer Ingelheim Pharma GmbH & Co.KG, 55218 Ingelheim am Rhein (DE); Boehringer Ingelheim International GmbH, 55218 Ingelheim am Rhein (DE)
(72) Erfinder: BRENNER, Michael, D-55411 Bingen am Rhein (DE); MAIER, Roland, D-88400 Biberach an der Riss 1 (DE); WIENRICH, Marion, D-64331 Weiterstadt (DE); WEISER, Thomas, D-55268 Nieder-Olm (DE); PALLUK, Rainer, D-55411 Bingen am Rhein (DE); BECHTEL, Wolf-Dietrich, D-55437 Appenheim (DE); SAGRADA, Angelo, I-20135 Milano (IT); ENSINGER, Helmut, D-55218 Ingelheim am Rhein (DE); PSCHORN, Uwe, D-55216 Mainz (DE); CESANA, Raffaele, I-20125 Milano (IT)
(74) Vertreter: Kläs, Heinz-Gerd
(86) Internationale Anmeldenummer: PCT/EP1997/005693
(87) Internationale Veröffentlichungsnummer: WO 1998/017652

(56) Entgegenhaltungen:
- EP-A- 0 091 726
- EP-A- 0 276 432
- EP-A- 0 384 310
- EP-A- 0 504 574
- WO-A-93/02677
- DE-A- 2 532 742
- DE-A- 4 401 107
- DE-A- 4 401 108
- DE-A- 19 528 189

## Beschreibung

Die Erfindung betrifft neue Oxadiazol-Derivate, Verfahren zu ihrer Herstellung sowie deren Verwendung als Arzneimittel.

Die neuen Oxadiazol-Derivate besitzen die Struktur der allgemeinen Formel (I) 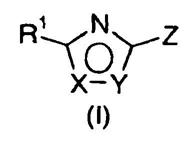
worin
- X und Y: Sauerstoff oder Stickstoff, wobei X und Y nicht beide gleichzeitig Sauerstoff oder Stickstoff sind,
- Z: ein Rest der Formel

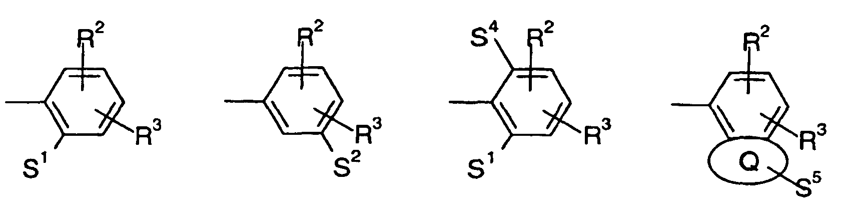

worin
- S¹: ein Rest der Formel 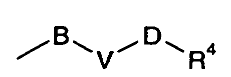 worin V Sauerstoff, Schwefel oder NR⁷ bedeutet und B und D, gleich oder verschieden, eine C₁-C₄-Alkylen-, C₂-C₄-Alkenylen- oder C₂-C₄-Alkinylen-Brücke repräsentieren, die durch =O, -OR⁷, Phenyl oder Halogen, bevorzugt Fluor, Chlor oder Brom substituiert sein kann,
- S¹: ein Rest der Formel 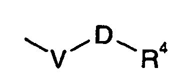 wobei V und D die oben genannte Bedeutung aufweisen,
- S¹: ein Rest der Formel 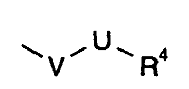 worin V die zuvor genannte Bedeutung aufweisen kann und U eine C₃-C₆-Cycloalkyl- oder Phenyl-Gruppe repräsentiert, die durch C₁-C₄-Alkyl, -OR⁷, C₆-C₁₀-Aryl oder Halogen, bevorzugt Fluor, Chlor oder Brom substituiert sein kann,
- S¹: ein Rest der Formel 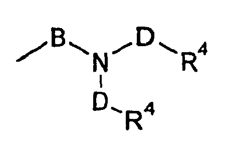
wobei B und D die oben genannte Bedeutung aufweisen und die beiden Gruppen D sowie die beiden Reste R⁴ gleich oder verschieden sind,
- S¹: ein Rest der Formel 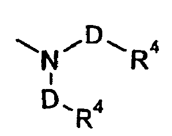 wobei D die oben genannte Bedeutung aufweist und die beiden Gruppen D sowie die beiden Reste R⁴ gleich oder verschieden sind,
- S¹: ein Rest der Formel 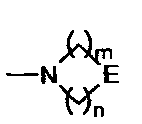 in dem E Sauerstoff, Schwefel oder NR⁷ darstellt (mit n,m=1,2 oder 3 und n+m>2) und der Rest gegebenenfalls substituiert ist durch Halogen, bevorzugt Fluor, Chlor oder Brom, = O, -OR⁷ oder einen oder mehrere C₁-C₄-Alkyl-Reste;
- S¹: ein Rest der Formel 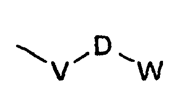 wobei V und D die oben genannte Bedeutung aufweisen und W ein gegebenenfalls durch Halogen, = O, -OR⁷, -OCOR⁷, C₁-C₄-Alkyl, C₂-C₆-Alkenyl oder C₂-C₆-Alkinyl substituierter Rest der Formel 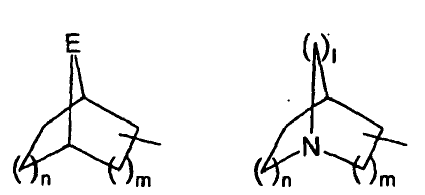 sein kann, wobei E Sauerstoff oder NR⁷ bedeutet, n,m, I = 0,1 oder 2 sein können, oder W ein C-verknüpfter 5 oder 6-gliedriger Heterocyclus ist, der ein oder mehrere Heteroatome aus der Gruppe Stickstoff, Sauerstoff oder Schwefel enthält und der gegebenenfalls durch Benzyl oder C₁-C₄-Alkyl substituiert sein kann;
- S¹: ein Rest der Formel

―V―W

wobei V und W die oben angegebenen Bedeutung aufweisen;
- S²: ein Rest der Formel 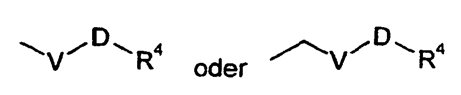 wobei V und D die oben genannte Bedeutung aufweisen,
- S⁴: ein Rest der Formel 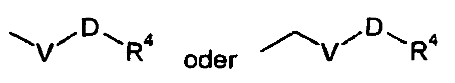 wobei V und D die oben genannte Bedeutung aufweisen,
- Q: ein ankondensierter, einfach oder mehrfach ungesättigter 5 oder 6- gliedriger, heterocyclischer Ring, der ein oder mehrere Heteroatome aus der Gruppe Sauerstoff, Stickstoff oder Schwefel enthalten kann und gegebenenfalls durch OR⁷, NR⁵R⁶, Halogen, CN, Nitro, CF₃, COOR⁷, C₁-C₄-Alkyl, C₂-C₄-Alkenyl oder C₂-C₄-Alkinyl substituiert sein kann;
- S⁵: ein Rest der Formel

―D―R⁴

wobei D die oben genannte Bedeutung aufweist;
- R¹: Benzyl oder Phenyl, wobei der Phenylring ein oder mehrfach durch einen oder mehrere der Reste Fluor, Chlor oder Brom, -C₁-C₄-Alkyl, -CF₃, -CR⁷=NOR⁷ (wobei die Reste R⁷ gleich oder verschieden sein können), -NMe₂, NEt₂, -NO₂ oder -OR⁷ substituiert sein kann,
R¹ Phenyl, das durch einen Rest der Formel 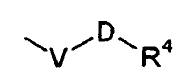 substituiert ist, mit der Maßgabe, daß V Sauerstoff oder NR⁷ ist und D eine C₁-C₄-Alkyl-Brücke repräsentiert,
- R¹: ein C- oder N-verknüpfter 5 oder 6-gliedriger Heterocyclus, der ein oder mehrere Heteroatome aus der Gruppe Stickstoff, Sauerstoff oder Schwefel enthält und gegebenenfalls ein oder mehrfach durch Benzyl, Methyl, Fluor, Chlor, Brom oder Hydroxy substituiert sein kann,
- R¹: Cyclopropyl, Cyclopentyl oder Cyclohexyl, die gegebenenfalls durch = O oder -OR⁷ substituiert sind,
- R¹: Norbornan, Norbomen, Dicyclopropylmethyl, Adamantan oder Noradamantan, die gegebenenfalls durch Methyl substituiert sind,
- R¹: ein Rest der Formel 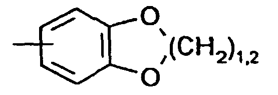
- R¹: -CH=CH-Phenyl, wobei der Phenylring durch Methoxy oder Hydroxy substituiert sein kann;
- R²: Wasserstoff, Fluor, Chlor, Brom, C₁-C₄-Alkyloxy, C₁-C₄-Alkyl oder Hydroxy;
- R3: Wasserstoff;
- R⁴: Hydroxy, CN oder NR⁵R⁶;
- R⁴: ein N-Oxid der Formel 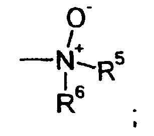
- R⁵: Wasserstoff, C₁-C₃-Alkyl, Benzyl oder Phenyl;
- R6: Wasserstoff, C₁-C₃-Alkyl, Benzyl oder Phenyl;
oder
- R⁵ und R⁶: bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus ein oder mehrfach durch Methyl substituiert sein kann;
- R⁷: Wasserstoff, C₁-C₄-Alkyl, ein Benzyl- oder Phenyl-Rest, wobei der Phenylring gegebenenfalls ein- oder mehrfach substituiert ist durch OH oder OCH₃,
bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderem Interesse sind Verbindungen der allgemeinen Formel (I) 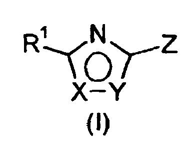
worin
- X und Y: Sauerstoff oder Stickstoff, wobei X und Y nicht beide gleichzeitig Sauerstoff oder Stickstoff sind,
- Z: ein Rest der Formel 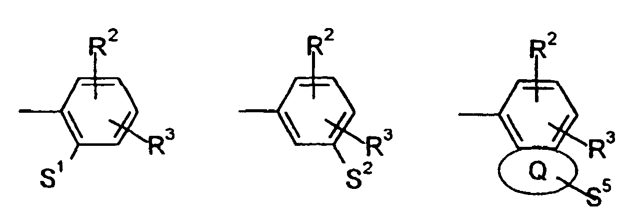 worin
- S¹: ein Rest der Formel 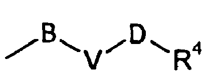
worin V Sauerstoff, Schwefel oder NR⁷ bedeutet, B -CH₂- ist und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
- S¹: ein Rest der Formel 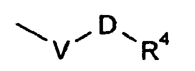 worin V und D die zuvor genannte Bedeutung aufweisen,
- S¹: Piperazin-1-yl, 4-Methyl-piperazin-1-yl oder 4-Benzyl-piperazin-1-yl;
- S¹: ein Rest der Formel 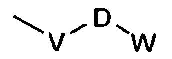
wobei V und D die oben genannte Bedeutung aufweisen und W ein gegebenenfalls durch C₁-C₄-Alkyl substituierter Rest der Formel 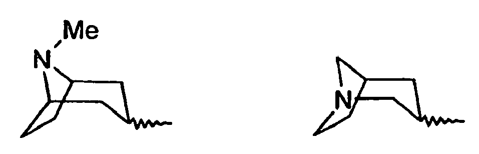 sein kann,
oder
W ein C-verknüpfter 5 oder 6-gliedriger Stickstoffheterocyclus ist, der gegebenenfalls durch Benzyl oder C₁-C₄-Alkyl substituiert sein kann;
- S¹: ein Rest der Formel

―V―W

wobei V und W die oben angegebenen Bedeutung aufweisen;
- S²: ein Rest der Formel

-(CH₂)_{0,1}-O-(CH₂)_{2,3}-R⁴
- Q: ein ankondensierter, einfach oder mehrfach ungesättigten 5 oder 6- gliedriger Ring, der ein Heteroatom aus der Gruppe Sauerstoff, Stickstoff oder Schwefel enthalten kann;
- S⁵: ein Rest der Formel

―D―R⁴

wobei D die oben genannte Bedeutung aufweist;
- R¹: Cyclopropyl, Cyclopentyl, Benzyl oder Phenyl, wobei der Phenylring ein oder mehrfach durch einen oder mehrere der Reste Fluor, Chlor, Brom, C₁-C₄-Alkyl, -CF₃, -CMe=NOH, -NMe₂, -NO₂ oder -OR⁷ substituiert sein kann,
- R¹: Phenyl, das durch einen Rest der Formel

―O―(CH₂)_{2,3}―R⁴

substituiert ist,
- R¹: Furan, Thiophen, Pyridin oder Pyrrol, die gegebenenfalls ein- oder mehrfach durch Methyl substituiert sein können,
- R¹: Norboman, Norbomen, Adamantan oder Noradamantan,
- R¹: -CH=CH-Phenyl, wobei der Phenylring durch Hydroxy substituiert sein kann;
- R²: Wasserstoff, Fluor, Chlor, Brom, C₁-C₄-Alkyloxy, C₁-C₄-Alkyl oder Hydroxy;
- R3: Wasserstoff;
- R⁴: N-Morpholinyl, N-Pyrrolidinyl, N-Piperidinyl, N-Piperazinyl, 4-Methylpiperazin-1-yl oder 4-Benzyl-piperazin-1-yl;
- R⁴: CN, NR⁵R⁶ oder ein N-Oxid der Formel 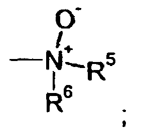
- R⁵: Wasserstoff, C₁-C₃-Alkyl, Benzyl oder Phenyl;
- R⁶: Wasserstoff, C₁-C₃-Alkyl, Benzyl oder Phenyl;
- R⁷: Wasserstoff, Methyl, Ethyl, Propyl, iso-Propyl, Butyl, tert.-Butyl, ein Benzyl- oder Phenyl-Rest, wobei der Phenylring gegebenenfalls ein- oder mehrfach substituiert ist durch OH oder OCH₃, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderem Interesse sind ferner Verbindungen der allgemeinen Formel (I) 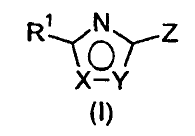
worin
- X und Y: Sauerstoff oder Stickstoff, wobei X und Y nicht beide gleichzeitig Sauerstoff oder Stickstoff sind,
- Z: ein Rest der Formel 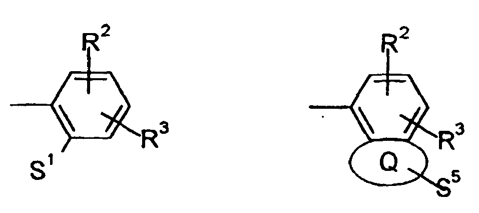 worin
- S¹: ein Rest der Formel 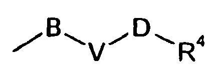
worin V Sauerstoff, Schwefel oder NR⁷ bedeutet, B -CH₂- ist und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
- S¹: ein Rest der Formel 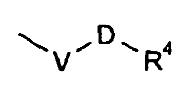 worin V und D die zuvor genannte Bedeutung aufweisen,
- S¹: Piperazin-1-yl, 4-Methyl-piperazin-1-yl oder 4-Benzyl-piperazin-1-yl;
- S¹: ein Rest der Formel 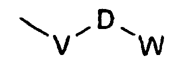
wobei V und D die oben genannte Bedeutung aufweisen und W ein gegebenenfalls durch C₁-C₄-Alkyl substituierter Rest der Formel 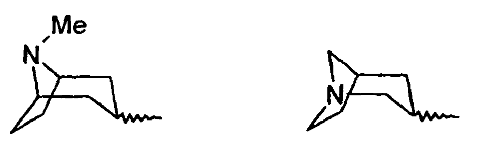 sein kann,
oder
W ein C-verknüpfter 5 oder 6-gliedriger Stickstoffheterocyclus ist, der gegebenenfalls durch Benzyl oder C₁-C₄-Alkyl substituiert sein kann;
- S¹: ein Rest der Formel

―V―W

wobei V und W die oben angegebenen Bedeutungen aufweisen;
- Q: ein ankondensierter, einfach oder mehrfach ungesättigter 5 oder 6- gliedriger, heterocyclischer Ring, der ein Heteroatom aus der Gruppe Sauerstoff, Stickstoff oder Schwefel enthalten kann;
- S⁵: ein Rest der Formel

―D―R⁴

wobei D die oben genannte Bedeutung aufweist;
- R¹: Cyclopropyl, Cyclopentyl oder Phenyl, wobei der Phenylring ein oder mehrfach durch einen oder mehrere der Reste Fluor, Chlor, Brom, C₁-C₄-Alkyl, -CF₃, -CMe=NOH, -NMe₂, -NO₂ oder -OR⁷ substituiert sein kann,
- R¹: Furan, Thiophen, Pyridin oder Pyrrol, die gegebenenfalls ein- oder mehrfach durch Methyl substituiert sein können,
- R¹: Norboman, Norbornen, Adamantan oder Noradamantan,
- R¹: -CH=CH-Phenyl, wobei der Phenylring durch Hydroxy substituiert sein kann;
- R²: Wasserstoff, Fluor, Chlor, Brom, C₁-C₄-Alkyloxy, C₁-C₄-Alkyl oder Hydroxy;
- R3: Wasserstoff;
- R⁴: CN, NR⁵R⁶ oder ein N-Oxid der Formel 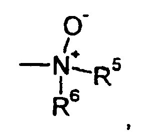
- R⁴: N-Morpholinyl, N-Pyrrolidinyl, N-Piperidinyl, N-Piperazinyl, 4-Methylpiperazin-1-yl oder 4-Benzyl-piperazin-1-yl;
- R⁵: Wasserstoff, C₁-C₃-Alkyl, Benzyl oder Phenyl;
- R⁶: Wasserstoff, C₁-C₃-Alkyl, Benzyl oder Phenyl;
- R⁷: Wasserstoff, Methyl, Ethyl, Propyl, iso-Propyl, Butyl, tert.-Butyl, ein Benzyl- oder Phenyl-Rest, wobei der Phenylring gegebenenfalls einoder mehrfach substituiert ist durch OH oder OCH₃, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Ferner sind von großem Interesse Verbindungen der allgemeinen Formel (I) 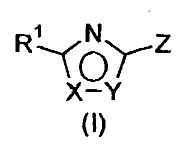
worin
- X und Y: Sauerstoff oder Stickstoff, wobei X und Y nicht beide gleichzeitig Sauerstoff oder Stickstoff sind,
- Z: ein Rest der Formel 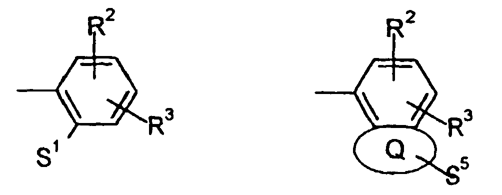
worin
- S¹: ein Rest der Formel 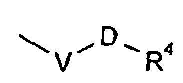
worin V Sauerstoff, Schwefel oder NR⁷ bedeutet und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
- S¹: Piperazin-1-yl, 4-Methyl-piperazin-1-yl oder 4-Benzyl-piperazin-1-yl;
- S¹: ein Rest der Formel 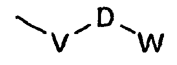 wobei V und D die oben genannte Bedeutung aufweisen und W ein Rest der Formel 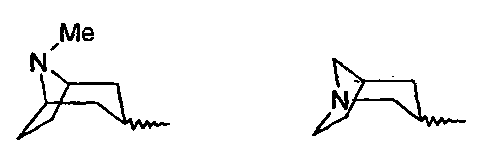 oder
W ein C-verknüpfter 5 oder 6-gliedriger Stickstoffheterocyclus ist, der gegebenenfalls durch Methyl substituiert sein kann;
- S¹: ein Rest der Formel

―V―W

wobei V und W die oben angegebenen Bedeutungen aufweisen;
- Q: ein ankondensierter, einfach oder mehrfach ungesättigter 5-gliedriger, heterocyclischer Ring, der ein Heteroatom aus der Gruppe Sauerstoff oder Stickstoff enthalten kann;
- S⁵: ein Rest der Formel

―D―R⁴

wobei D die oben genannte Bedeutung aufweist;
- R¹: Phenyl, das gegebenenfalls ein- oder mehrfach durch einen oder mehrere der Reste Fluor, Chlor, Brom, Methyl, Ethyl, n-Propyl, i-Propyl, n-Butyl, tert.-Butyl, -CF₃ oder -OR⁷ substituiert sein kann,
- R¹: Furan, Thiophen oder Pyridin, die gegebenenfalls ein- oder mehrfach durch Methyl substituiert sein können,
- R2: Wasserstoff, Fluor, Chlor, Brom, Methyl, Methyloxy oder Hydroxy;
- R³: Wasserstoff;
- R⁴: NR⁵R⁶ oder ein N-Oxid der Formel 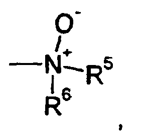
- R⁴: N-Morpholinyl, N-Pyrrolidinyl, N-Piperidinyl, N-Piperazinyl, 4-Methylpiperazin-1-yl oder 4-Benzyl-piperazin-1-yl;
- R⁵: Wasserstoff, Methyl, Ethyl, n-Propyl, i-Propyl, Benzyl oder Phenyl;
- R⁶: Wasserstoff, Methyl, Ethyl, n-Propyl, i-Propyl, Benzyl oder Phenyl;
- R⁷: Wasserstoff; Methyl oder Ethyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Besondere Bedeutung haben femer die Verbindungen der allgemeinen Formel (I) 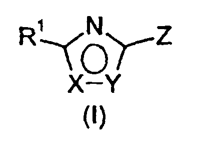
worin
- X und Y: Sauerstoff oder Stickstoff, wobei X und Y nicht beide gleichzeitig Sauerstoff oder Stickstoff sind,
- Z: ein Rest der Formel 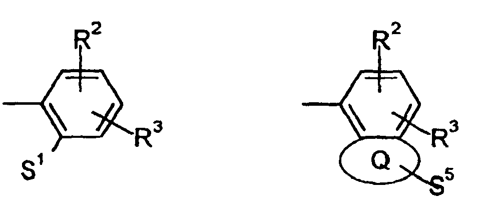 worin
- S¹: ein Rest der Formel 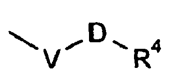 worin V Sauerstoff bedeutet und D eine der Gruppen -CH₂-, -CH₂-CH₂-, -CH₂-C(CH₃)H-, -CH₂-CH₂-CO- sein kann;
- S¹: N-Piperazinyl, 4-Benzyl-piperazin-1-yl;
- S¹: ein Rest der Formel 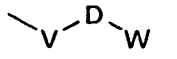 wobei V und D die oben genannte Bedeutung aufweisen und W ein Rest der Formel 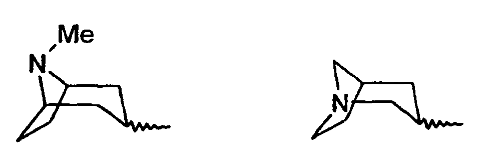 oder
W ein C-verknüpfter 5 oder 6-gliedriger Stickstoffheterocyclus ist, der gegebenenfalls durch Methyl substituiert sein kann;
- S¹: ein Rest der Formel

―V―W

wobei V und W die oben angegebenen Bedeutungen aufweisen;
- Q: ein ankondensierter, einfach oder mehrfach ungesättigter 5-gliedriger, heterocyclischer Ring, der als Heteroatom Sauerstoff enthält;
- S⁵: ein Rest der Formel

―D―R⁴

wobei D die oben genannte Bedeutung aufweist;
- R¹: Phenyl, das ein oder mehrfach durch einen oder mehrere der Reste Fluor, Chlor, Brom, Methyl, -CF₃, Hydroxy, Methyloxy oder Ethyloxy substituiert sein kann,
- R¹: Furan, Thiophen oder Pyridin;
- R²: Wasserstoff, Fluor, Chlor oder Methyl;
- R³: Wasserstoff;
- R4: NR⁵R⁶,
- R⁴: N-Morpholinyl, N-Pyrrolidinyl, N-Piperidinyl oder 4-Methylpiperazin-1-yl;
- R⁵: Wasserstoff, Methyl, Ethyl, n-Propyl, i-Propyl, Benzyl oder Phenyl;
- R⁶: Wasserstoff, Methyl, Ethyl, n-Propyl, i-Propyl, Benzyl oder Phenyl, bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Erfindungsgemäß besonders bevorzugt sind Verbindungen der allgemeinen Formel (I) 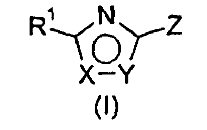
worin
- X und Y: Sauerstoff oder Stickstoff, wobei X und Y nicht beide gleichzeitig Sauerstoff oder Stickstoff sind,
- Z: ein Rest der Formel 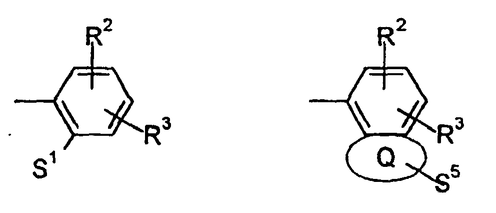 worin
- S¹: einer der Reste
-O-CH₂-CH₂-R⁴, -O-CH₂-C(CH₃)H-R⁴, -O-C(CH₃)H-CH₂-R⁴ oder -CH₂-CH₂-CO-R⁴ sein kann;
- S¹: 4-Benz yl-piperazin-1-yl;
- S¹: einer der Reste
-O-CH₂-W oder -O-W sein kann, wobei
W ein C-verknüpfter 5 oder 6-gliedriger Stickstoffheterocyclus ist, der gegebenenfalls durch Methyl substituiert sein kann;
- Q: ein ankondensierter, einfach oder mehrfach ungesättigter 5-gliedriger, heterocyclischer Ring, der als Heteroatom Sauerstoff enthält;
- S⁵: ein Rest der Formel -CH₂-R⁴;
- R¹: Phenyl, das ein- oder mehrfach durch einen oder mehrere der Reste Fluor, Chlor, Brom, Methyl, -CF₃, Hydroxy, Methyloxy oder Ethyloxy substituiert sein kann,
- R¹: Thiophen;
- R²: Wasserstoff, Fluor, Chlor oder Methyl;
- R³: Wasserstoff;
- R⁴: NR⁵R⁶,
- R⁴: N-Pyrrolidinyl oder N-Piperidinyl;
- R⁵: Wasserstoff, Methyl, Ethyl, i-Propyl, Benzyl oder Phenyl;
- R⁶: Wasserstoff, Methyl, Ethyl, i-Propyl, Benzyl oder Phenyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Als Alkylgruppen (auch soweit sie Bestandteil anderer Reste sind, beispielsweise Alkylenbrücken) werden, soweit nicht anders angegeben, verzweigte und unverzweigte Alkylgruppen mit 1bis 10 Kohlenstoffatomen bevorzugt 1- 4 Kohlenstoffatomen bezeichnet, beispielsweise werden genannt: Methyl, Ethyl, n-Propyl, iso-Propyl, Butyl, iso-Butyl, sec. Butyl, tert.-Butyl, Pentyl, iso-Pentyl, Hexyl, Heptyl und Octyl. Zur Bezeichnung der Gruppen Methyl, Ethyl, Butyl oder auch tert.-Butyl werden auch die Abkürzungen Me, Et, Bu oder tBu verwendet.

Substituierte Alkylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, C₁-C₆-Alkyloxy, Amino, Alkylamino, Dialkylamino, Cyano, Nitro, =O, -CHO, -COOH, -COO-C₁-C₆-Alkyl, -S-C₁-C₆-Alkyl.

Als Alkenylgruppen (auch soweit sie Bestandteil anderer Reste sind) werden verzweigte und unverzweigte Alkenylgruppen mit 2 bis 10 Kohlenstoffatomen, bevorzugt 2 bis 3 Kohlenstoffatomen genannt, soweit sie mindestens eine Doppelbindung aufweisen, beispielsweise auch oben genannte Alkylgruppen bezeichnet soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Substituierte Alkenylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, C₁-C₆-Alkyloxy, Amino, Alkylamino, Dialkylamino, Cyano, Nitro, =O, -CHO, -COOH, -COO-C₁-C₆-Alkyl, -S-C₁-C₆-Alkyl.

Als Alkinylgruppen (auch soweit sie Bestandteil anderer Reste sind) werden Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl.

Substituierte Alkinylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, C₁-C₆-Alkyloxy, Amino, Alkylamino, Dialkylamino, Cyano, Nitro, =O, -CHO, -COOH, -COO-C₁-C₆-Alkyl, -S-C₁-C₆-Alkyl.

Als Cycloalkylreste mit 3 - 6 Kohlenstoffatomen werden beispielsweise Cyclopropyl, Cyclobutyl, Cyclopentyl oder Cyclohexyl bezeichnet, die auch durch verzweigtes oder unverzweigtes Alkyl mit 1 bis 4 Kohlenstoffatomen, Hydroxy, und/oder Halogen oder wie zuvor definiert substituiert sein können. Als Halogen wird im allgemeinen Fluor, Chlor, Brom oder Jod bezeichnet.

Der Begriff Aryl steht für ein aromatisches Ringsystem mit 6 bis 10 Kohlenstoffatomen, das, soweit nicht anders beschrieben, beispielsweise einen oder mehrere der nachfolgend genannten Substituenten tragen kann: C₁-C₆-Alkyl, C₁-C₆-Alkyloxy, Halogen, Hydroxy, Mercapto, Amino, Alkylamino, Dialkylamino, CF₃, Cyano, Nitro, -CHO, -COOH, -COO-C₁-C₆-Alkyl, -S-C₁-C₆-Alkyl. Bevorzugter Arylrest ist Phenyl.

Als Beispiele für N-verknüpfte cyclische Reste der allgemeinen Formel NR⁵R⁶ seien genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen durch Alkyl mit 1 bis 4 Kohlenstoffatomen, bevorzugt Methyl substituiert sein können.

Als C-verknüpfte 5- oder 6-gliedrige heterocyclische Ringe, die als Heteroatome Stickstoff, Sauerstoff oder Schwefel enthalten können, werden beispielsweise Furan, Tetrahydrofuran, 2-Methyltetrahydrofuran, 2-Hydroxymethylfuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Oxazol, Isoxazol, Oxazin, Thiazol, Isothiazol, Thiadiazol, Oxadiazol, Pyrazolidin genannt, wobei der Heterocyclus wie in den Definitionen angegeben, substituiert sein kann.

"=O" bedeutet ein über eine Doppelbindung verknüpftes Sauerstoffatom.

Die vorliegende Erfindung beschreibt Verbindungen, die überraschenderweise eine hohe Affinität zu folgenden Rezeptortypen aufweisen: "Na⁺ Kanal site 2" Bindungsstelle, Histamin H1 Rezeptor, 5-Hydroxytryptamin 1A Rezeptor, 5-Hydroxytryptamin 2A Rezeptor, Sigma Rezeptor. Darüber hinaus zeigen diese Verbindungen antagonistische Aktivität am AMPA-Rezeptor. Die neuroprotektive Wirkung der erfindungsgemäßen Verbindungen wurde ebenfalls an einem Tiermodell bestätigt. Aufgrund dieser Befunde können die erfindungsgemäßen Verbindungen bei neurodegenerativen Erkrankungen sowie Gehirnischämie verschiedener Genese eingesetzt werden.

Die erfindungsgemäßen Verbindungen können unter Anwendung bekannter Verfahren u.a. wie folgt dargestellt werden.
In einer ersten Stufe wird ein Nitril der allgemeinen Formel (1) in Anlehnung an literaturbekannte Verfahren (L.F. Tietze, T. Eicher, "Reaktionen und Synthesen im Organisch-chemischen Praktikum und Forschungslaboratorium", 2. Auflage, 1991, Georg Thieme Verlag Stuttgart, New York, S. 340) mit Hydroxylamin zu einem Amidoxim der allgemeinen Formel (2) umgesetzt (Schema 1). Unter basischen Reaktionsbedingungen führt die Umsetzung dieses Amidoxims (2) mit durch nukleophile Gruppen substituierten Benzoesäurederivaten der allgemeinen Formel (3) zu Oxadiazolen der allgemeinen Formel (4). Benzoesäurederivate (3), die funktionalisierte Seitenketten tragen, sind unter Verwendung geeigneter Schutzgruppen einsetzbar.
Als Base kommen Alkali- oder Erdalkalialkoholate beispielsweise des Methanols, Ethanols, Isopropanols, n-, sec-, tert-Butylalkohols in Betracht. Geeignete Alkali- und Erdalkalimetalle sind beispielsweise Lithium, Natrium, Kalium, Magnesium, Calzium. Natriummethanolat, Natriumethanolat, Natriumisopropanolat, Kalium-tert-butanolat und Kaliumethanolat sind als Base besonders bevorzugt. Weiterhin kommen erfindungsgemäß als Basen Alkali- oder Erdalkalihydride in Betracht. 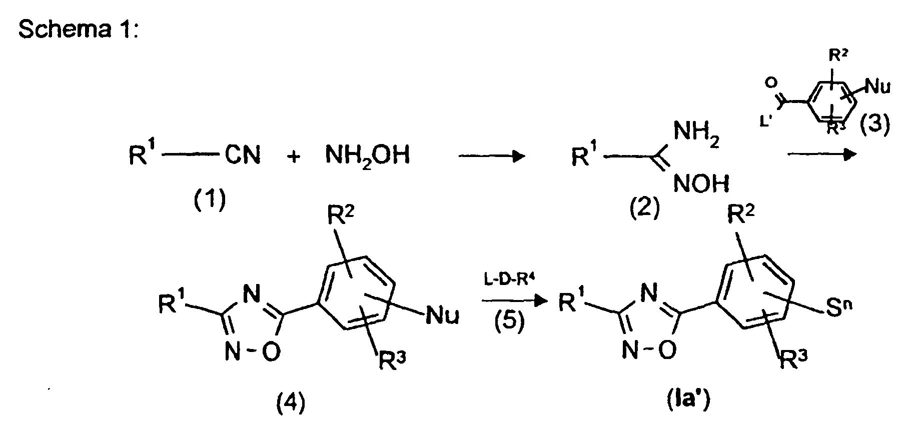 mit L'= Abgangsgruppe, beispielsweise Chlor, Brom, Alkyloxy; L= Abgangsgruppe, beispielsweise Chlor, Brom, lod, Methansulfonyl; Nu= OH, SH, NH₂ = VH gemäß der zuvor genannten Definitionen oder Nu= -B-OH, -B-SH, -B-NH₂ = B-VH gemäß der zuvor genannten Definitionen; Sⁿ= S¹, S², S³, S⁴ oder S⁵ gemäß der zuvor genannten Definitionen;

Die Modifikation der Seitenkette unter Bildung der Oxadiazolderivate der allgemeinen Formel (la') erfolgt gemäß Schema 1 durch abschließende Reaktion von (4) mit Elektrophilen der allgemeinen Formel (5). Hierzu werden die Oxadiazole (4) nach Zusatz einer Base in einem inerten Lösemittel bei Raumtemperatur mit den Elektrophilen (5) versetzt und nach bis zu einer Stunde, bevorzugt nach 15 bis 30 Minuten über einen Zeitraum von 4 bis 12 Stunden, bevorzugt 6 bis 8 Stunden zum Rückfluß erhitzt. Nach Abkühlen auf Raumtemperatur wird das Lösemittel zum großen Teil im Vakuum abdestilliert und das Produkt nach Waschen und Trocknen durch Kristallisation oder Chromatographie gereinigt. Erfindungsgemäß kommen als Basen Alkali- oder Erdalkalihydride in Betracht. Die Hydride des Natriums, Lithiums, Kaliums sowie des Magnesiums, Calziums sind bevorzugt. Geeignete inerte Lösemittel sind Dimethylformamid, Methylenchlorid sowie cyclische Ether wie Tetrahydrofuran oder bevorzugt Dioxan. Ferner können als Base Alkali- oder Erdalkalialkoholate beispielsweise des Methanols, Ethanols, Isopropanols, n-, sec-, tert-Butylalkohols zum Einsatz kommen. Geeignete Alkali- und Erdalkalimetalle sind beispielsweise Lithium, Natrium, Kalium, Magnesium, Calzium. Natriummethanolat, Natriumethanolat, Natriumisopropanotat, Kalium-tert-butanolat und Kaliumethanolat. Erfindungsgemäß können ebenfalls Alkali- oder Erdalkalihydroxide des Lithiums, Natriums, Kaliums sowie des Magnesiums, Calziums, bevorzugt jedoch Natriumhydroxid, Kaliumhydroxid, Lithiumhydroxid und Calziumhydroxid in alkoholischer oder wässriger Lösung verwendet werden.
Oxadiazole der allgemeinen Formel (Ia), die an Stelle des in (Ia') dargestellten Phenylrings einen heteroaromatischen Ring tragen sind in analoger Art und Weise darstellbar.

Die Reaktion von aromatischen Nitrilen der allgemeinen Formel (6) mit Elektrophilen der allgemeinen Formel (5) führt gemäß Schema 2 zu den durch die Seitenkette Sⁿ (n=1,2,3,4) substituierten aromatischen Cyaniden der allgemeinen Formel (7). Hierzu werden die Nitrile (6) nach Zusatz einer Base in einem inerten Lösemittel bei Raumtemperatur oder unter Erwärmen, bevorzugt auf 40 bis 80°C deprotoniert und anschließend mit den Elektrophilen (5) versetzt. Die erhaltene Lösung wird über einen Zeitraum von 0,25 bis 2 Stunden auf 40 bis 80°C erwärmt und nach Abkühlen auf Raumtemperatur im Vakuum vom Lösemittel befreit. Das Produkt wird nach Waschen und Trocknen ohne weitere Reinigung direkt in die nächste Stufe eingesetzt. Erfindungsgemäß sind als Basen Alkali- oder Erdalkalihydride, bevorzugt Hydride des Natriums, Lithiums, Kaliums sowie des Magnesiums und Calziums einsetzbar. Geeignete inerte Lösemittel sind Dimethylformamid, Methylenchlorid sowie cyclische Ether wie Tetrahydrofuran oder bevorzugt Dioxan. 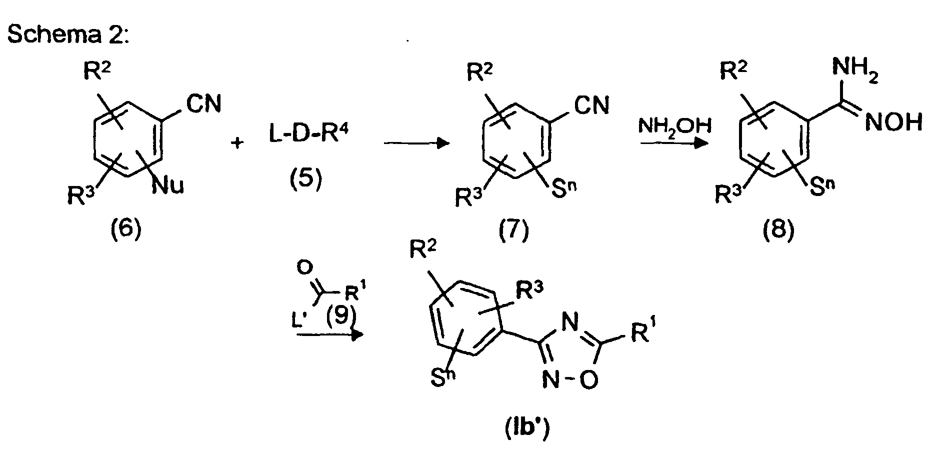 mit L'= Abgangsgruppe, beispielsweise Chlor, Brom, Alkyloxy; L= Abgangsgruppe, beispielsweise Chlor, Brom, lod, Methansulfonyl; Nu= OH, SH, NH₂ = VH gemäß der zuvor genannten Definitionen oder Nu= -B-OH, -B-SH, -B-NH B-VH gemäß der zuvor genannten Definitionen; Sⁿ= S¹, S², S³, S⁴ oder S⁵ gemäß der zuvor genannten Definitionen;

Diese Nitrile lassen sich in bekannter Weise (L. F. Tietze, T. Eicher, "Reaktionen und Synthesen im Organisch-chemischen Praktikum und Forschungslaboratorium", Georg Thieme Verlag, Stuttgart, 2. Auflage, 1991, S. 340) in die aromatischen Amidoxime der allgemeinen Formel (8) überführen. Im basischen Milieu liefern diese Amidoxime unter Reaktion mit den Carbonsäurederivaten (9) die Oxadiazole der Formel (Ib'). Hierzu werden die Amidoxime (8) mit den Carbonsäurederivaten (9) in einem inerten Lösemittel, bevorzugt ein Alkohol, besonders bevorzugt Ethanol, gelöst und unter Einwirkung einer Base erwärmt. Nach Abkühlen auf Raumtemperatur wird das Lösemittel zum großen Teil im Vakuum abdestilliert und das Produkt nach Waschen und Trocknen durch Kristallisation oder Chromatographie gereinigt. Als Basen kommen Alkali- oder Erdalkalialkoholate beispielsweise des Methanols, Ethanols, Isopropanols, n-, sec-, tert-Butylalkohols in Betracht. Geeignete Alkali- und Erdalkalimetalle sind beispielsweise Lithium, Natrium, Kalium, Magnesium, Calzium. Natriummethanolat, Natriumethanolat, Natriumisopropanolat, Kalium-tert-butanolat und Kaliumethanolat sind als Base besonders bevorzugt.
Oxadiazole der allgemeinen Formel (Ib), die an Stelle des in (Ib') dargestellten Phenylrings einen heteroaromatischen Ring tragen sind in analoger Art und Weise darstellbar. In der Seitenkette weiter funktionalisierte Oxadiazole der allgemeinen Formel (Ib) sind unter Verwendung geeigneter Schutzgruppen zugänglich.

Oxadiazolderivate der allgemeinen Formel (10), die am aromatischen Ring eine durch eine Abgangsgruppe substituierte Seitenkette tragen lassen sich erfindungsgemäß durch Umsetzung mit den Nukleophilen der allgemeinen Formel (11) in Verbindungen der allgemeinen Formel (I') überführen (Schema 3). 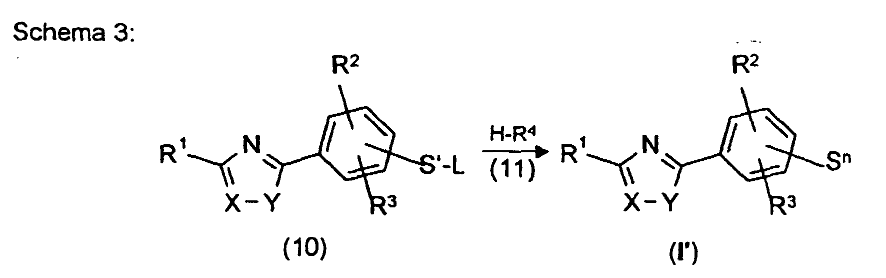 mit L= Abgangsgruppe, beispielsweise Chlor, Brom, lod, Methansulfonyl; S'= -B-V-D, -V-D wobei B, V und D die in den Definitionen angegebene Bedeutung haben; Sⁿ= S¹, S², S³, S⁴ oder S⁵ mit der zuvor genannten Bedeutung;

Hierzu werden die Verbindungen (10) in einem inerten Lösemittel gelöst und nach Zusatz der Nukleophile (11) über einen Zeitraum von 0,5 bis 2 Stunden, bevorzugt 1 bis 1,5 Stunden, auf 50 bis 120°C erwärmt. Nach Abkühlen auf Raumtemperatur wird das Lösemittel zum großen Teil im Vakuum abdestilliert und das Produkt nach Waschen und Trocknen durch Kristallisation oder Chromatographie gereinigt.

Geeignete inerte Lösemittel sind Dimethylformamid, Methylenchlorid sowie cyclische Ether wie Tetrahydrofuran oder bevorzugt Dioxan.
Oxadiazole der allgemeinen Formel (I), die an Stelle des in (I') dargestellten Phenylrings einen heteroaromatischen Ring tragen sind in analoger Art und Weise darstellbar. Die Synthese von in den Seitenketten weiter funktionalisierten Oxadiazolen der allgemeinen Formel (I) ist unter Verwendung geeigneter Schutzgruppen durchführbar.

Die vorliegende Erfindung soll anhand der folgenden Synthesevorschriften näher erläutert werden ohne sie auf deren Gegenstand zu beschränken.

### Beispiel 1: 5-{2-[2(N,N-Dimethylamino)ethyl]oxymethyl-phenyl}-3-phenyl-1,2,4-oxadiazol

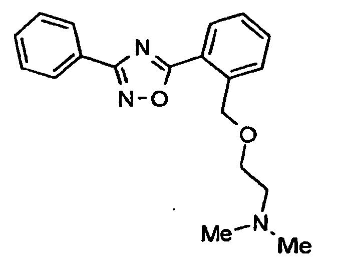

### a) Darstellung des Benzoesäureamidoxims:

14 g Hydroxylamin Hydrochlorid werden in 50 ml Wasser gelöst und unter Rühren und Eiskühlung mit 16,8 g Natriumhydrogencarbonat versetzt. Zu dieser Mischung wird eine Lösung von 10,3 g Benzoesäurenitril in 100 ml Ethanol gegeben und 3 Stunden unter Rückfluß erhitzt. Anschließend wird das Ethanol im Vakuum verdampft und der Rückstand 2 mal mit Diethylether extrahiert. Die vereinigten Ether-Phasen werden über Natriumsulfat getrocknet und eingeengt. Der Rückstand (13,4g = 98,5% der Th.) wird ohne weitere Reinigung in die Cyclisierungsreaktion eingesetzt.

### b) Darstellung des 5-(2-Hydroxymethyl-phenyl)-3-phenyl-1,2,4-oxadiazols:

1,36 g Benzoesäureamidoxim werden zu einer frisch hergestellten Lösung aus 0,46 g Natrium in 50 ml wasserfreiem Ethanol gegeben und 15 Minuten gerührt. Anschließend gibt man 2,68 g Phthalid unter Rühren zu und erhitzt für 15 Stunden unter Rückfluß. Die dunkelrote Lösung wird im Vakuum eingeengt und der Rückstand in Wasser aufgenommen. Es mit mit 2 N Salzsäure neutralisiert und mit Dichlormethan extrahiert. Die organische Phase wird über Natriumsulfat getrocknet, eingeengt und an Kieselgel mit Dichlormethan/Methanol (98:2) chromatographiert. Ausbeute: 1,2 g (48 % d. Th.).

### c) Darstellung von 5-{2-[2(N,N-Dimethylamino)ethyl]oxymethyl-phenyl}-3-phenyl-1.2.4-oxadiazol:

1 g 5-(2-Hydroxymethyl-phenyl)-3-phenyl-1,2,4-oxadiazol werden in 20 ml DMF gelöst und mit 0,2 g Natriumhydrid (60% in ÖI) versetzt. Man rührt anschließend noch 30 Minuten bei 20-23°C und gibt dann eine zuvor 30 Minuten gerührte Mischung aus 2-N,N-Dimethylaminoethylchlorid und 0,22 g Natriumhydrid (60% in Öl) in 20 ml DMF zu. Diese Mischung wird erhitzt man für 5 Stunden auf 100°C, verdampft anschließend das Lösemittel im Vakuum. Der Rückstand wird in Wasser aufgenommen, mit 2 N Salzsäure angesäuert und mit Essigsäureesthylester extrahiert. Die wässrige Phase wird mit Natronlauge alkalisch gestellt und mit Dichlormethan extrahiert. Die organische Phase wird über Natriumsulfat getrocknet, im Vakuum eingeengt und an Kieselgel chromatographiert (Methanoi). Das Produkt wird mit etherischer HCl-Lösung ins Hydrochlorid überführt und aus Ethanol/Ether umkristallisiert. Ausbeute: 0,07 g (5 % d. Th.), Smp.: 107°C (Zers.).

### Beispiel 2: 5-{2-[2-(N,N-Dimethylamino)ethyl]oxy-phenyl}-3-phenyl-1,2,4-oxadiazol

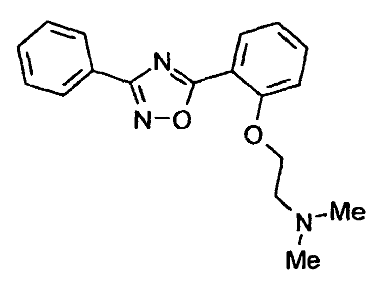

a) Darstellung des 5-(2-Hydroxyphenyl)-3-phenyl-1,2,4-oxadiazols: 6,8 g Benzoesäureamidoxim und 15,2 g Salicylsäuremethylester werden in 150 ml wasserfreiem Ethanol gelöst, mit 2,3 g Natrium versetzt und 3 mal 25 Minuten (mit jeweils 5 Minuten Unterbrechung) bei 400 W in der Mikrowelle erhitzt. Die Reaktionsmischung wird im Vakuum auf ca. 1/3 des Volumens eingeengt und der Rückstand mit Wasser versetzt. Unter Kühlung wird mit 2N Salzsäure auf pH 8-9 eingestellt, der entstehende Niederschlag wird abgesaugt und mit Wasser gewaschen. Zum restlosen Entfernen des Wassers wird in Dichlormethan gelöst, mit Natriumsulfat getrocknet und eingeengt. Ausbeute: 12,9 g (92% d. Th. bez. auf Benzoesäureamidoxim). Smp.: 156-158°C.

### b)Darstellung des 5-{2-[2-(N,N-Dimethylamino)ethyl]oxy-phenyl}-3-phenyl-1,2,4-oxadiazols:

2,38 g 5-(2-Hydroxyphenyl)-3-phenyl-1,2,4-oxadiazol werden in 100 ml wasserfreiem Dioxan gelöst und mit 0,3 g 80%iger Natriumhydrid-Suspension in Öl 15 Minuten bei 25-30°C gerührt. Zu dieser Lösung gibt man 60 ml wasserfreies Dioxan, versetzt mit 2,88 g 2-(N,N-Dimethylamino)ethylchlorid Hydrochlorid und 0,6 g 80%ige Natriumhydrid-Suspension in Öl. Diese Lösung wird ebenfalls 15 Minuten bei 25-30°C gerührt. Die vereinigten Lösungen werden 8 Stunden unter Rückfluß erhitzt, man läßt über Nacht stehen und dampft im Vakuum ein. Der Rückstand wird mit Wasser und 20 ml 1 N Natronlauge versetzt und mit Essigester extrahiert. Die organische Phase wird über Natriumsulfat getrocknet, das Lösemittel im Vakuum verdampft und der Rückstand an Kieselgel mit Essigester/Isopropanol (70:30, versetzt mit 2,5% einer 25%igen Ammoniak-Lösung) chromatographiert. Die so erhaltene Base wird in wasserfreiem Ethanol gelöst, mit etherischer HCl angesäuert und mit Diethylether ausgefällt. Der Rückstand wird aus wasserfreiem Ethanol und Diethylether umkristallisiert. Ausbeute: 2,2 g (64% d. Th.). Smp.: 186-187°C.

### Beispiel 3: 3-{2-[2-(N,N-Dimethylamino)ethyl]oxy-phenyl}-5-phenyl-1,2,4-oxadiazol

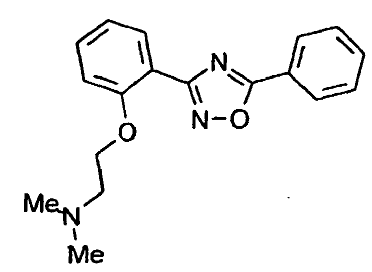

### a) Darstellung des 2-[2-(N,N-Dimethylamino)ethyloxy]-benzoesäurenitrils:

23,8 g 2-Hydroxybenzoesäurenitril werden mit 6,0 g 80%iger Natriumhydrid-Suspension in Öl in 200 ml Dioxan 30 Minuten bei 60°C gerührt. Zu dieser Lösung gibt man 100 ml wasserfreies Dioxan, versetzt mit 28,8 g 2-(N,N-Dimethylamino)ethylchlorid Hydrochlorid und 6,0 g 80%ige Natriumhydrid-Suspension in ÖI. Diese Lösung wird ebenfalls 30 Minuten bei 60°C gerührt. Die vereinigten Lösungen werden 4 mal 8 Minuten bei 400 W in der Mikrowelle erhitzt und anschließend das Lösemittel im Vakuum eingedampft. Der Rückstand wird mit Wasser und 1 N Natronlauge versetzt und mit Diethylether extrahiert. Die organische Phase wird über Natriumsulfat getrocknet, das Lösemittel im Vakuum verdampft. Ausbeute. 19,6 g (52% d. Th.).

### b) Darstellung des 2-[2-(N,N-Dimethylamino)ethyloxy]-benzoesäureamidoxims:

14 g Hydroxylamin Hydrochlorid werden in 100 ml Wasser gelöst und unter Rühren portionsweise mit 16,8 g Natriumhydrogencarbonat versetzt. Zu dieser Mischung wird eine Lösung von 19,0 g 2-(N,N-Dimethylamino)ethyloxy-benzoesäurenitril in 150 ml Ethanol gegeben und 5 Stunden unter Rückfluß erhitzt. Anschließend wird das Ethanol im Vakuum verdampft und der Rückstand 2 mal mit Diethylether extrahiert. Die vereinigten Ether-Phasen werden über Natriumsulfat getrocknet und eingeengt. Der Rückstand (16 g = 72% d. Th.) wird ohne weitere Reinigung in die Cyclisierungsreaktion eingesetzt.

### c) Darstellung des 3-{2-[2-(N,N-Dimethylamino)ethyl]oxy-phenyl}-5-phenyl-1,2,4-oxadiazols:

4,46 g 2-(N,N-Dimethylamino)ethyloxy-benzoesäureamidoxim und 5,44 g Benzoelsäuremethylester werden in 150 ml absolutem Ethanol gelöst, mit 2,3 g Natrium versetzt und 2 mal 11 Minuten (mit 5 Minuten Unterbrechung) bei 300 W in der Mikrowelle erhitzt. Die Reaktionsmischung wird im Vakuum eingeengt, der Rückstand mit Wasser versetzt und mit Essigester extrahiert. Die organische Phase wird über Natriumsulfat getrocknet, das Lösemittel im Vakuum verdampft und der Rückstand an Kieselgel mit Essigester/Isopropanol (70:30, versetzt mit 2,5% einer 25%igen Ammoniak-Lösung) chromatographiert. Die so erhaltene Base wird in wasserfreiem Ethanol gelöst, mit etherischer HCl angesäuert und mit Diethylether ausgefällt. Der Rückstand wird aus wasserfreiem Ethanol und Diethylether umkristallisiert. Ausbeute: 2,5 g (36d. Th.). Smp.: 174-175°C

### Beispiel 4: 5-{2-[2-(Morpholino)ethyl]oxy-phenyl}-3-phenyl-1,2,4-oxadiazol

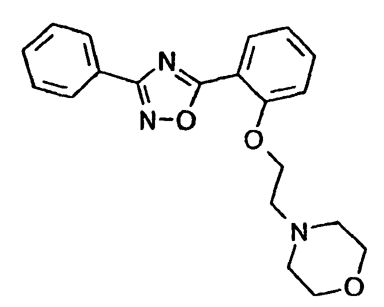

2,38 g 5-(2-Hydroxyphenyl)-3-phenyl-1,2,4-oxadiazol werden in 100 ml wasserfreiem Dioxan gelöst und mit 0,3 g 80%iger Natriumhydrid-Suspension in Öl 15 Minuten bei 25-30°C gerührt. Zu dieser Lösung gibt man 75 ml absolutes Dioxan, versetzt mit 3,72 g 2-(Morpholino)ethylchlorid Hydrochlorid und 0,6 g 80%ige Natriumhydrid-Suspension in Öl. Diese Lösung wird ebenfalls 15 Minuten bei 25-30°C gerührt. Die vereinigten Lösungen werden 6 Stunden bei 100°C erhitzt und anschließend im Vakuum eingeengt. Der Rückstand wird mit Wasser und 20 ml 1 N Natronlauge versetzt und mit Essigester extrahiert. Die organische Phase wird über Natriumsulfat getrocknet, das Lösemittel im Vakuum abdestilliert und der Rückstand an Kieselgel mit Essigester/Isopropanol (70:30, versetzt mit 1,5% einer 25%igen Ammoniak-Lösung) chromatographiert. Die so erhaltene Base wird in wasserfreiem Ethanol gelöst, mit etherischer HCl angesäuert und mit Diethylether ausgefällt. Der Rückstand wird aus wasserfreiem Ethanol und Diethylether umkristallisiert. Ausbeute: 1,9 g (49% d. Th.). Smp.: 194-195°C.

### Beispiel 5: 5-{2-[2-(4-Methylpiperazin-1-yl)ethyl]oxy-phenyl}-3-phenyl-1,2,4-oxadiazol

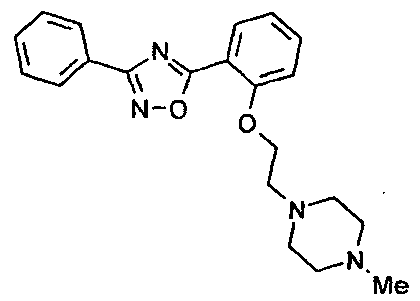

a) Darstellung von 5-(2-(2-Bromethyl)oxy-phenyl]-3-phenyl-1,2,4-oxadiazol: 1,85 g 5-(2-Hydroxyphenyl)-3-phenyl-1,2,4-oxadiazol werden in 80 ml Methylethylketon gelöst, mit 5 ml 1,2-Dibromethan, 6 g Kaliumcarbonat und mit 0,1 g Kaliumiodid versetzt. Die Mischung erhitzt man 12 Stunden unter Rückfluß und filtriert nach dem Abkühlen den Niederschlag ab. Die organische Phase wird im Vakuum eingeengt und an Kieselgel zunächst mit Toluol, dann mit Dichlormethan als Eluent chromatographiert. Ausbeute: 2,3 g (86% d. Th.).

b) Darstellung von 5-{2-[2-(4-Methylpiperazin-1-yl)ethyl]oxy-phenyl}-3-phenyl-1,2,4-oxadiazol:1,72 g 5-[2-(2-Bromethyl)oxy-phenyl]-3-phenyl-1,2,4-oxadiazol werden in 50 ml wasserfreiem Dioxan gelöst und mit 2 g N-Methylpiperazin versetzt. Die Lösung wird eine Stunde unter Rückfluß erhitzt und anschließend im Vakuum eingeengt. Der Rückstand an Kieselgel mit Dichlormethan/Methanol (90/10) chromatographiert. Die so erhaltene Base wird in wasserfreiem Ethanol gelöst, mit etherischer HCl angesäuert und mit Diethylether ausgefällt. Der Rückstand wird aus wasserfreiem Ethanol und Diethylether umkristallisiert. Ausbeute: 1,7 g (78% d. Th.). Smp.: 251-253°C.

### Beispiel 6: 5-Phenyl-3-[2-(4-methylpiperazin-1-yl)-phenyl]-1,2,4-oxadiazol

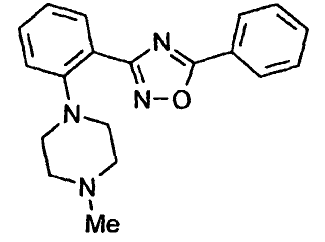

### a) Darstettung von 1-(2-Cyanophenyl)piperazin:

(in Analogie zu G. E. Martin, R. J. Elgin, J. R. Mathiasen, C. B. Davis, J. M. Kesslick, J. Med. Chem. 32 (1989) 1052-1056)

### b) Darstellung von 1-(2-Cyanophenyl)-4-methylpiperazin:

7,48 g 1-(2-Cyanophenyl)-Piperazin werden mit 30 ml Formaldehyd und 30 ml Ameisensäure 1 Stunde unter Rückfluß erhitzt. Die Lösemittel werden im Vakuum verdampft und der Rückstand in Ether aufgenommen und mit Wasser versetzt. Mit 20%iger Natronlauge wird alkalisch gestellt, mit Kaliumcarbonat gesättigt und mit Ether extrahiert. Die organische Phase wird über Natriumsulfat getrocknet und im Vakuum eingeengt. Zur Reinigung wird an Kieselgel mit Essigester/lsopropanol (70:30, versetzt mit 1% einer 25%igen Ammoniak-Lösung) chromatographiert. Ausbeute: 6,7 g (83% d. Th.).

### c) Die Darstellung der Amidoxime erfolgt gemäß der für Beispiel 3b beschriebenen Vorgehensweise.

### d) Die Darstellung der 1,2,4-Oxadiazole erfolgt gemäß der für Beispiel 3c beschriebenen Vorgehensweise.

### Beispiel 7: 3-Phenyl-5-[2-(piperazin-1-yl)-phenyl]-1,2,4-oxadiazol

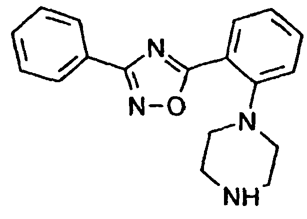

### a) Darstellung von 2-(Piperazin-1-yl)-benzoesäuremethylester:

(in Analogie zu G. S. Poindexter, M. A. Bruce, K. L. LeBoulluec, I. Monkovic, Tetrahedron Lett. 35 (1994) 7331-7334)

### b) Darstellung von 5-[2-(Piperazin-1-vl)-phenyl]-3-phenyl-1,2,4-oxadiazol:

1,36 g Benzoesäureamidoxim und 2,34 g 2-(Piperazin-1-yl)-benzoesäureethylester Maleinat werden mit 0,92 g Natrium in 100 ml wasserfreiem Ethanol versetzt und 6 mal 30 Minuten (mit jeweils 5 Minuten Unterbrechung) bei 350 W in der Mikrowelle erhitzt. Nach dem Abkühlen wird das Lösemittel im Vakuum eingeengt und der Rückstand mit Dichlormethan gegen Wasser extrahiert. Die organische Phase wird über Natriumsulfat getrocknet und im Vakuum eingeengt. Der Rückstand wird an Kieselgel mit Essigester/Isopropanol (70:30, versetzt mit 5% einer 25%igen Ammoniak-Lösung) chromatographiert. Ausbeute: 90 mg (3% d. Th.). Smp.: 254-255°C.

### Beispiel 8: 5-{2-[2-(N,N-Dimethylamino)ethyl]oxy-phenyl}-3-(4-hydroxyphenyl)-1,2,4-oxadiazol

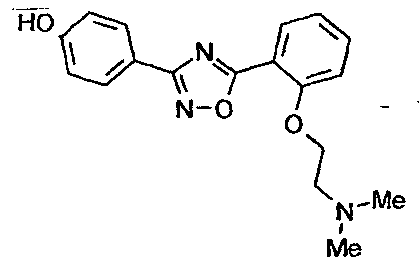

### a) Darstellung von 2-[2-(N,N-Dimethylamino)ethyl]oxy-benzoesäuremethylester:

15,2 g Salicylsäuremethylester werden in 200 ml wasserfreiem Acetonitril gelöst und mit 4,4 g 60%iger Natriumhydrid-Suspension in Öl 60 Minuten bei Raumtemperatur gerührt. Zu dieser Lösung gibt man 200 ml absolutes Acetonitril, versetzt mit 17,4 g 2-(N,N-Dimethylamino)ethylchlorid Hydrochlorid und 5,2 g 60%ige Natriumhydrid-Suspension in Öl. Diese Lösung wird ebenfalls 60 Minuten bei Raumtemperatur gerührt. Die vereinigten Lösungen werden 1 Stunden unter Rückfluß erhitzt und anschließend im Vakuum eingedampft. Der Rückstand wird mit Wasser versetzt und mit Essigester extrahiert. Die organische Phase wird über Natriumsulfat getrocknet, das Lösemittel im Vakuum abdestilliert und der Rückstand an Kieselgel mit Essigester/Methanol (1:1) chromatographiert. Man erhält so 14,6 g eines gelben Öls (65% d. Th.).

### b) Darstellung von 2-[2-(N,N-Dimethylamino)ethyl]oxy-benzoesäure:

4,4 g 2-[2-(N,N-Dimethylamino)ethyl]oxy-benzoesäuremethylester werden mit 30 ml 5N Salzsäure15 Minuten bei 300 W in der Mikrowelle erhitzt. Es wird mit Essigester extrahiert, die Wasserphase eingeengt und aus Acetonitril/Ether umkristallisiert. Ausbeute: 4,2 g (86% d. Th.).

### c) Darstellung von 5-{2-[2-(N,N-Dimethylamino)ethyl]oxy-phenyl}-3-(4-hydroxyphenyl)-1,2,4-oxadiazol:

2,45 g 2-[2-(N,N-Dimethylamino)ethyl]oxy-benzoesäure werden mit 1,91 g N-Ethyl-N-dimethylaminopropyl)carbodiimid Hydrochlorid und katalyt. Mengen Hydroxybenzotriazol in 50 ml DMF gelöst. Nach 15 Minuten gibt man 1,52 g 4-Hydroxybenzoesäureamidoxim hinzu und erhitzt die Mischung 15 Minuten bei 700 W in der Mikrowelle. Es wird eingeengt, der Rückstand in Essigester aufgenommen, mit Wasser gewaschen, über Natriumsulfat getrocknet und im Vakuum eingeengt. Der Rückstand wird über Kieselgel mit Ethanol filtriert und wie oben beschrieben in das Salz überführt. Ausbeute: 1,1g (29% d. Th.). Smp.: 170°C Zers.

### Beispiel 9: 5-{2-[(Carboxamido)methyl]oxy-phenyl}-3-phenyl-1,2,4-oxadiazo)

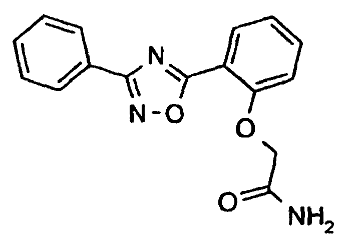

3,57 g 5-(2-Hydroxyphenyl)-3-phenyl-1,2,4-oxadiazol werden in 70 ml wasserfreiem DMF gelöst und mit 0,6 g 60%iger Natriumhydrid-Suspension in ÖI 30 Minuten bei 25-30°C gerührt. Zu dieser Lösung gibt man 1,4 g 2-Chloressigsäureamid und rührt für 2 Stunden bei 100°C. Anschließend dampft man das Lösemittel im Vakuum ein. Der Rückstand wird mit Wasser versetzt und abgesaugt. Der Niederschlag wird nacheinander mit Methanol und Essigsäureethylester ausgekocht. Ausbeute: 3,3 g (75 % d. Th.). Smp.: 249-251 °C.

### Beispiel 10: 5-{2-[2-(Carboxamido)ethyl]oxy-phenyl}-3-phenyl-1,2,4-oxadiazol

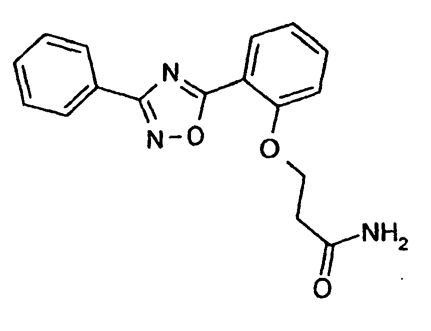

### a) Darstellung des 5-{2-[2-(1.3-Dioxan-2-yl)ethyl]oxy-phenyl}-3-phenyl-1,2 4-oxadiazol:

4,1 g 5-(2-Hydroxyphenyl)-3-phenyl-1,2,4-oxadiazol werden in 60 ml wasserfreiem DMF gelöst und mit 0,688 g 60%iger Natriumhydrid-Suspension in ÖI versetzt. Zu dieser Mischung gibt man 3,15 g 2-(2-Bromethyl)-1,3-dioxan und rührt für 4 Stunden bei 100°C. Nach dem Abkühlen verdampft man das Lösemittel im Vakuum und versetzt den Rückstand mit Wasser. Es wird mit Essigester extrahiert, die organische Phase über Natriumsulfat getrocknet und im Vakuum eingeengt. Die verbleibende Lösung wird an Kieselgel mit Essigsäureethylester chromatographiert. Ausbeute: 2,5 g (41 % d. Th.).

### b Darstellung von 5-{2-[2-(Carboxyl)ethyl]oxy-Phenyl}-3-Phenyl-1,2,4-oxadiazol:

2,5 g 5-{2-[2-(1,3-Dioxan-2-yl)ethyl]oxy-phenyl}-3-phenyl-1,2,4-oxadiazol werden in 50 ml Aceton gelöst und bei 0°C mit einer Lösung von Chrom(VI)oxid in 30proz. Schwefelsäure tropfenweise versetzt. Man rührt anschließend noch 20 Stunden bei 20-23°C und gibt dann unter Kühlung bei 5°C 25 ml lsopropanol zu. Die Mischung wird in eine Suspension von 100 ml Dichlormethan und 100 ml Wasser gegeben und die organische Phase abgetrennt. Man extrahiert die Wasserphase noch einmal mit Dichlormethan und die vereinigten organischen Lösungen nochmals mit Wasser. Man trocknet über Natriumsulfat, engt im Vakuum ein und chromatographiert den Rückstand an Kieselgel mit Dichlormethan/Methanol (97:3). Das Produkt wird aus Essigesäureethylester umkristallisiert. Ausbeute: 0,23 g (11 % d. Th.), Smp.: 170-171°C.

### c) Darstettung von 5-{2-[2-(Carboxamido)ethyl]oxy-phenyl}-3-phenyl-1,2,4-oxadiazol:

0,8 g 5-{2-[2-(Carboxyl)ethyl]oxy-phenyl}-3-phenyl-1,2,4-oxadiazol werden in 40 ml wasserfreiem Dichlormethan gelöst und auf 0°C gekühlt. Zu dieser Lösung gibt man dann 2 ml Oxalylchlorid, gelöst in 5 ml wasserfreiem Dichlormethan und rührt noch 1,5 Stunden bei 20 °C. Das Lösemittel wird im Vakuum verdampft und mit 30 ml wasserfreiem Dichlormethan versetzt. Unter Kühlung gibt man ammoniakalische Dichlormethan-Lösung zu, bis das Reaktionsmedium basisch reagiert. Man läßt 14 Stunden stehen, versetzt mit Wasser, trennt den entstandenen Niederschlag und die Dichlormethan-Phase ab und trocknet die organische Phase über Natriumsulfat. Nach dem Einengen beträgt der Rückstand 0,7 g. Dieser wird an Kieselgel mit Dichlormethan/Methanol (98:2) chromatographiert und das Produkt aus Essigester umkristallisiert. Ausbeute: 0,24 g (33 % d. Th.), Smp.: 137-138 °C.

In Analogie zu den zuvor beschriebenen Verfahren und Synthesebeispielen wurden u.a. die folgenden Verbindungen dargestellt:

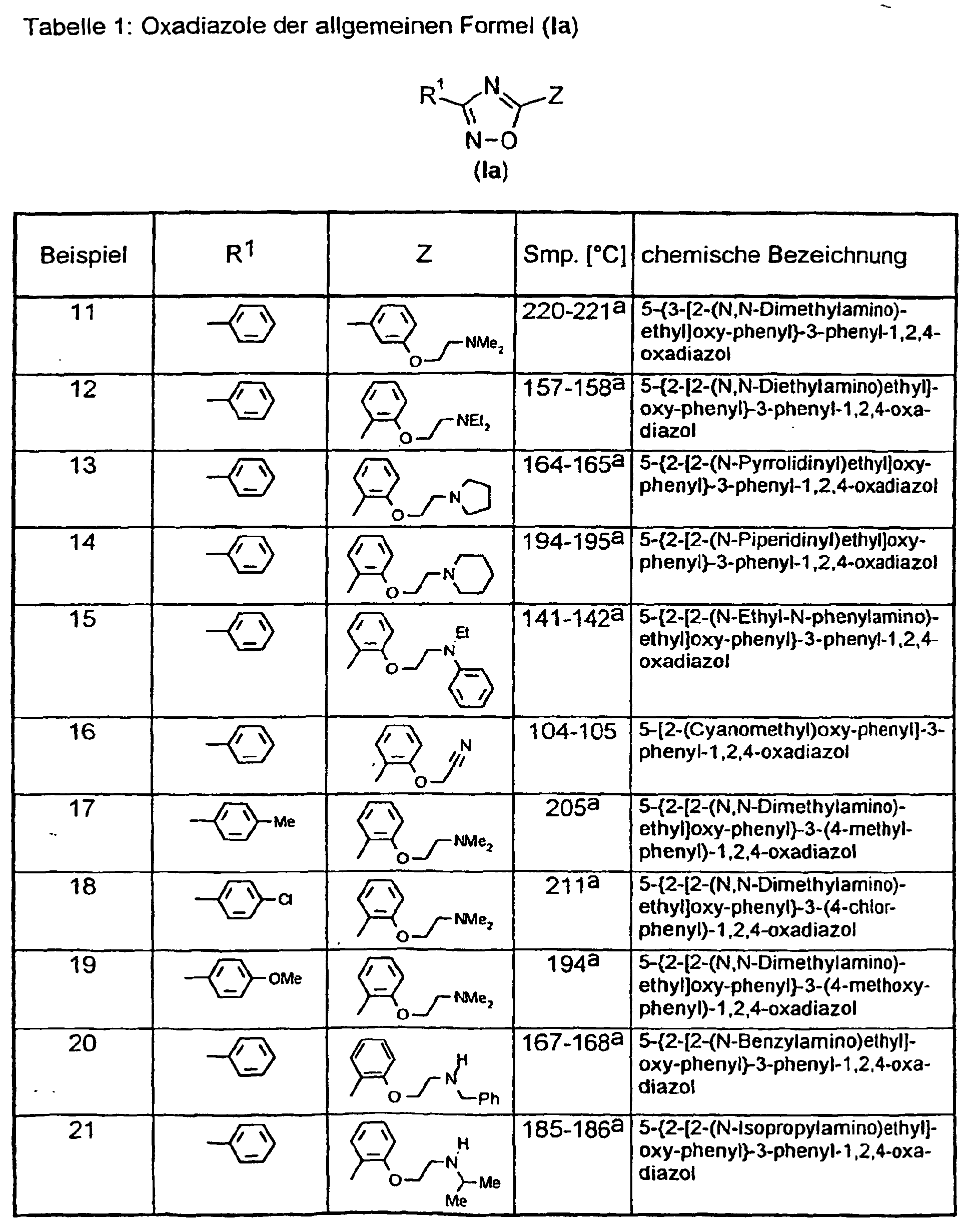

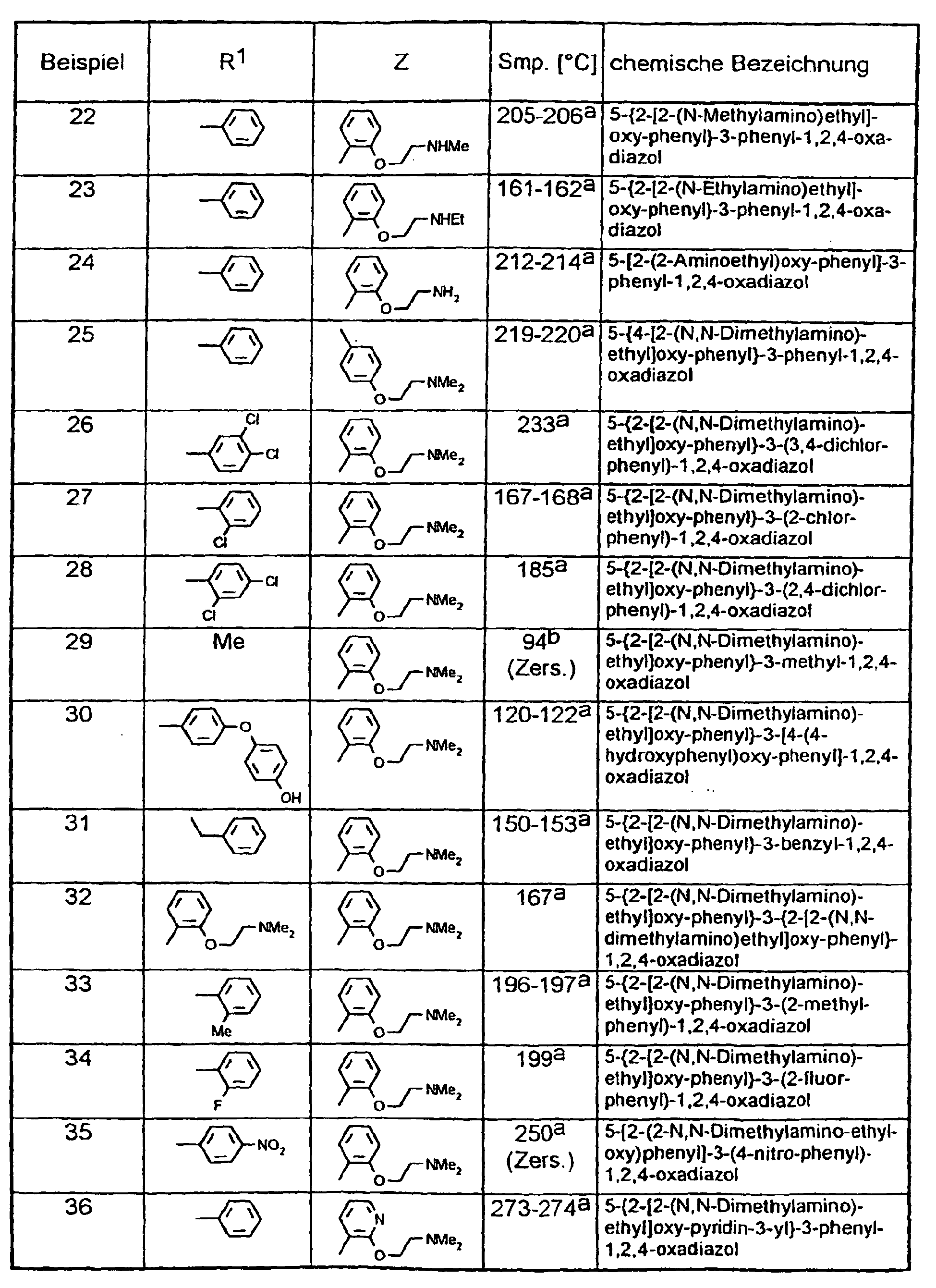

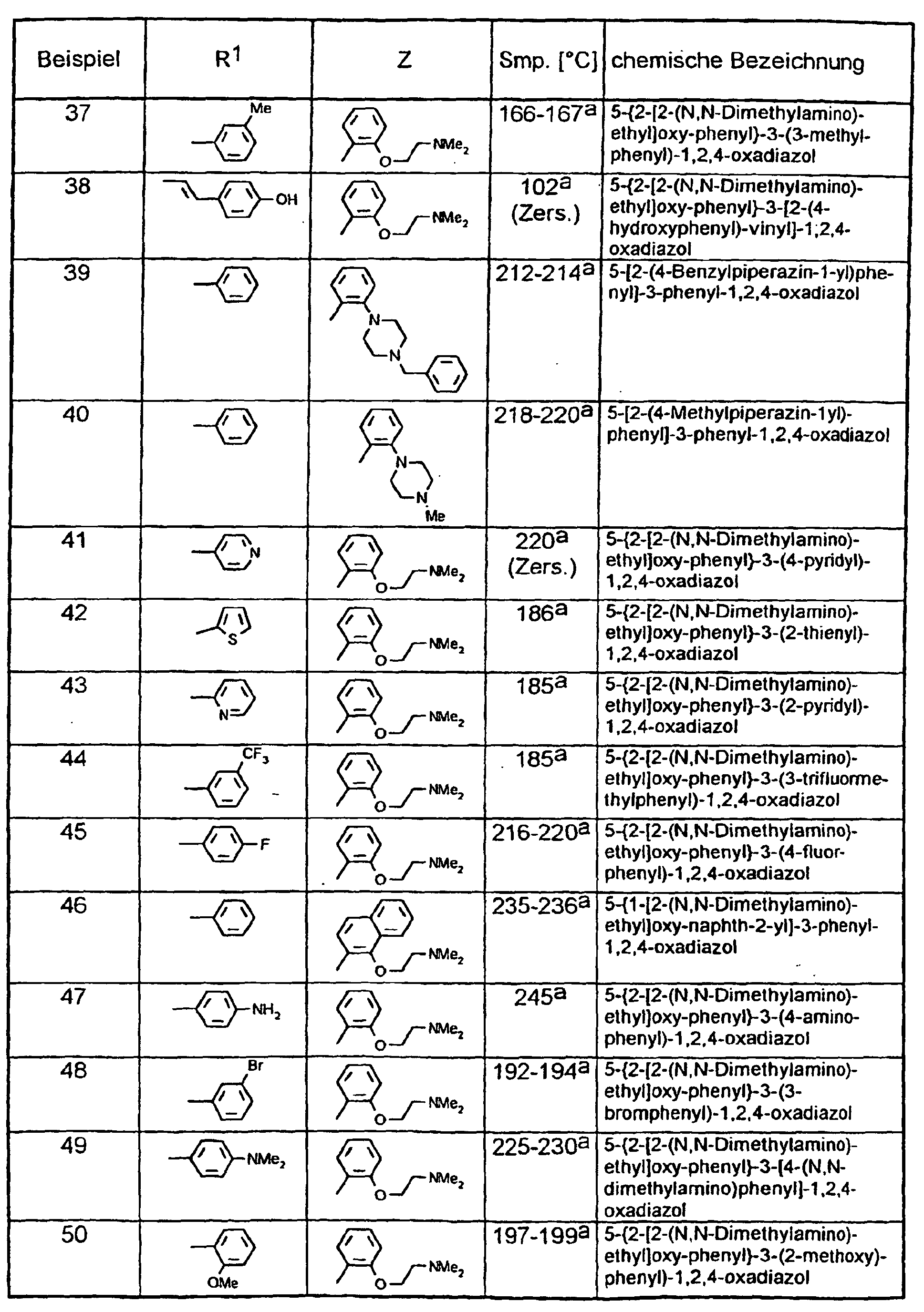

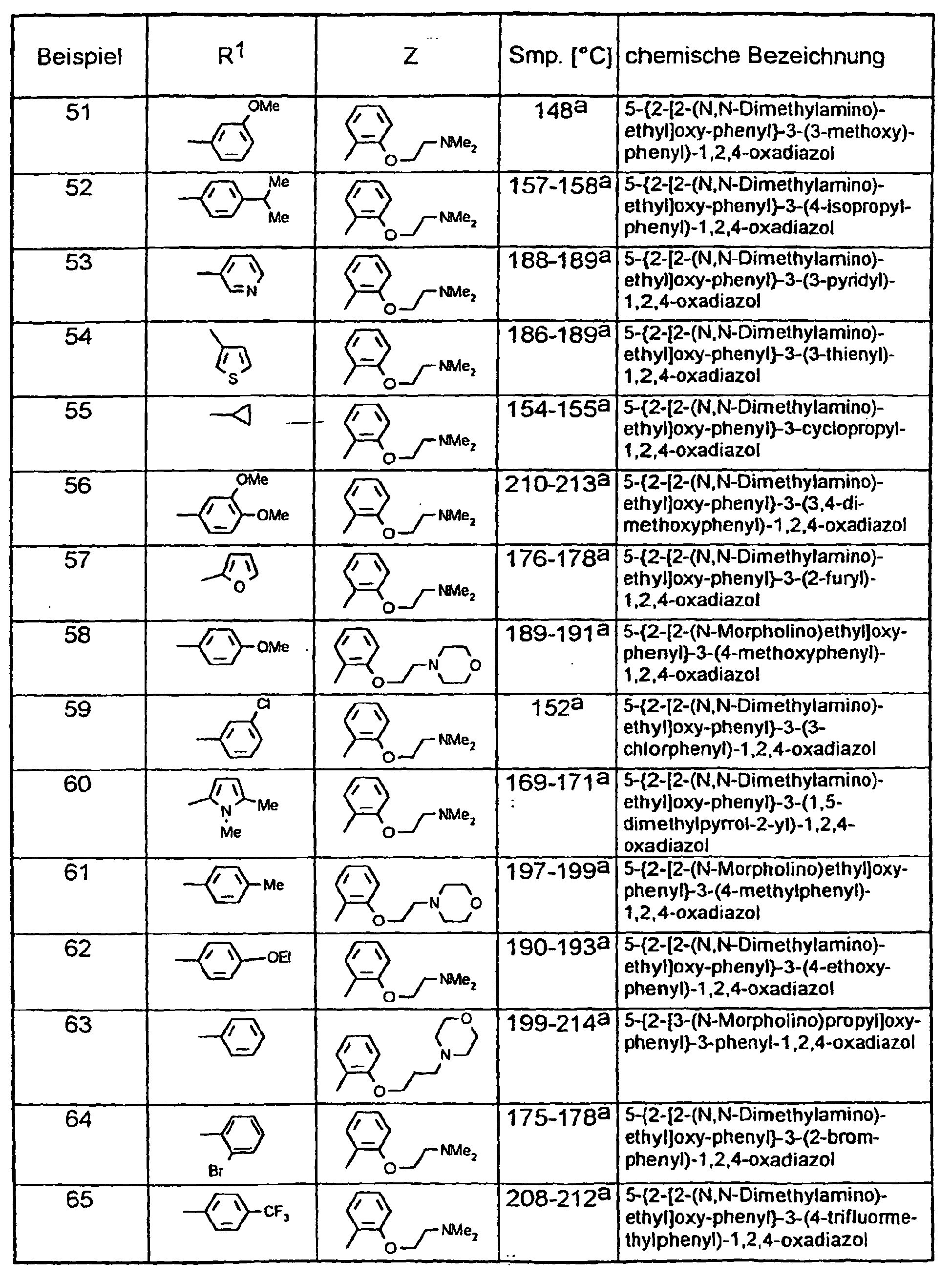

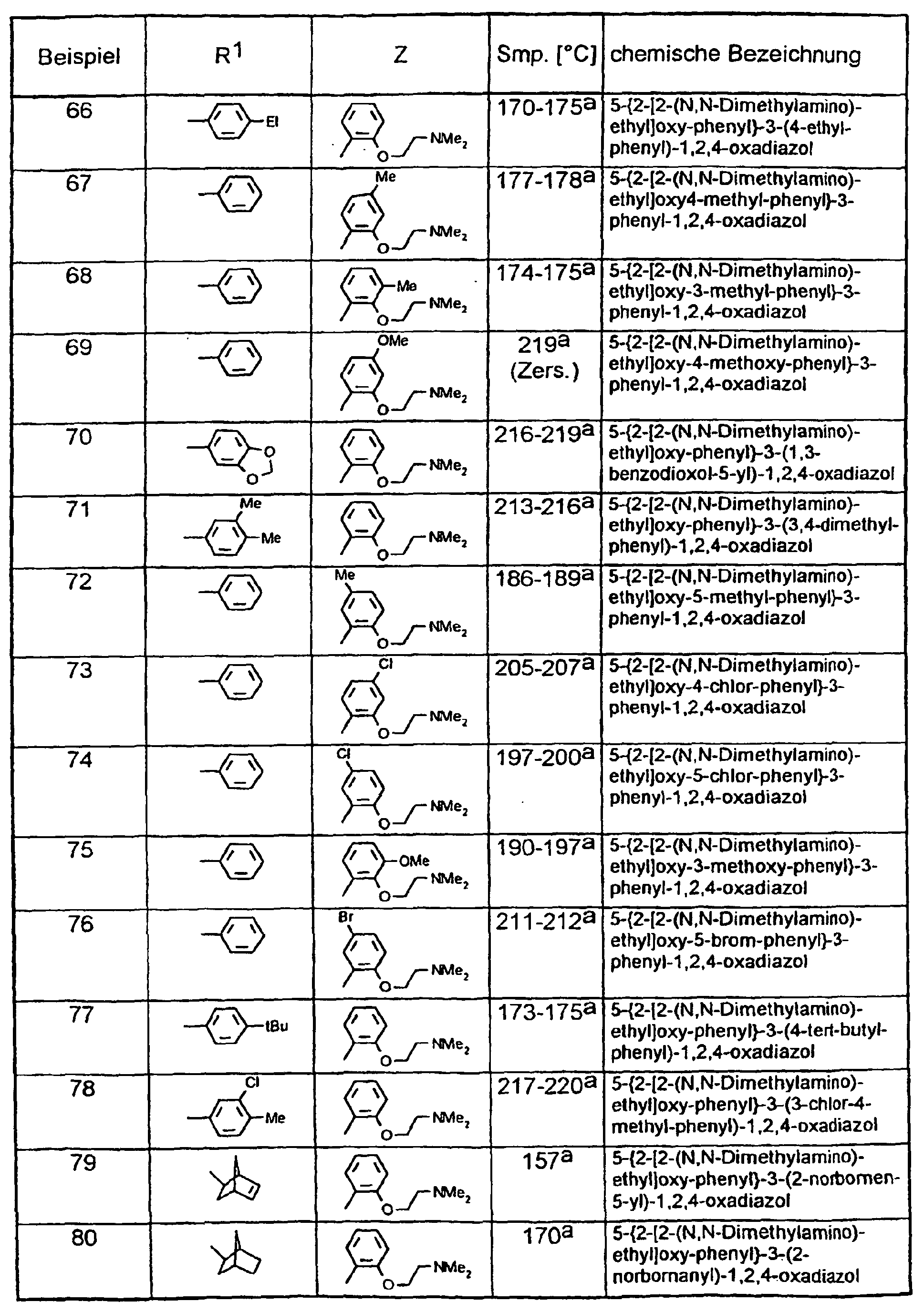

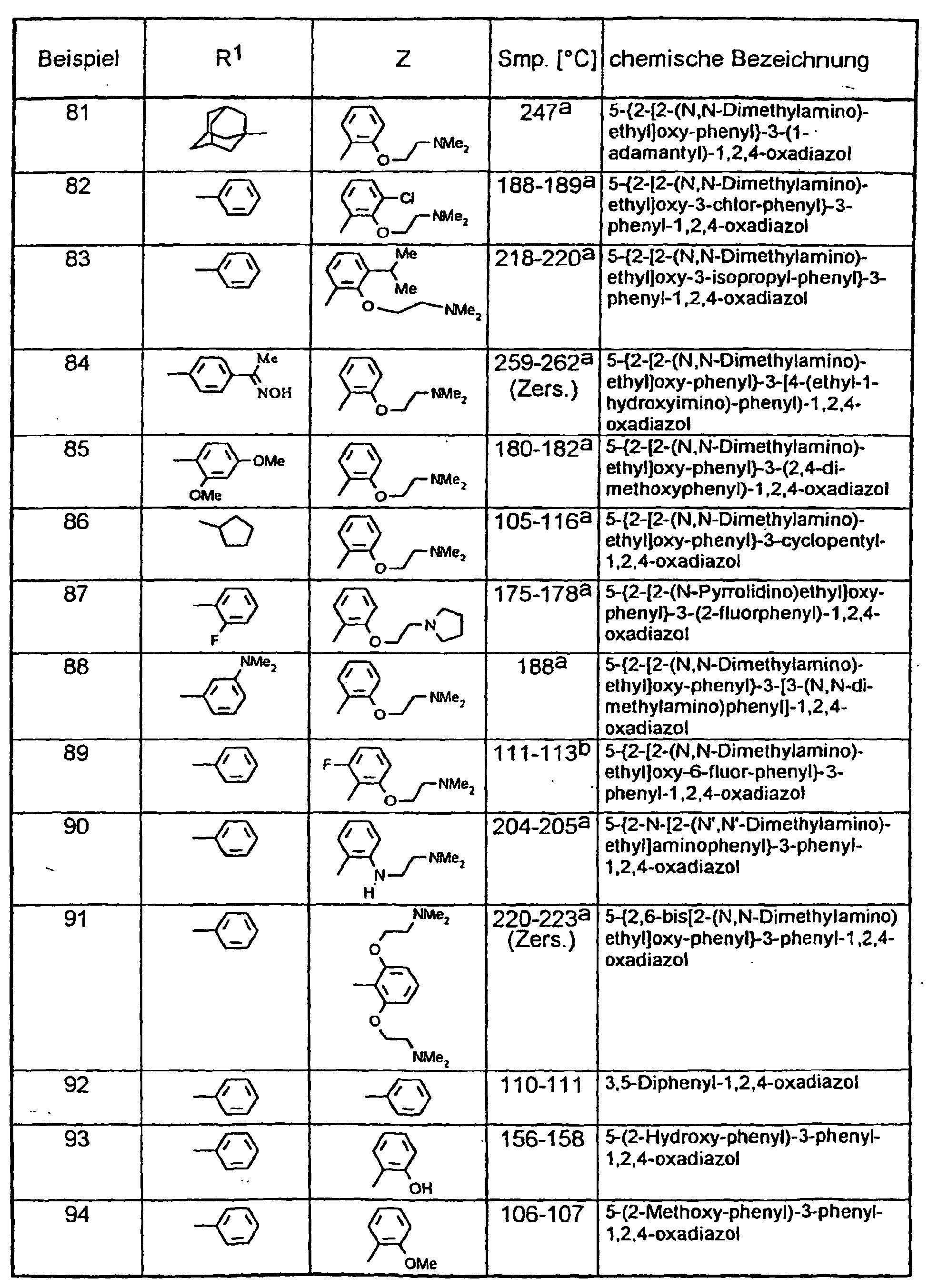

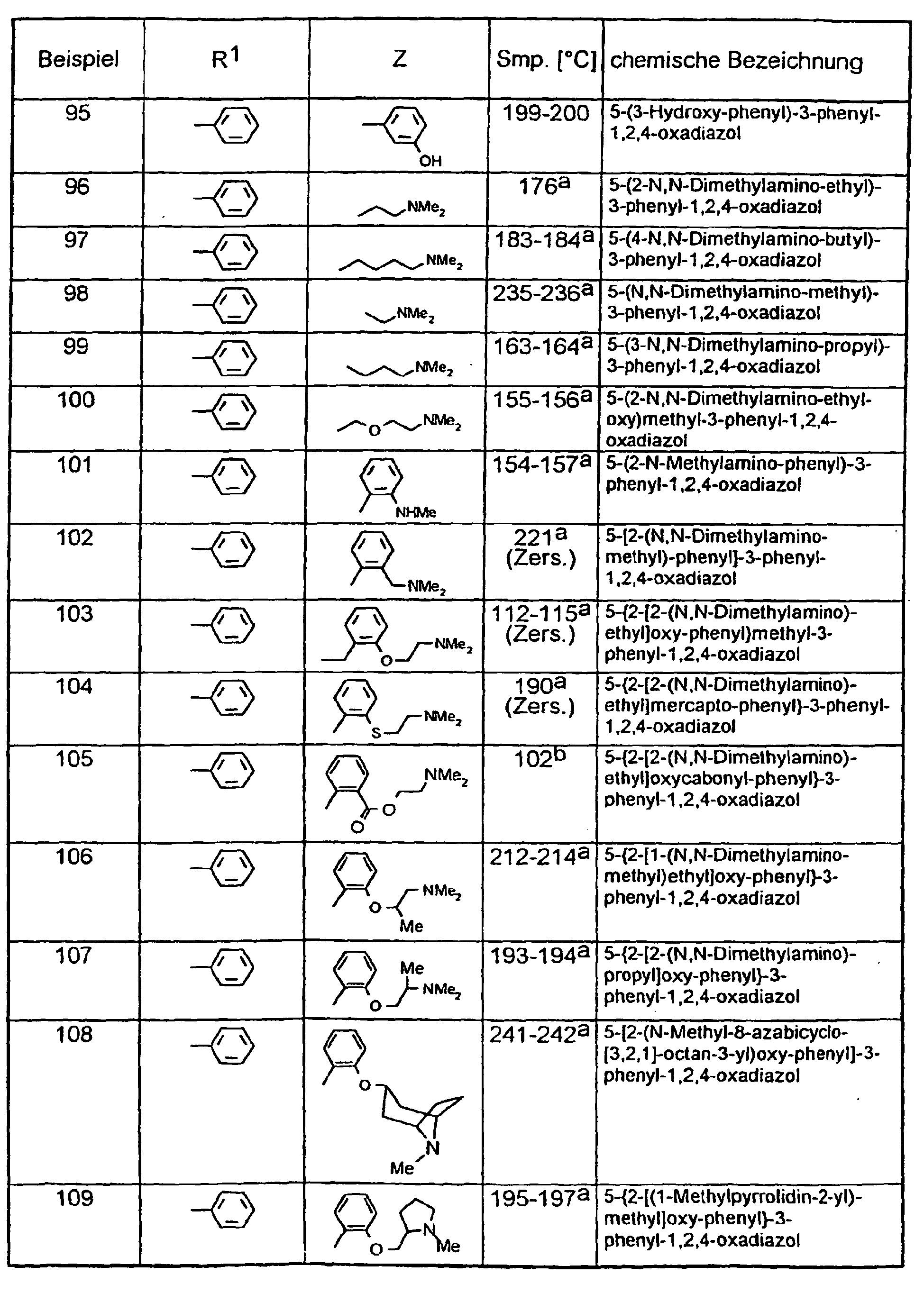

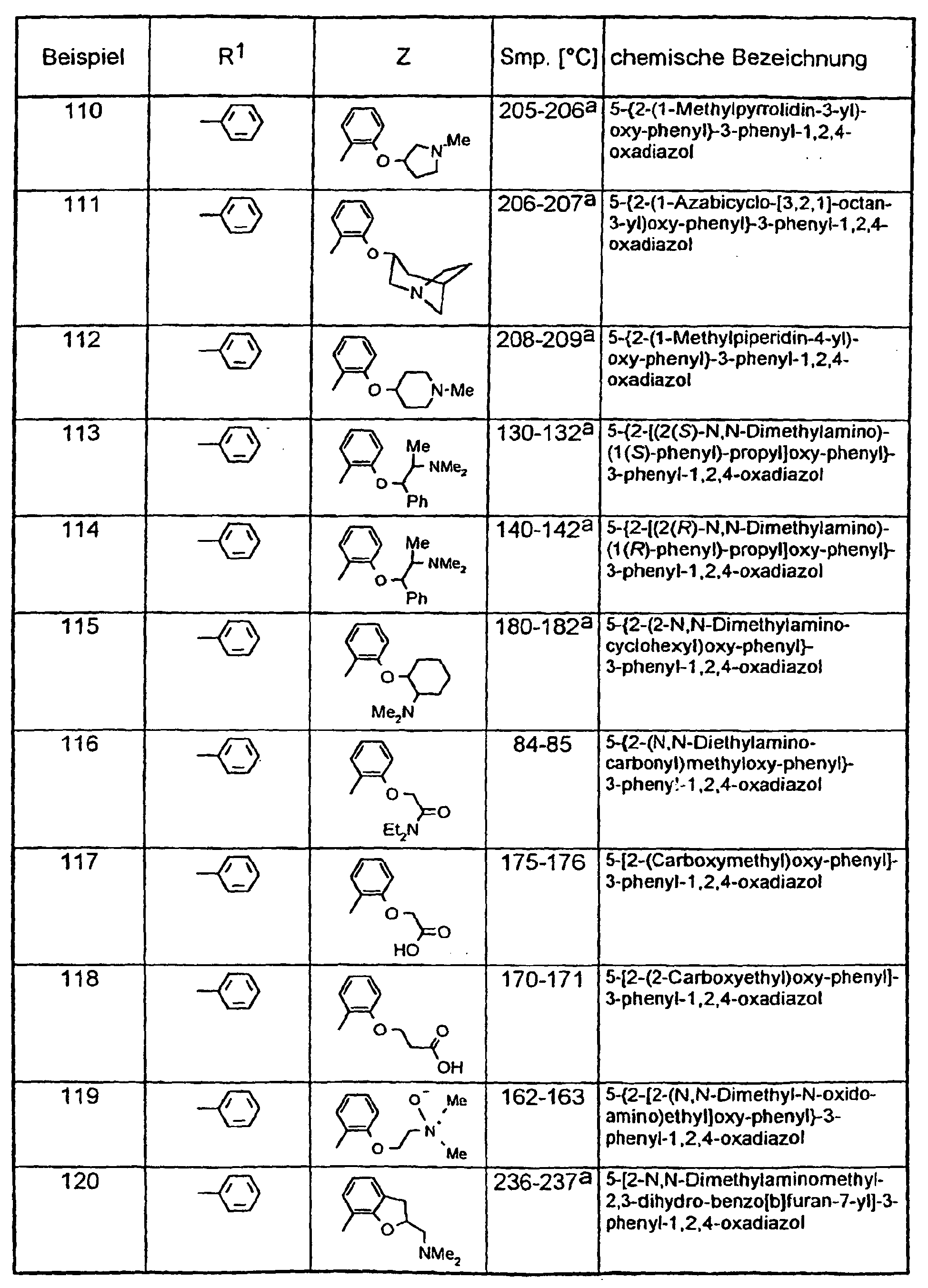

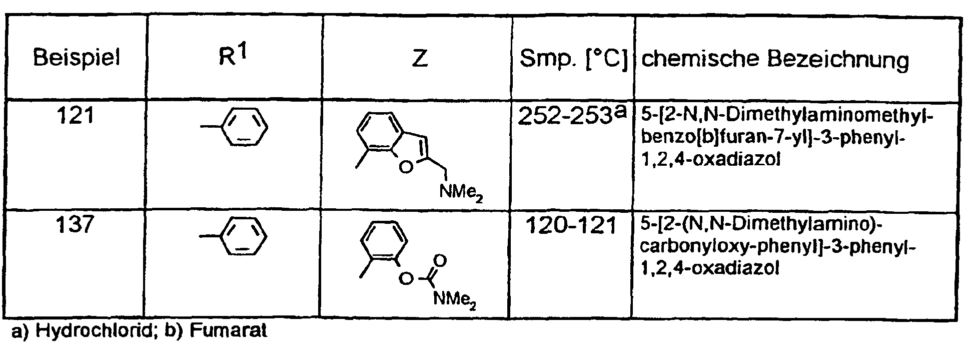

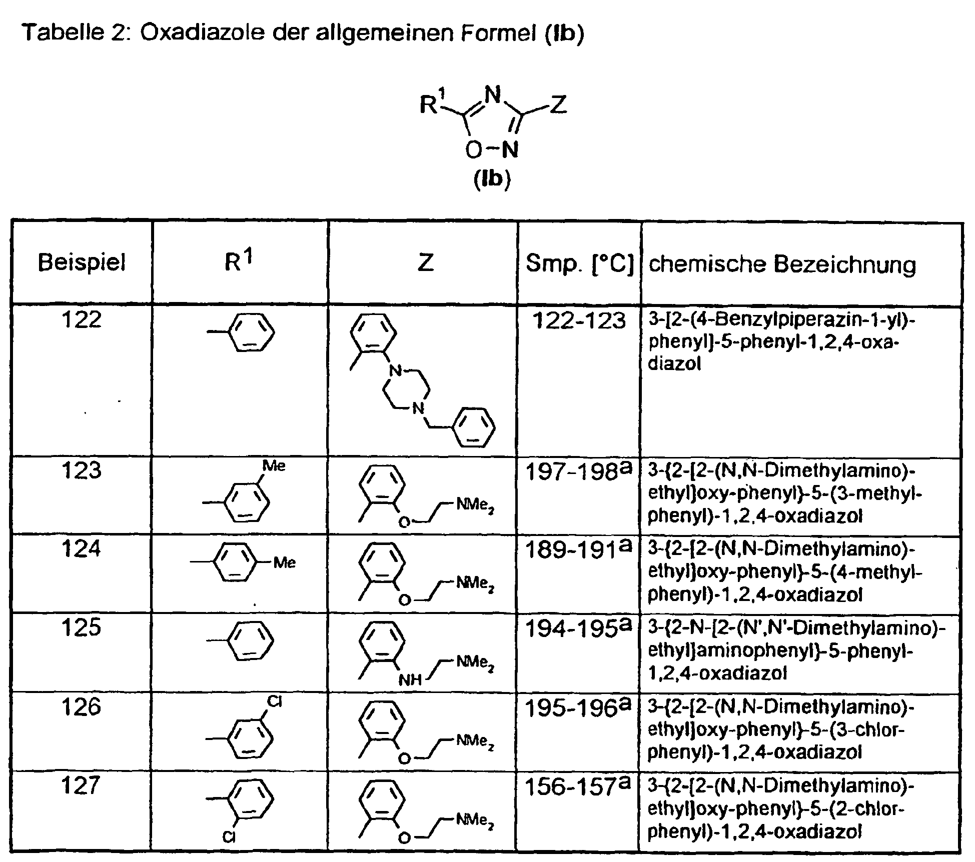

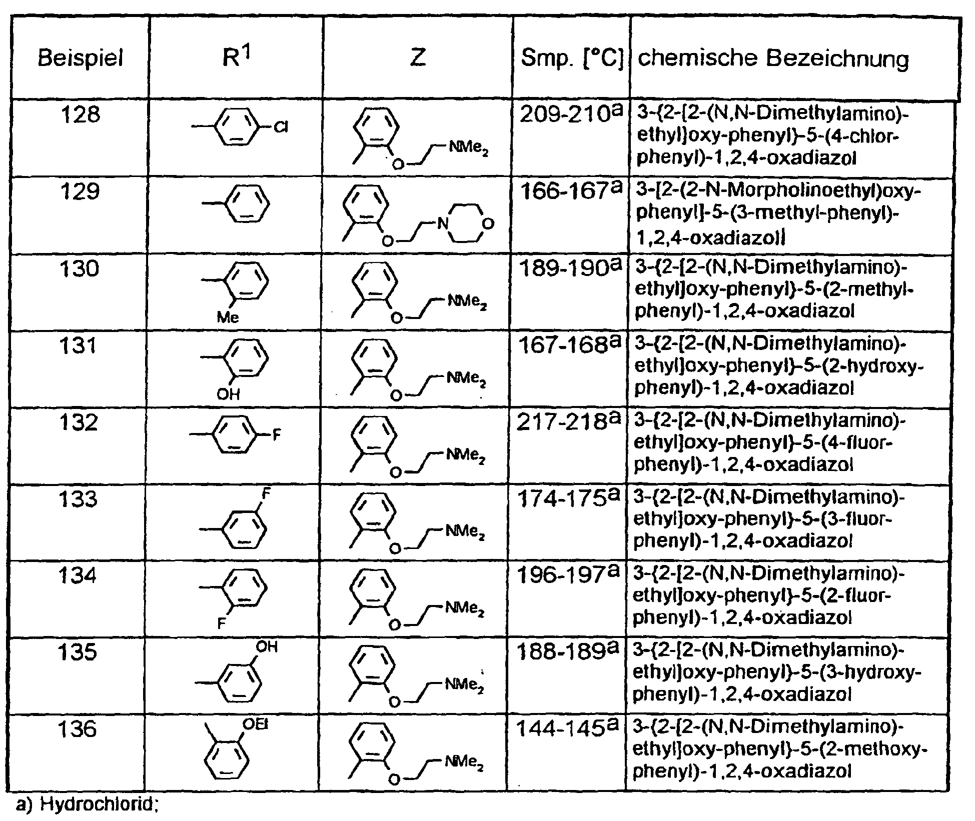

Überraschenderweise wurde gefunden, daß die erfindungsgemäßen Verbindungen eine Affinität zu oder Aktivität an den verschiedensten Rezeptortypen zeigen und eine neuroprotektive Wirkung aufweisen.

*In vitro* und *in vivo* Versuche haben gezeigt, daß die im Gehirn infolge von Hypoglykämie, Hypoxie, Anoxie, globale und fokale Ischämie, Schädel-Hirn-Trauma, Hirn-Ödem und Him-Druck auftretenden Zellschäden und Funktionsausfälle z. T. auf einer erhöhten synaptischen Aktivität und somit vermehrter Transmitterfreisetzung beruhen. Neben Glutamat sind Histamin und Serotonin als Neurotransmitter von besonderer Bedeutung. Darüberhinaus werden die Konzentrationen von insbesondere Calcium und Natrium Ionen verändert.

Es ist bekannt, daß nach systemischer Applikation von Glutamat Neuronen im Gehirn von Mäusen zerstört werden (S.M. Rothman und T.W. Olney, Trends in Neurosciences 10 (1987) 299). Dieser Befund läßt unter anderem den Schluß zu, daß Glutamat eine Rolle bei neurodegenerativen Erkrankungen spielt (R.Schwarcz und B. Meldrum, The Lancet 11 (1985) 140). Weiterhin sind Substanzen wie z.B. Quisqualinsäure, Kaininsäure, fbotensäure, Glutaminsäure, N-Methyl-D-asparaginsäure (NMDA) und α-Amino-3-hydroxy-5-methyl-4-isooxazol-propionsäure (AMPA) als exogene bzw. endogene Neurotoxine bekannt. Gehirnläsionen, die mit solchen Substanzen induziert werden können, sind vergleichbar mit jenen, welche mit Zusammenhang mit Epilepsie und anderen neurodegenerativen Erkrankungen - wie z.B. Morbus Huntington und Morbus Alzheimer - auftreten. Substanzen und lonen, welche die Aktivität der Glutamat-Rezeptoren und des mit diesem Rezeptor verbundenen lonenkanals hemmen - wie z.B. kompetitive und nicht-kompetitive Antagonisten exzitatorischer Aminosäuren - schützen Gehimzellen vor hypoxischen bzw. ischämischen Schäden. Diese Befunde zeigen, daß die Glutamat-Rezeptoren eine wichtige Rolle bei der Vermittlung des ischämischen Schadens spielen.

Der Nachweis der Wirkung am AMPA Rezeptor wurde mittels Elektrophysiologie an neuronalen Zellen (Patch-Clamp-Methode) geführt (M. L. Mayer, L. Vyklicky and G. L. Westbrook, J. Physiol. 415 (1989) 329-350).
Die Testung erfolgte bei einer Testkonzentration von 100 µM.

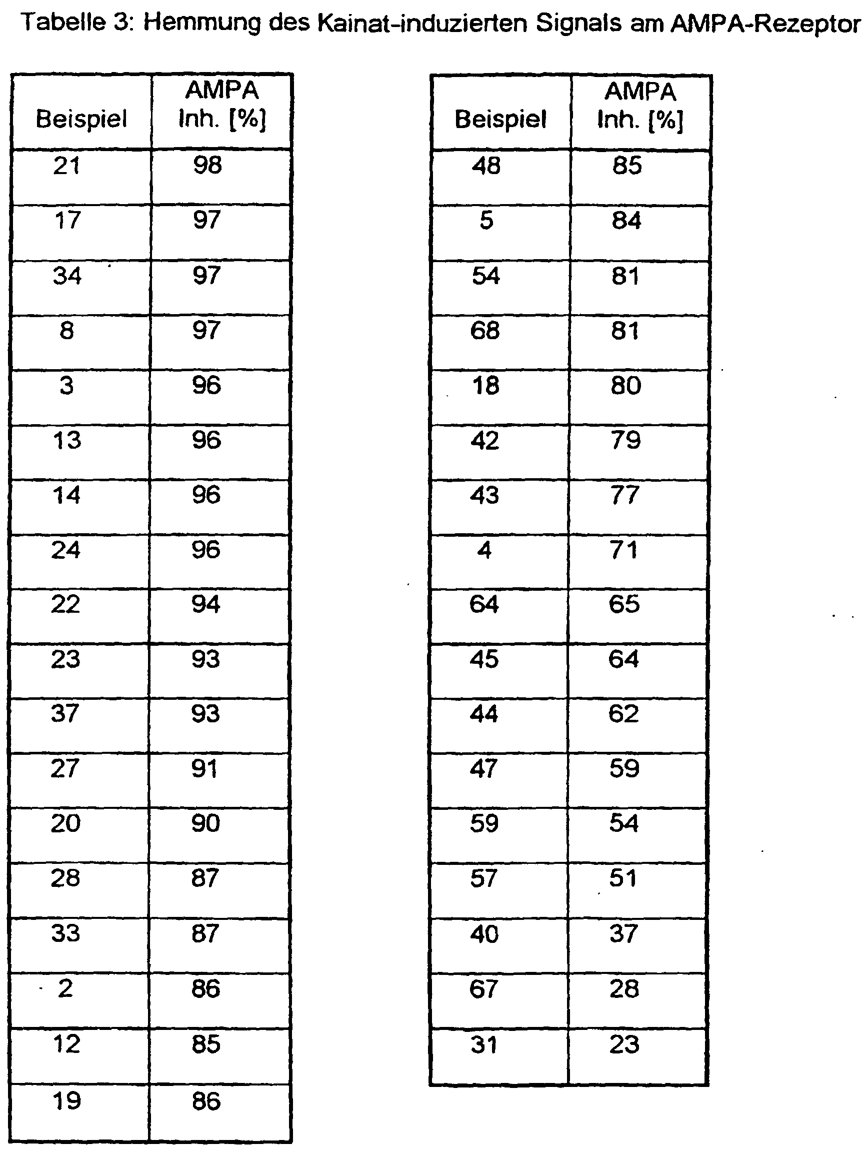

Der Nachweis der Affinität zur "Na⁺ Kanal site 2"-Bindungsstelle wurde wie von G.B. Brown (J. Neurosci. 6 (1986) 2064) beschrieben geführt.
Die Testung erfolgte typischerweise bei einer Testkonzentration von 10µM.
Die Hemmwerte sind in Tabelle 4 dargestellt.

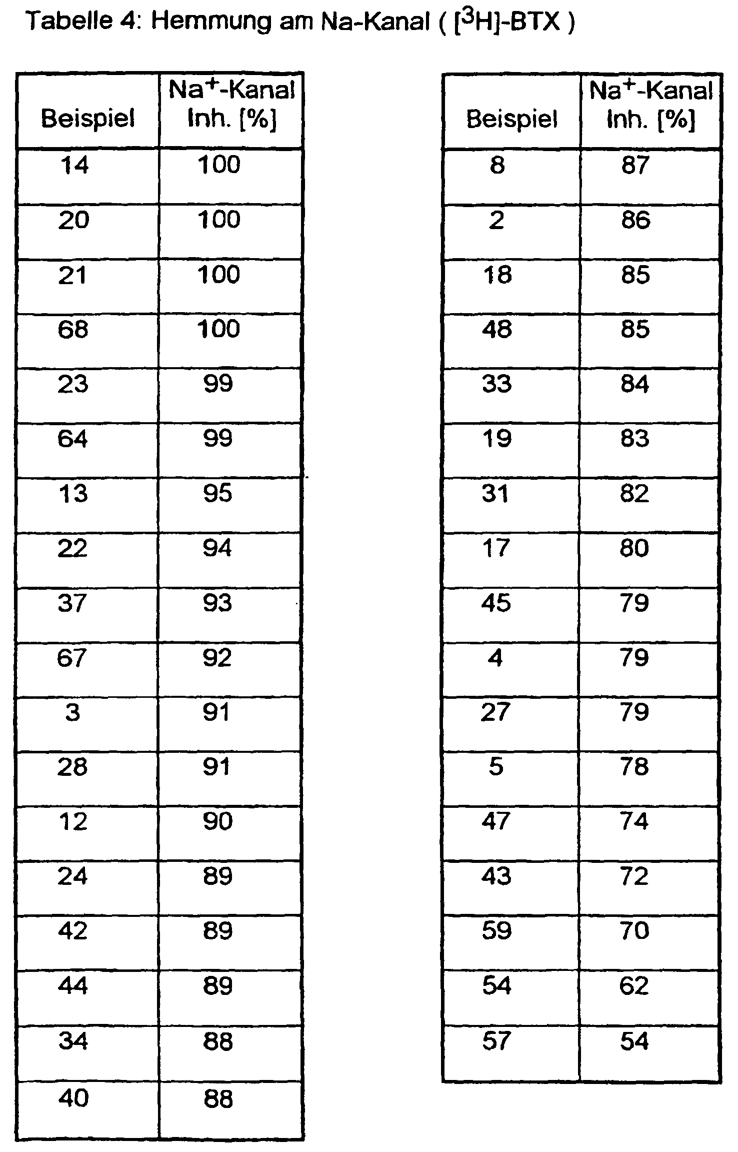

Zellschädigungen durch Hypoglykämie, Hypoxie, Anoxie und Ischämie führen durch eine Mangelversorgung zu einem reduzierten Angebot von Energieträgern wie z.B. Glucose in Neuronen.
Effekte von Histamin Rezeptor Antagonisten auf Hypoxie- und Hypoglykämieinduzierte Schädigung von 2-Deoxyglukose-Aufnahme wurde an Ratten Hippocampus Slice-Präparationen untersucht (S. Shibata und S. Watanabe, Neuroscience Letters 151 (1993) 138). Zugabe von Histamin verschlimmert die Ischämie-induzierte Abnahme der 2-Deoxyglukose-Aufnahme. Es wurde gezeigt, daß Histamin H1 Rezeptor Antagonisten die ischämie-induzierte Reduktion der 2-Deoxyglukose-Aufnahme verbessern, während Histamin H2 Rezeptor Antagonisten hierauf keinen Effekt haben. Der protektive Effekt von Histamin H1 Rezeptor Antagonisten kann durch Histamin aufgehoben werden. Diese Untersuchung legt nahe, daß Histamin Rezeptoren eine wichtige Rolle in der Ischämie-induzierten Reduktion des Glucose-Metabolismus spielen.
Exzessive neuronale Aktivität kann in Kombination mit einem massiven Anstieg von Neurotransmittem zu einer neuronalen Degeneration in Tiermodellen mit transienter cerebrater Ischämie führen (H. Benveniste, H. Drejer, A. Schousboe, N.H. Diemer, J. Neurochem. 43 (1984) 1369). Neuronale Aktivität kann durch Substanzen, die an Neurotransmitter Rezeptoren binden wie z.B. 5-Hydroxytryptamin (Serotonin) inhibitiert werden (R. Andrade, R.A. Nicoll, Soc. Neurosci. Abstr. 11 (1985) 297). Es konnte weiterhin gezeigt werden, daß Administration von 5-Hydroxytryptamin Agonisten in Tiermodellen mit Okklusion der mittleren Cerebralarterie zu einer Reduktion des Infarktvolumen führen (J.H.M. Prehn, C. Backhauß, C. Karkoutly, J. Nuglisch, B. Peruche, C. Rossberg, J. Krieglstein, Eur. J. Pharmacol. 203 (1991) 213).

Als Testsystem für den Nachweis der Affinität zu den folgenden Rezeptoren wurden Rezeptorbindungsstudien nach den folgenden Referenzen durchgeführt:
Histamin H1 (S. Dini et al. Agents and Actions 33 (1991) 181);
5-Hydroxytryptamin 1A (M.D. Hall et al., J. Neurochem. 44 (1985) 1685);
5-Hydroxytryptamin 2A (J.E. Leysen et al., Mol Pharmacol. 21 (1982) 301);
Die Testung erfolgte typischerweise bei einer Testkonzentration von 10µM.
Tabelle 5 faßt die Hemmwerte an obigen Rezeptoren zusammen:

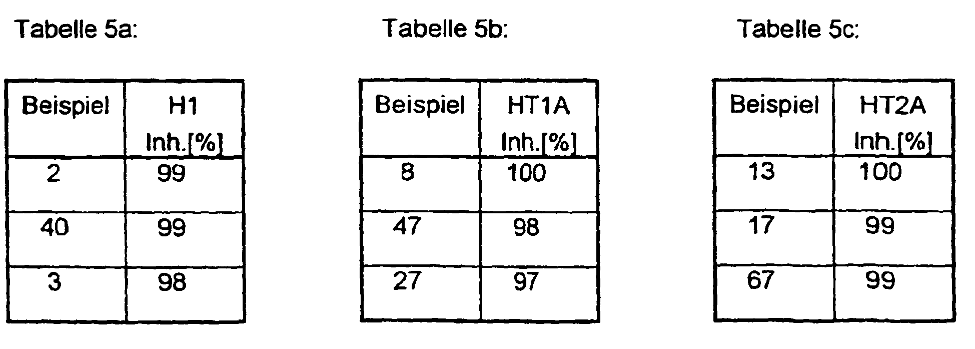

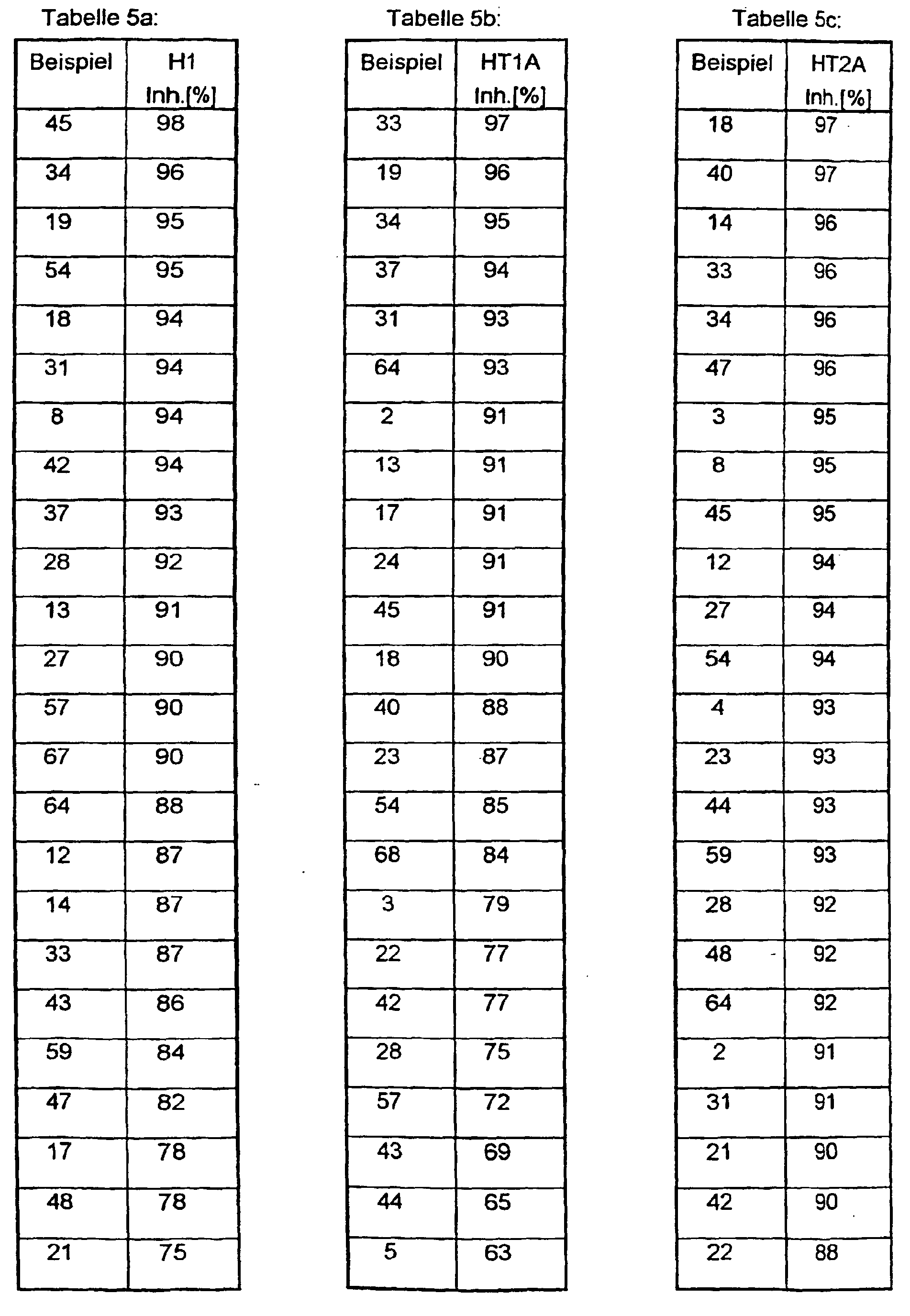

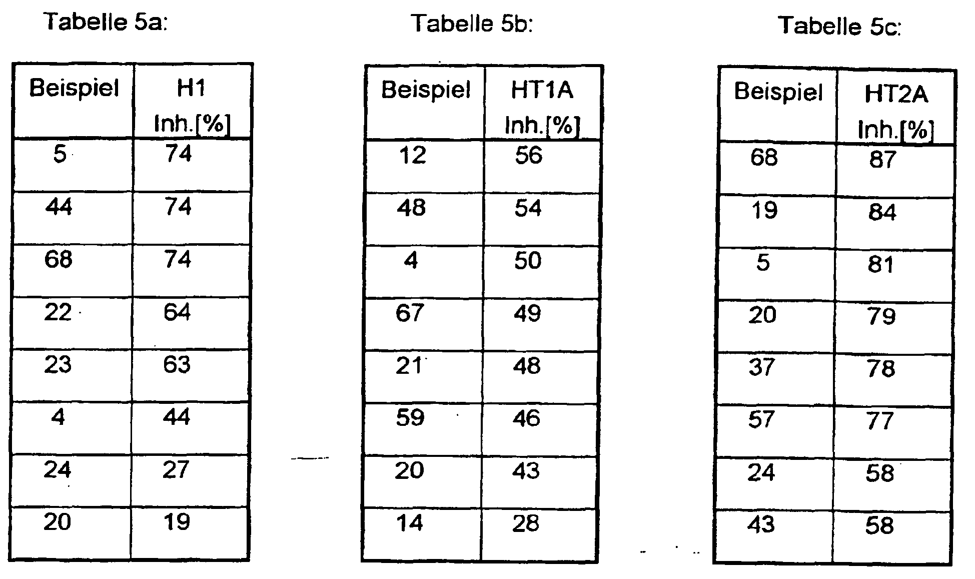

Nach H. Takahashi et al. (Stroke 26 (1995) 1676) sind Sigma Rezeptoren am Mechanismus von akuten Schädigungen nach transienter fokaler Ischämie involviert. Takahashi et al. konnten beispielsweise bei der Untersuchung eines potenten Liganden des Sigma Rezeptors im Modell der transienten fokalen Ischämie eine Reduktion des Infarktvolumens nachweisen.

Als Testsystem für den Nachweis der Affinität der erfindungsgemäßen Verbindungen zum Sigma Rezeptor wurden Rezeptorbindungsstudien nach E.W. Karbon, K. Naper, M.J. Pontecorvo, Eur. J. Pharmacol. 193 (1991)21 durchgeführt. Die Testung erfolgte typischerweise bei einer Testkonzentration von 10µM.
Die Hemmwerte sind in untenstehenderTabelle angegeben.

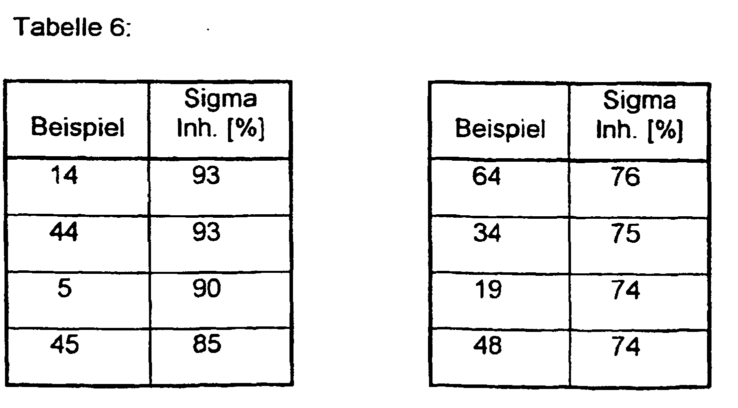

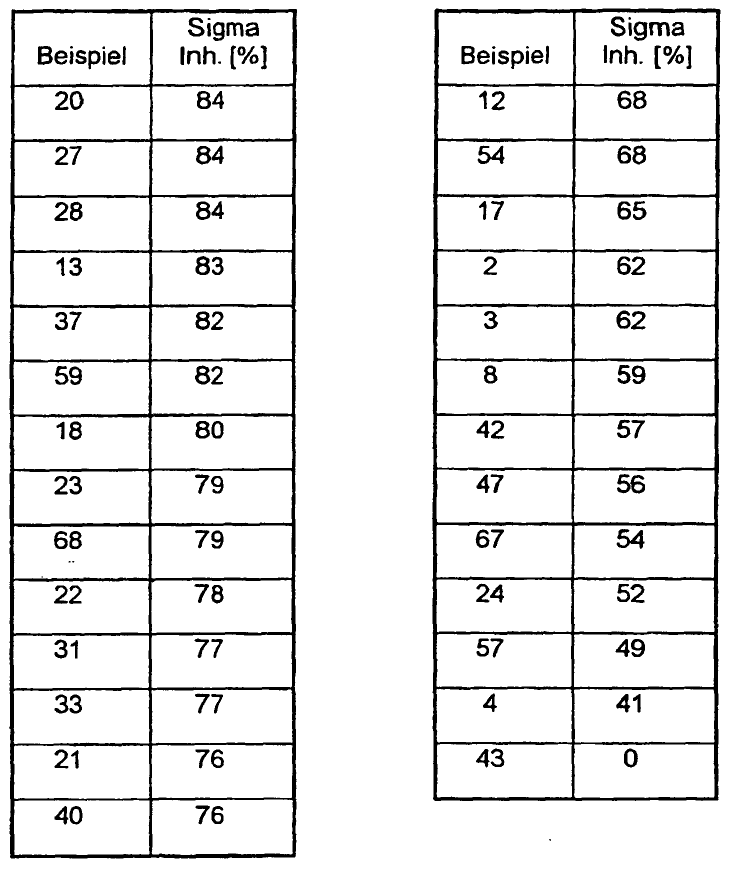

Der Nachweis für die neuroprotektive Wirkung in vivo wurde in einem Schlaganfallmodell an der Ratte durchgeführt. Dabei wird eine permanente focale cerebrale Ischämie durch die operative Occlusion der Arteria cerebri media (MCAO) induziert (basierend auf A. Tamura, D.I. Graham, J. McCulloch und G.M. Teasdale, J. Cereb. Blood Flow Metab. 1 (1981) 53-60).
Mit 5-{2-[2-(N,N-Dimethylamino)ethyl]oxy-phenyl}-3-phenyl-1,2,4-oxadiazol (Beispiel 2) gelang es, das Läsionsvolumen deutlich und signifikant zu verkleinern.

Die oben beschriebenen Ergebnisse zeigen, daß die Oxadiazolderivate der allgemeinen Formel I bei neurodegenerativen Erkrankungen sowie Gehirnischämie verschiedener Genese eingesetzt werden können. Hierunter fallen beispielsweise: Status epileptikus, Hypoglykämie, Hypoxie, Anoxie, Gehimtrauma, Gehimoedem, amyotrope laterale Sklerose, Huntington's Disease, Morbus Alzheimer, Hypotonie, Herzinfarkt, Hirndruck (erhöhter intrakranialer Druck), ischämischer und hämorrhagischer Stroke, globale cerebrale Ischämie bei Herzstillstand, Diabetische Polyneuropathie, Tinitus, perinatale Asphyxie, Psychose, Schizophrenie, Depression und Morbus Parkinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 bis 90 Gew.%, bevorzugt 0,5 bis 50 Gew.-% der Gesamtzusammensetzung liegen, d.h. in Mengen die ausreichend sind, um den unten angegebenen Dosierungsbereich zu erreichen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z. B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parenteral - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.
Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Ferner können die Verbindungen der allgemeinen Formel I bzw. deren Säureadditionssalze auch mit andersartigen Wirkstoffen kombiniert werden.

Die nachfolgenden Beispiele illustrieren die vorliegende Erfindung ohne sie jedoch in ihrem Umfang zu beschränken:

### Pharmazeutische Formulierungsbeispiele

A)

| Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.
B)

| Tabletten | pro Tablette |
|---|---|
| Wirkstoff | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.
C)

| | |
|---|---|
| Ampullenlösung | |
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Oxadiazol-Derivate der allgemeinen Formel (I) 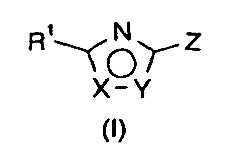 worin
X und Y Sauerstoff oder Stickstoff, wobei X und Y nicht beide gleichzeitig Sauerstoff oder Stickstoff sind,
Z ein Rest der Formel 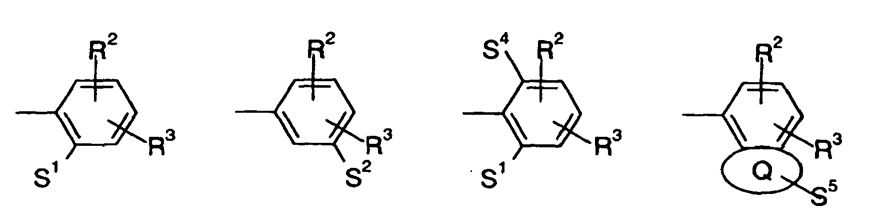
worin
S¹ ein Rest der Formel 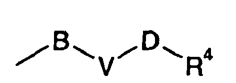
worin V Sauerstoff, Schwefel oder NR⁷ bedeutet und B und D, gleich oder verschieden, eine C₁-C₄-Alkylen-, C₂-C₄-Alkenylen- oder C₂-C₄-Alkinylen-Brücke repräsentieren, die durch =O, -OR⁷, Phenyl oder Halogen substituiert sein kann,
S¹ ein Rest der Formel 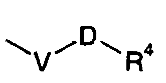
wobei V und D die oben genannte Bedeutung aufweisen,
S¹ ein Rest der Formel 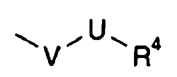
worin V die zuvor genannte Bedeutung aufweisen kann und U eine C₃-C₆-Cycloalkyl- oder Phenyl-Gruppe repräsentiert, die durch C₁-C₄-Alkyl, -OR⁷, C₆-C₁₀-Aryl oder Halogen substituiert sein kann,
S¹ ein Rest der Formel 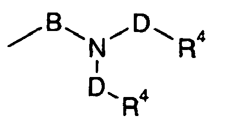
wobei B und D die oben genannte Bedeutung aufweisen und die beiden Gruppen D sowie die beiden Reste R⁴ gleich oder verschieden sind,
S¹ ein Rest der Formel 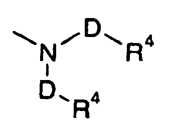
wobei D die oben genannte Bedeutung aufweist und die beiden Gruppen D sowie die beiden Reste R⁴ gleich oder verschieden sind,
S¹ ein Rest der Formel 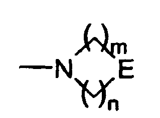 in dem E Sauerstoff, Schwefel oder NR⁷ darstellt (mit n,m=1,2 oder 3 und n+m>2) und der Rest gegebenenfalls substituiert ist durch Halogen, = O, -OR⁷ oder einen oder mehrere C₁-C₄-Alkyl-Reste;
S¹ ein Rest der Formel 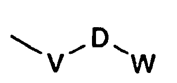
wobei V und D die oben genannte Bedeutung aufweisen und W ein gegebenenfalls durch Halogen, = O, -OR⁷, -OCOR⁷, C₁-C₄-Alkyl, C₂-C₆-Alkenyl oder C₂-C₆-Alkinyl substituierter Rest der Formel 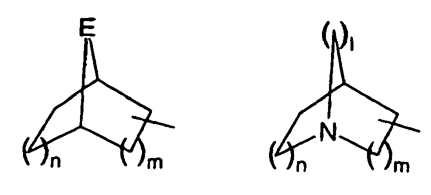 sein kann, wobei E Sauerstoff oder NR⁷ bedeutet, n,m, I = 0,1 oder 2 sein können, oder
W ein C-verknüpfter 5 oder 6-gliedriger Heterocyclus ist, der ein oder mehrere Heteroatome aus der Gruppe Stickstoff, Sauerstoff oder Schwefel enthält und der gegebenenfalls durch Benzyl oder C₁-C₄-Alkyl substituiert sein kann;
S¹ ein Rest der Formel
―V―W
wobei V und W die oben angegebenen Bedeutung aufweisen;
S² ein Rest der Formel 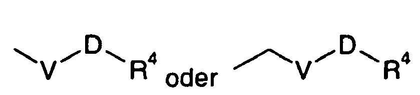
wobei V und D die oben genannte Bedeutung aufweisen,
S⁴ ein Rest der Formel 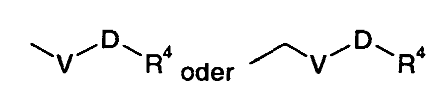
wobei V und D die oben genannte Bedeutung aufweisen,
Q ein ankondensierter, einfach oder mehrfach ungesättigter 5 oder 6- gliedriger, heterocyclischer Ring, der ein oder mehrere Heteroatome aus der Gruppe Sauerstoff, Stickstoff oder Schwefel enthalten kann und gegebenenfalls durch OR⁷, NR⁵R⁶, Halogen, CN, Nitro, CF₃, COOR⁷, C₁-C₄-Alkyl, C₂-C₄-Alkenyl oder C₂-C₄-Alkinyl substituiert sein kann;
S⁵ ein Rest der Formel
―D―R⁴
wobei D die oben genannte Bedeutung aufweist;
R¹ Benzyl oder Phenyl, wobei der Phenylring ein oder mehrfach durch einen oder mehrere der Reste Fluor, Chlor oder Brom, -C₁-C₄-Alkyl, -CF₃, -CR⁷=NOR⁷ (wobei die Reste R⁷ gleich oder verschieden sein können), -NMe₂, NEt₂, -NO₂ oder -OR⁷ substituiert sein kann,
R¹ Phenyl, das durch einen Rest der Formel 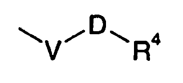 substituiert ist, mit der Maßgabe, daß V Sauerstoff oder NR⁷ ist und D eine C₁-C₄-Alkyl-Brücke repräsentiert,
R¹ ein C- oder N-verknüpfter 5 oder 6-gliedriger Heterocyclus, der ein oder mehrere Heteroatome aus der Gruppe Stickstoff, Sauerstoff oder Schwefel enthält und gegebenenfalls ein oder mehrfach durch Benzyl, Methyl, Fluor, Chlor, Brom oder Hydroxy substituiert sein kann,
R¹ Cyclopropyl, Cyclopentyl oder Cyclohexyl, die gegebenenfalls durch = O oder -OR⁷ substituiert sind,
R¹ Norbornan, Norbornen, Dicyclopropylmethyl, Adamantan oder Noradamantan, die gegebenenfalls durch Methyl substituiert sind,
R¹ ein Rest der Formel 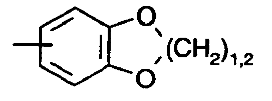
R¹ -CH=CH-Phenyl, wobei der Phenylring durch Methoxy oder Hydroxy substituiert sein kann;
R² Wasserstoff, Fluor, Chlor, Brom, C₁-C₄-Alkyloxy, C₁-C₄-Alkyl oder Hydroxy;
R³ Wasserstoff;
R⁴ Hydroxy, CN oder NR⁵R⁶;
R⁴ ein N-Oxid der Formel 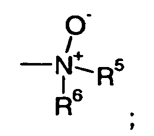
R⁵ Wasserstoff, C₁-C₃-Alkyl, Benzyl oder Phenyl;
R⁶ Wasserstoff, C₁-C₃-Alkyl, Benzyl oder Phenyl;
oder
R⁵ und R⁶ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus ein oder mehrfach durch Methyl substituiert sein kann;
R⁷ Wasserstoff, C₁-C₄-Alkyl, ein Benzyl- oder Phenyl-Rest, wobei der Phenylring gegebenenfalls ein- oder mehrfach substituiert ist durch OH oder OCH₃,
bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

2. Verbindungen der allgemeinen Formel (I) gemäß Anspruch 1, worin
X und Y Sauerstoff oder Stickstoff, wobei X und Y nicht beide gleichzeitig Sauerstoff oder Stickstoff sind,
Z ein Rest der Formel 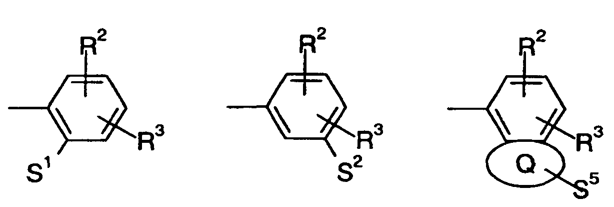
worin
S¹ ein Rest der Formel 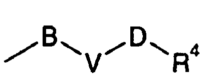
worin V Sauerstoff, Schwefel oder NR⁷ bedeutet, B -CH₂- ist und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
S¹ ein Rest der Formel 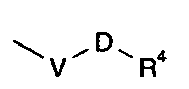
worin V und D die zuvor genannte Bedeutung aufweisen,
S¹ Piperazin-1-yl, 4-Methyl-piperazin-1-yl oder 4-Benzyl-piperazin-1-yl;
S¹ ein Rest der Formel 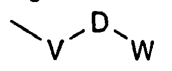
wobei V und D die oben genannte Bedeutung aufweisen und W ein gegebenenfalls durch C₁-C₄-Alkyl substituierter Rest der Formel 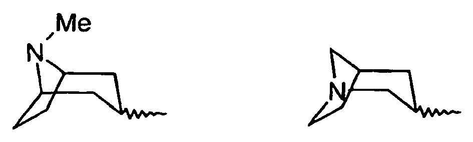 sein kann,
oder
W ein C-verknüpfter 5 oder 6-gliedriger Stickstoffheterocyclus ist, der gegebenenfalls durch Benzyl oder C₁-C₄-Alkyl substituiert sein kann;
S¹ ein Rest der Formel
―V―W
wobei V und W die oben angegebenen Bedeutung aufweisen;
S² ein Rest der Formel
―(CH₂)_{0,1}―O―(CH₂)_{2,3}―R⁴
Q ein ankondensierter, einfach oder mehrfach ungesättigten 5 oder 6- gliedriger Ring, der ein Heteroatom aus der Gruppe Sauerstoff, Stickstoff oder Schwefel enthalten kann;
S⁵ ein Rest der Formel
―D―R⁴
wobei D die oben genannte Bedeutung aufweist;
R¹ Cyclopropyl, Cyclopentyl, Benzyl oder Phenyl, wobei der Phenylring ein oder mehrfach durch einen oder mehrere der Reste Fluor, Chlor, Brom, C₁-C₄-Alkyl, -CF₃, -CMe=NOH, -NMe₂, -NO₂ oder -OR⁷ substituiert sein kann,
R¹ Phenyl, das durch einen Rest der Formel
-O-(CH₂)_{2,3}- R⁴
substituiert ist,
R¹ Furan, Thiophen, Pyridin oder Pyrrol, die gegebenenfalls ein- oder mehrfach durch Methyl substituiert sein können,
R¹ Norbornan, Norbornen, Adamantan oder Noradamantan,
R¹ -CH=CH-Phenyl, wobei der Phenylring durch Hydroxy substituiert sein kann;
R² Wasserstoff, Fluor, Chlor, Brom, C₁-C₄-Alkyloxy, C₁-C₄-Alkyl oder Hydroxy;
R³ Wasserstoff;
R⁴ N-Morpholinyl, N-Pyrrolidinyl, N-Piperidinyl, N-Piperazinyl, 4-Methylpiperazin-1-yl oder 4-Benzyl-piperazin-1-yl;
R⁴ CN, NR⁵R⁶ oder ein N-Oxid der Formel 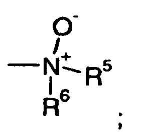
R⁵ Wasserstoff, C₁-C₃-Alkyl, Benzyl oder Phenyl;
R⁶ Wasserstoff, C₁-C₃-Alkyl, Benzyl oder Phenyl;
R⁷ Wasserstoff, Methyl, Ethyl, Propyl, iso-Propyl, Butyl, tert.-Butyl, ein Benzyl- oder Phenyl-Rest, wobei der Phenylring gegebenenfalls ein- oder mehrfach substituiert ist durch OH oder OCH₃, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

3. Verbindungen der allgemeinen Formel (I) gemäß Anspruch 1, worin
X und Y Sauerstoff oder Stickstoff, wobei X und Y nicht beide gleichzeitig Sauerstoff oder Stickstoff sind,
Z ein Rest der Formel 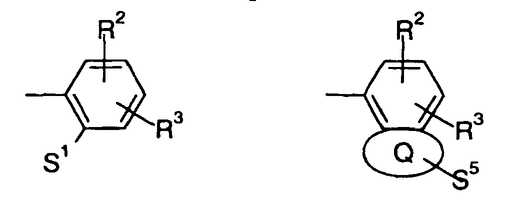 worin
S¹ ein Rest der Formel 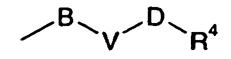
worin V Sauerstoff, Schwefel oder NR⁷ bedeutet, B -CH₂- ist und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
S¹ ein Rest der Formel 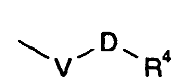 worin V und D die zuvor genannte Bedeutung aufweisen,
S¹ Piperazin-1-yl, 4-Methyl-piperazin-1-yl oder 4-Benzyl-piperazin-1-yl;
S¹ ein Rest der Formel 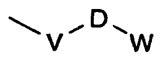
wobei V und D die oben genannte Bedeutung aufweisen und W ein gegebenenfalls durch C₁-C₄-Alkyl substituierter Rest der Formel 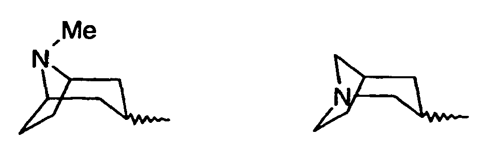 sein kann,
oder
W ein C-verknüpfter 5 oder 6-gliedriger Stickstoffheterocyclus ist, der gegebenenfalls durch Benzyl oder C₁-C₄-Alkyl substituiert sein kann;
S¹ ein Rest der Formel
-V-W
wobei V und W die oben angegebenen Bedeutungen aufweisen;
Q ein ankondensierter, einfach oder mehrfach ungesättigter 5 oder 6- gliedriger, heterocyclischer Ring, der ein Heteroatom aus der Gruppe Sauerstoff, Stickstoff oder Schwefel enthalten kann;
S⁵ ein Rest der Formel
―D―R⁴
wobei D die oben genannte Bedeutung aufweist;
R¹ Cyclopropyl, Cyclopentyl oder Phenyl, wobei der Phenylring ein oder mehrfach durch einen oder mehrere der Reste Fluor, Chlor, Brom, C₁-C₄-Alkyl, -CF₃, -CMe=NOH, -NMe₂, -NO₂ oder -OR⁷ substituiert sein kann,
R¹ Furan, Thiophen, Pyridin oder Pyrrol, die gegebenenfalls ein- oder mehrfach durch Methyl substituiert sein können,
R¹ Norboman, Norbomen, Adamantan oder Noradamantan,
R¹ -CH=CH-Phenyl, wobei der Phenylring durch Hydroxy substituiert sein kann;
R² Wasserstoff, Fluor, Chlor, Brom, C₁-C₄-Alkyloxy, C₁-C₄-Alkyl oder Hydroxy;
R³ Wasserstoff;
R⁴ CN, NR⁵R⁶ oder ein N-Oxid der Formel 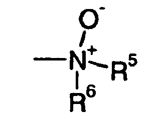
R⁴ N-Morpholinyl, N-Pyrrolidinyl, N-Piperidinyl, N-Piperazinyl, 4-Methylpiperazin-1-yl oder 4-Benryl-piperazin-1-yl;
R⁵ Wasserstoff, C₁-C₃-Alkyl, Benzyl oder Phenyl;
R⁶ Wasserstoff, C₁-C₃-Alkyl, Benzyl oder Phenyl;
R⁷ Wasserstoff, Methyl, Ethyl, Propyl, iso-Propyl, Butyl, tert.-Butyl, ein Benzyl- oder Phenyl-Rest, wobei der Phenylring gegebenenfalls ein- oder mehrfach substituiert ist durch OH oder OCH₃, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

4. Verbindungen der allgemeinen Formel (I) gemäß Anspruch 1, worin
X und Y Sauerstoff oder Stickstoff, wobei X und Y nicht beide gleichzeitig Sauerstoff oder Stickstoff sind,
Z ein Rest der Formel 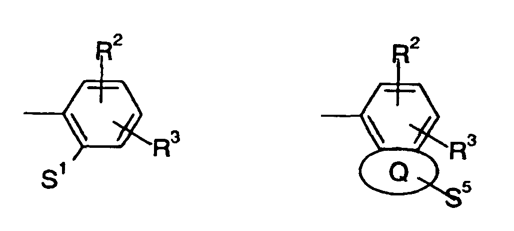
worin
S¹ ein Rest der Formel 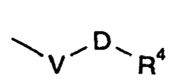
worin V Sauerstoff, Schwefel oder NR⁷ bedeutet und D eine der Gruppen -CH₂-, -CH₂-CH₂-, -CH₂-CH₂-CH₂-, -CH₂-C(CH₃)H-, -CH₂-CO-, -CH₂-CH₂-CO- sein kann;
S¹ Piperazin-1-yl, 4-Methyl-piperazin-1-yl oder 4-Benzyl-piperazin-1-yl;
S¹ ein Rest der Formel 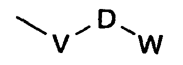
wobei V und D die oben genannte Bedeutung aufweisen und W ein Rest der Formel 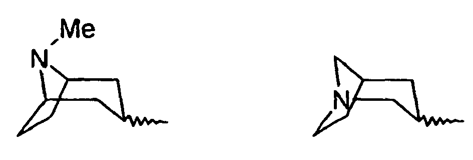 oder
W ein C-verknüpfter 5 oder 6-gliedriger Stickstoffheterocyclus ist, der gegebenenfalls durch Methyl substituiert sein kann;
S¹ ein Rest der Formel
―V―W
wobei V und W die oben angegebenen Bedeutungen aufweisen;
Q ein ankondensierter, einfach oder mehrfach ungesättigter 5-gliedriger, heterocyclischer Ring, der ein Heteroatom aus der Gruppe Sauerstoff oder Stickstoff enthalten kann;
S⁵ ein Rest der Formel
―D―R⁴
wobei D die oben genannte Bedeutung aufweist;
R¹ Phenyl, das gegebenenfalls ein- oder mehrfach durch einen oder mehrere der Reste Fluor, Chlor, Brom, Methyl, Ethyl, n-Propyl, i-Propyl, n-Butyl, tert.-Butyl, -CF₃ oder -OR⁷ substituiert sein kann,
R¹ Furan, Thiophen oder Pyridin, die gegebenenfalls ein- oder mehrfach durch Methyl substituiert sein können,
R² Wasserstoff, Fluor, Chlor, Brom, Methyl, Methyloxy oder Hydroxy;
R³ Wasserstoff;
R⁴ NR⁵R⁶ oder ein N-Oxid der Formel 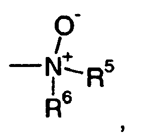
R⁴ N-Morpholinyl, N-Pyrrolidinyl, N-Piperidinyl, N-Piperazinyl, 4-Methylpiperazin-1-yl oder 4-Benzyl-piperazin-1-yl;
R⁵ Wasserstoff, Methyl, Ethyl, n-Propyl, i-Propyl, Benzyl oder Phenyl;
R⁶ Wasserstoff, Methyl, Ethyl, n-Propyl, i-Propyl, Benzyl oder Phenyl;
R⁷ Wasserstoff; Methyl oder Ethyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

5. Verbindungen der allgemeinen Formel (I) gemäß Anspruch 1, worin
X und Y Sauerstoff oder Stickstoff, wobei X und Y nicht beide gleichzeitig Sauerstoff oder Stickstoff sind,
Z ein Rest der Formel 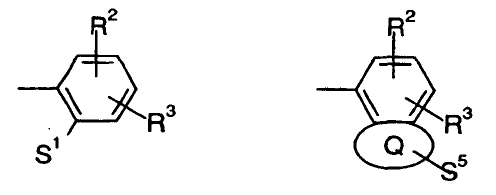
worin
S¹ ein Rest der Formel 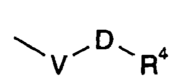
worin V Sauerstoff bedeutet und D eine der Gruppen -CH₂-, -CH₂-CH₂-, -CH₂-C(CH₃)H-, -CH₂-CH₂-CO- sein kann;
S¹ N-Piperazinyl, 4-Benzyl-piperazin-1-yl;
S¹ ein Rest der Formel 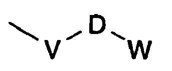
wobei V und D die oben genannte Bedeutung aufweisen und W ein Rest der Formel 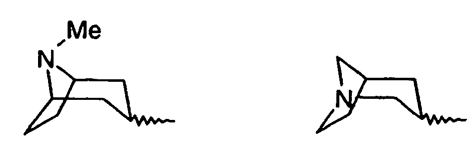 oder
W ein C-verknüpfter 5 oder 6-gliedriger Stickstoffheterocyclus ist, der gegebenenfalls durch Methyl substituiert sein kann;
S¹ ein Rest der Formel
―V―W
wobei V und W die oben angegebenen Bedeutungen aufweisen;
Q ein ankondensierter, einfach oder mehrfach ungesättigter 5-gliedriger, heterocyclischer Ring, der als Heteroatom Sauerstoff enthält;
S⁵ ein Rest der Formel
―D―R⁴
wobei D die oben genannte Bedeutung aufweist;
R¹ Phenyl, das ein oder mehrfach durch einen oder mehrere der Reste Fluor, Chlor, Brom, Methyl, -CF₃, Hydroxy, Methyloxy oder Ethyloxy substituiert sein kann,
R¹ Furan, Thiophen oder Pyridin;
R² Wasserstoff, Fluor, Chlor oder Methyl;
R³ Wasserstoff;
R⁴ NR⁵R⁶,
R⁴ N-Morpholinyl, N-Pyrrolidinyl, N-Piperidinyl oder 4-Methylpiperazin-1-yl;
R⁵ Wasserstoff, Methyl, Ethyl, n-Propyl, i-Propyl, Benzyl oder Phenyl;
R⁶ Wasserstoff, Methyl, Ethyl, n-Propyl, i-Propyl, Benzyl oder Phenyl, bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

6. Verbindungen der allgemeinen Formel (I) gemäß Anspruch 1, worin
X und Y Sauerstoff oder Stickstoff, wobei X und Y nicht beide gleichzeitig Sauerstoff oder Stickstoff sind,
Z ein Rest der Formel 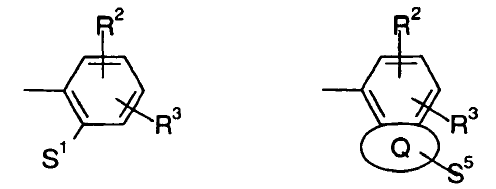
worin
S¹ einer der Reste
-O-CH₂-CH₂-R⁴, -O-CH₂-C(CH₃)H-R⁴, -O-C(CH₃)H-CH₂-R⁴ oder-CH₂-CH₂-CO-R⁴ sein kann;
S¹ 4-Benzyl-piperazin-1-yl;
S¹ einer der Reste
-O-CH₂-W oder -O-W sein kann, wobei
W ein C-verknüpfter 5 oder 6-gliedriger Stickstoffheterocyclus ist, der gegebenenfalls durch Methyl substituiert sein kann;
Q ein ankondensierter, einfach oder mehrfach ungesättigter 5-gliedriger, heterocyclischer Ring, der als Heteroatom Sauerstoff enthält;
S⁵ ein Rest der Formel -CH₂-R^{4;}
R¹ Phenyl, das ein- oder mehrfach durch einen oder mehrere der Reste Fluor, Chlor, Brom, Methyl, -CF₃, Hydroxy, Methyloxy oder Ethyloxy substituiert sein kann,
R¹ Thiophen;
R² Wasserstoff, Fluor, Chlor oder Methyl;
R³ Wasserstoff;
R⁴ NR⁵R⁶,
R⁴ N-Pyrrolidinyl oder N-Piperidinyl;
R⁵ Wasserstoff, Methyl, Ethyl, i-Propyl, Benzyl oder Phenyl;
R⁶ Wasserstoff, Methyl, Ethyl, i-Propyl, Benzyl oder Phenyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

7. Verwendung einer Verbindung der allgemeinen Formel (I) gemäß Anspruch 1 bis 6 zur Herstellung eines Arzneimittels zur Behandlung von neurodegenerativen Erkrankungen sowie Gehirnischämie verschiedener Genese.

8. Pharmazeutische Zubereitungen, enthaltend als Wirkstoff eine oder mehrere Verbindungen der allgemeinen Formel (I) gemäß den Ansprüchen 1 bis 6 oder deren physiologisch verträgliche Säureadditionssalze in Kombination mit üblichen Hilfs- und/oder Trägerstoffen.

9. Verfahren zur Herstellung von Oxadiazol-Derivaten der allgemeinen Formel (I), 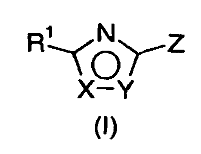 worin
a) X Stickstoff und Y Sauerstoff bedeuten und R' und Z die in den Ansprüchen 1 bis 6 genannte Bedeutung aufweisen,
**dadurch gekennzeichnet, daß** ein Oxadiazol-Derivat der allgemeinen Formel (II) 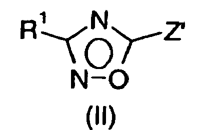 worin R¹ die in den Anprüchen 1 bis 6 genannte Bedeutung hat und Z' einen Rest der Formel 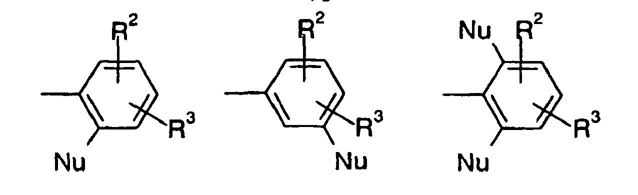 darstellt,
Nu eine nukleophile Gruppe der Formel VH oder B-VH repräsentiert wobei V, B, R² und R³ die in den Ansprüchen 1 bis 6 genannte Bedeutung aufweisen,
unter basischen Reaktionsbedingungen mit Elektrophilen der allgemeinen
Formel
L-D-R⁴
wobei L eine Abgangsgruppe wie beispielsweise Chlor, Brom, lod, Methansulfonyl, Trifluormethansulfonyl oder auch p-Toluolsulfonyl darstellt und D und R⁴ eine der in den Ansprüchen 1 bis 6 genannten Bedeutungen aufweisen,
umgesetzt wird.
b) X Sauerstoff und Y Stickstoff bedeuten und R¹ und Z die in den Ansprüchen 1 bis 6 genannte Bedeutung aufweisen,
**dadurch gekennzeichnet, daß** ein aromatisches Hydroxylamin der allgemeinen Formel (III) 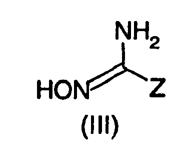 wobei
Z die in den Ansprüchen 1 bis 6 dargestellte Bedeutung aufweist,
mit Carbonsäure-Derivaten der allgemeinen Formel 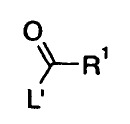
wobei L' beispielsweise Chlor, Brom oder Alkyloxy bedeutet und R¹ die zuvor genannte Bedeutungen aufweist,
umgesetzt wird.

10. Verfahren zur Herstellung von Oxadiazol-Derivaten der allgemeinen Formel (I), 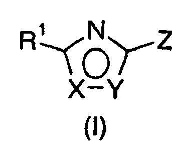 worin X, Y, R¹ und Z die in den Anprüchen 1 bis 6 genannte Bedeutung aufweisen,
**dadurch gekennzeichnet, daß** ein Oxadiazol-Derivat der allgemeinen Formel (IV) 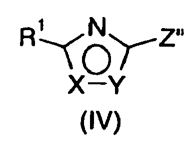 worin Z" einen Rest der Formel 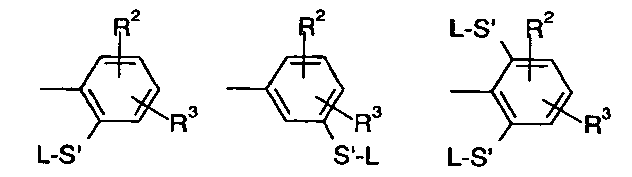 darstellt, wobei S' einen Rest der Formel
-B-V-D- oder -V-D-
repräsentiert, L eine Abgangsgruppe wie beispielsweise Chlor, Brom, lod, Methansulfonyl, Trifluormethansulfonyl oder p-Toluolmethansulfonyl bedeutet und B, V, D, R¹, R² und R³ die in den Ansprüchen 1 bis 6 genannte Bedeutung aufweisen,
mit einem Nukleophil der Formel
H-R⁴
wobei R⁴ die in den Ansprüchen 1 bis 6 angegebenen Bedeutungen aufweisen kann,
umgesetzt wird.

## Claims

1. Oxadiazole derivatives of general formula (I) 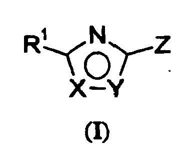 wherein
X and Y denote oxygen or nitrogen, but X and Y do not both simultaneously denote oxygen or nitrogen;
Z denotes a group of formula 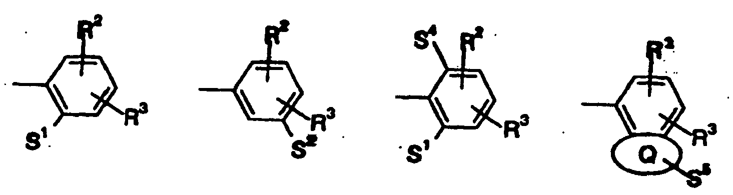
wherein
S¹ denotes a group of formula 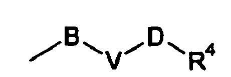
wherein V represents oxygen, sulphur or NR⁷ and B and D, which may be identical or different, denote a C₁₋₄-alkylene, C₂₋₄-alkenylene or C₂₋₄-alkynylene bridge, which may be substituted by =O, -OR⁷, phenyl or halogen,
S¹ denotes a group of formula 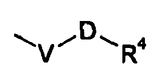
wherein V and D are as hereinbefore defined,
S¹ denotes a group of formula 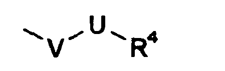
wherein V is as hereinbefore defined and U represents a C₃₋₆ - cycloalkyl or phenyl group which may be substituted by C₁₋₄-alkyl, -OR7, C₆₋₁₀-aryl or halogen,
S¹ denotes a group of formula 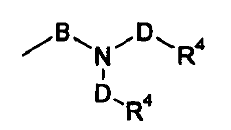
wherein B and D are as hereinbefore defined and the two groups D and the two groups R⁴ are identical or different,
S¹ denotes a group of formula 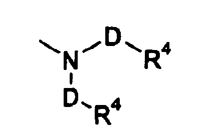
wherein D is as hereinbefore defined and the two groups D and the two groups R⁴ are identical or different,
S¹ denotes a group of formula 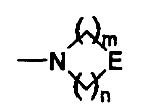
wherein E denotes oxygen, sulphur or NR⁷ (with n,m=1,2 or 3 and n+m>2), and the group is optionally substituted by halogen, = O, OR⁷, or one or more C₁C₄-alkyl groups;
S¹ denotes a group of formula 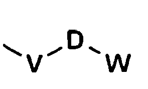
wherein V and D are as hereinbefore defined and
W may be a group of the formula 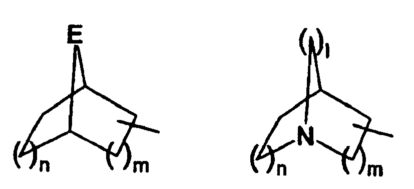 optionally substituted by halogen, =O, -OR⁷, -OCOR⁷, C₁₋₄-alkyl, C₂₋₆-alkenyl or C₂₋₆-alkynyl, wherein E denotes oxygen or N R⁷ and n,m, 1 may be 0, 1 or 2,
or W is a C-linked 5- or 6-membered heterocyclic group which contains one or more heteroatoms from the group comprising nitrogen, oxygen or sulphur and may optionally be substituted by benzyl or C₁₋₄-alkyl;
S¹ denotes a group of formula
-V-W
wherein V and W are as hereinbefore defined;
S² denotes a group of formula 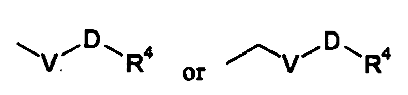 wherein V and D are as hereinbefore defined,
S⁴ denotes a group of formula 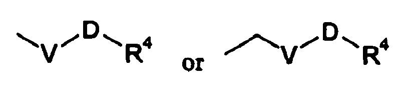 wherein V and D are as hereinbefore defined,
Q denotes a fused-on, mono- or polyunsaturated 5- or 6- membered heterocyclic ring which may contain one or more heteroatoms from the group comprising oxygen, nitrogen or sulphur, and may optionally be substituted by OR⁷, NR⁵R⁶, halogen, CN, nitro, CF₃, COOR⁷, C₁₋₄-alkyl, C₂₋₄-alkenyl or C₂₋₄-alkynyl,
S⁵ denotes a group of formula
-D-R4
wherein D is as hereinbefore defined;
R¹ denotes benzyl or phenyl, wherein the phenyl ring may optionally be mono- or polysubstituted by one or more of the groups fluorine, chlorine or bromine, C₁-C₄-alkyl, -CF₃, -CR⁷=NOR⁷ (wherein the groups R⁷ may be identical or different), -NMe₂, -NEt₂, -NO₂ or -OR⁷,
R¹ denotes phenyl, which is substituted by a group of formula 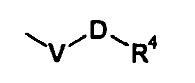 with the proviso that V is oxygen or NR⁷ and D represents a C₁₋₄-alkyl bridge,
R¹ denotes a C- or N-linked 5 or 6-membered heterocycle which contains one or more heteroatoms from the group comprising nitrogen, oxygen or sulphur and may optionally be mono- or polysubstituted by benzyl, methyl, fluorine, chlorine, bromine or hydroxy,
R¹ denotes cyclopropyl, cyclopentyl or cyclohexyl, which are optionally substituted by =O or -OR⁷,
R¹ denotes norbomane, norbornene, dicyclopropylmethyl, adamantane or noradamantane, which are optionally substituted by methyl,
R¹ denotes a group of formula 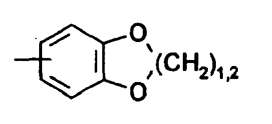
R¹ denotes -CH=CH-phenyl, wherein the phenyl ring may be substituted by methoxy or hydroxy,
R² denotes hydrogen, fluorine, chlorine or bromine, C₁₋₄-alkyloxy, C₁₋₄-alkyl or hydroxy,
R³ denotes hydrogen;
R⁴ denotes hydroxy, CN or -NR⁵R⁶;
R⁴ denotes an N-oxide of formula 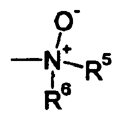
R⁵ denotes hydrogen, C₁₋₃-alkyl, benzyl or phenyl;
R⁶ denotes hydrogen, C₁-₃-alkyl, benzyl or phenyl; or
R⁵ and R⁶ together with the nitrogen atom form a saturated or unsaturated 5- or 6-membered ring, which may contain, as further heteroatoms, nitrogen, or oxygen, wherein the heterocycle may be mono- or polysubstituted by methyl;
R⁷ denotes hydrogen, C₁-C₄-alkyl, a benzyl or phenyl group, wherein the phenyl ring is optionally mono- or polysubstituted by OH or OCH₃;
optionally in the form of the racemates, the enantiomers, in the form of the diastereomers and mixtures thereof, and optionally the pharmacologically acceptable acid addition salts thereof.

2. Compounds of general formula (I) according to claim 1 wherein
X and Y denote oxygen or nitrogen, where X and Y do not both simultaneously represent oxygen or nitrogen,
Z denotes a group of formula 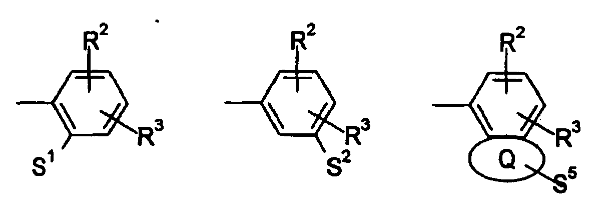
wherein
S¹ denotes a group of formula 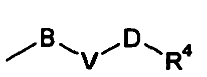
wherein V denotes oxygen, sulphur or NR⁷, B is -CH₂ and D may be one of the groups -CH₂, -CH₂-CH₂-, -CH₂-CH₂-CH₂-, -CH₂-C(CH₃)H-, -CH₂-CO or CH₂-CH₂-CO;
S¹ denotes a group of formula 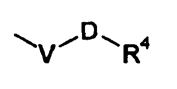
wherein V and D are as hereinbefore defined,
S¹ denotes piperazin-1-yl, 4-methyl-piperazin-1-yl or 4-benzylpiperazin-1-yl;
S¹ denotes a group of formula 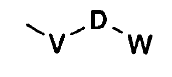
wherein V and D are as hereinbefore defined and W may represent a group of the formula 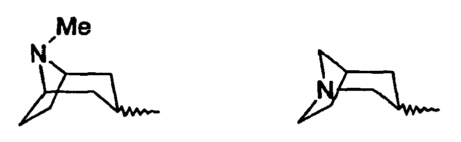 optionally substituted by C₁₋₄-alkyl,
or W is a C-linked 5-or 6-membered nitrogen heterocycle which may optionally be substituted by benzyl or C₁₋₄-alkyl;
S¹ denotes a group of formula
-V-W
wherein V and W are as hereinbefore defined;
S² denotes a group of formula
―(CH₂)_{0,1}―O―(CH₂)_{2,3}―R⁴
Q denotes a fused-on, mono- or polyunsaturated 5- or 6- membered ring which may contain a heteroatom selected from the group comprising oxygen, nitrogen or sulphur;
**S**^{**5**} denotes a group of the formula
―D―R⁴
wherein D is as hereinbefore defined;
R¹ denotes cyclopropyl, cyclopentyl, benzyl or phenyl, wherein the phenyl ring may be mono- or polysubstituted by one or more of the groups fluorine, chlorine, bromine, C₁₋₄-alkyl, -CF₃, -CMe=NOH, -NMe₂, -NO₂ or -OR⁷,
R¹ denotes phenyl which is substituted by a group of formula
―O―(CH₂)_{2,3}―R⁴
R¹ denotes furan, thiophene, pyridine or pyrrole, which may optionally be mono- or polysubstituted by methyl,
R¹ denotes norbornane, norbornene, adamantane or noradamantane,
R¹ denotes -CH=CH-phenyl, wherein the phenyl ring may be substituted by hydroxy;
R² denotes hydrogen, fluorine, chlorine, bromine, C₁₋₄-alkyloxy, C₁₋₄-alkyl or hydroxy;
R3 denotes hydrogen;
R⁴ denotes N-morpholinyl, N-pyrrolidinyl, N-piperidinyl, N-piperazinyl, 4-methyl-piperazin-1-yl or 4-benzyl-piperazin-1-yl,
R⁴ denotes CN, NR⁵R⁶ or an N-oxide of formula 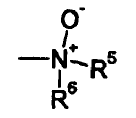
R⁵ denotes hydrogen, C₁₋₃-alkyl, benzyl or phenyl;
R⁶ denotes hydrogen, C₁₋₃-alkyl, benzyl or phenyl;
R⁷ denotes hydrogen, methyl, ethyl, propyl, isopropyl, butyl, tert.- butyl, a benzyl or phenyl group, wherein the phenyl ring is optionally mono- or polysubstituted by OH or OCH₃,
optionally in the form of their racemates, enantiomers, in the form of their diastereomers and mixtures thereof and optionally the pharmacologically acceptable acid addition salts thereof.

3. Compounds of general formula (I) according to claim 1, wherein
X and Y denote oxygen or nitrogen, where X and Y do not both simultaneously represent oxygen or nitrogen,
Z denotes a group of formula 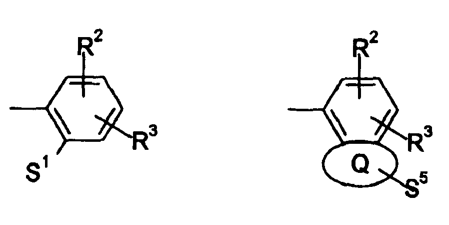
wherein
S¹ denotes a group of formula 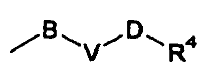
wherein V denotes oxygen, sulphur or NR⁷, B is -CH₂ and D may be one of the groups -CH₂, -CH₂-CH₂-, -CH₂-CH₂-CH₂-, -CH₂-C(CH₃)H-, -CH₂-CO or CH₂-CH₂-CO;
S¹ denotes a group of formula 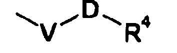
wherein V and D are as hereinbefore defined,
S¹ denotes piperazin-1-yl, 4-methyl-piperazin-1-yl or 4-benzylpiperazin-1-yl;
S¹ denotes a group of formula 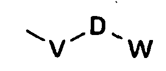
wherein V and D are as hereinbefore defined and W may represent a group of the formula 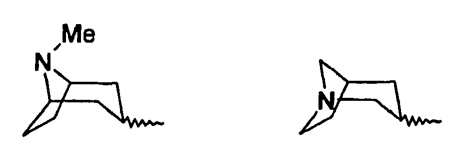 optionally substituted by C₁₋₄-alkyl,
or W is a C-linked 5-or 6-membered nitrogen heterocycle which may optionally be substituted by benzyl or C₁₋₄-alkyl;
**S**^{**1**} denotes a group of formula
-V-W
wherein V and W are as hereinbefore defined;
Q denotes a fused-on, mono- or polyunsaturated 5- or 6- membered heterocyclic ring which may contain a heteroatom selected from the group comprising oxygen, nitrogen or sulphur;
S⁵ denotes a group of the formula
-D-R⁴
wherein D is as hereinbefore defined;
R¹ denotes cyclopropyl, cyclopentyl or phenyl, wherein the phenyl ring may be mono- or polysubstituted by one or more of the groups fluorine, chlorine, bromine, C₁₋₄-alkyl, -CF₃, -CMe=NOH, -NMe₂, -NO₂ or -OR⁷,
R¹ denotes furan, thiophene, pyridine or pyrrole, which may optionally be mono- or polysubstituted by methyl,
R¹ denotes norbornane, norbornene, adamantane or noradamantane,
R¹ denotes -CH=CH-phenyl, wherein the phenyl ring may be substituted by hydroxy;
R² denotes hydrogen, fluorine, chlorine, bromine, C₁₋₄-alkyloxy, C₁₋₄-alkyl or hydroxy;
R³ denotes hydrogen;
R⁴ denotes CN, NR⁵R⁶ or an N-oxide of formula 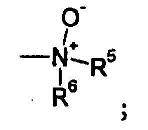
R⁴ denotes N-morpholinyl, N-pyrrolidinyl, N-piperidinyl, N-piperazinyl, 4-methyl-piperazin-1-yl or 4-benzyl-piperazin-1-yl;
R⁵ denotes hydrogen, C₁₋₃-alkyl, benzyl or phenyl;
R⁶ denotes hydrogen, C₁₋₃-alkyl, benzyl or phenyl;
R⁷ denotes hydrogen, methyl, ethyl, propyl, isopropyl, butyl, tert.- butyl, a benzyl or phenyl group, wherein the phenyl ring is optionally mono- or polysubstituted by OH or OCH₃,
optionally in the form of their racemates, enantiomers, in the form of their diastereomers and mixtures thereof and optionally the pharmacologically acceptable acid addition salts thereof.

4. Compounds of general formula (I) according to claim 1, wherein
X and Y denote oxygen or nitrogen, where X and Y do not both simultaneously represent oxygen or nitrogen,
Z denotes a group of formula 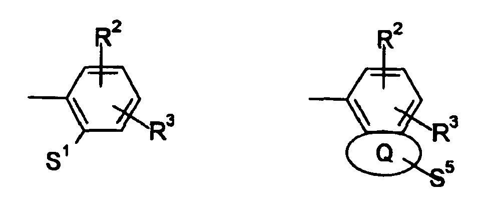
wherein
S¹ denotes a group of formula 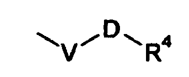
wherein V denotes oxygen, sulphur or NR⁷ and D may be one of the groups -CH₂, -CH₂-CH₂-, -CH₂-CH₂-CH₂-, -CH₂-C(CH₃)H-, -CH₂-CO or CH₂-CH₂-CO;
S¹ denotes piperazin-1-yl, 4-methyl-piperazin-1-yl or 4-benzylpiperazin-1-yl;
S¹ denotes a group of formula 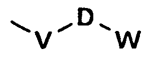
wherein V and D are as hereinbefore defined and W may represent a group of the formula 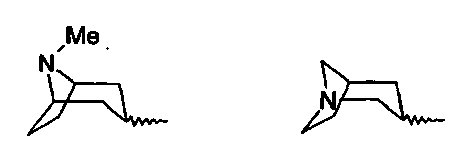 or W is a C-linked 5-or 6-membered nitrogen heterocycle which may optionally be substituted by methyl;
S¹ denotes a group of formula
-V-W
wherein V and W are as hereinbefore defined;
Q denotes a fused-on, mono- or polyunsaturated 5-membered heterocyclic ring which may contain a heteroatom selected from the group comprising oxygen or nitrogen;
S⁵ denotes a group of the formula
-D-R⁴
wherein D is as hereinbefore defined;
R¹ denotes phenyl which may optionally be mono- or polysubstituted by one or more of the groups fluorine, chlorine, bromine, methyl, ethyl, n-propyl, isopropyl, n-butyl, tert. butyl, -CF₃ or-OR⁷,
R¹ denotes furan, thiophene or pyridine, which may optionally be mono- or polysubstituted by methyl,
R² denotes hydrogen, fluorine, chlorine, bromine, methyl, methyloxy or hydroxy;
R3 denotes hydrogen;
R⁴ denotes NR⁵R⁶ or an N-oxide of formula 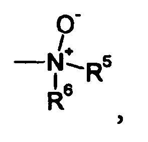
R⁴ denotes N-morpholinyl, N-pyrrolidinyl, N-piperidinyl, N-piperazinyl, 4-methyl-piperazin-1-yl or 4-benzyl-piperazin-1-yl;
R⁴ denotes N-morpholinyl, N-pyrrolidinyl, N-piperidinyl, N-piperazinyl, 4-methyl-piperazin-1-yl or 4-benzyl-piperazin-1-yl,
R⁵ denotes hydrogen, methyl, ethyl, n-propyl, isopropyl, benzyl or phenyl;
**R**^{**6**} denotes hydrogen, methyl, ethyl, n-propyl, isopropyl, benzyl or phenyl;
R⁷ denotes hydrogen, methyl or ethyl,
optionally in the form of their racemates, enantiomers, in the form of their diastereomers and mixtures thereof and optionally the pharmacologically acceptable acid addition salts thereof.

5. Compounds of general formula (I) according to claim 1, wherein X and Y denote oxygen or nitrogen, but X and Y do not both simultaneously represent oxygen or nitrogen,
Z denotes a group of formula 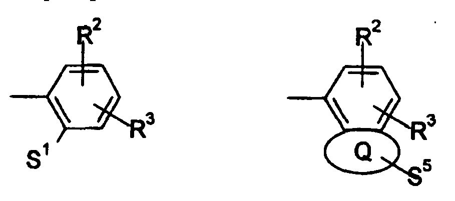 wherein
S¹ denotes a group of formula 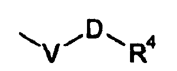
wherein V denotes oxygen and D may be one of the groups -CH₂, -CH₂-CH₂-, -CH₂-C(CH₃)H- or CH₂-CH₂-CO;
**S**^{**1**} denotes N-piperazinyl or 4-benzyl-piperazin-1-yl;
**S**^{**1**} denotes a group of formula 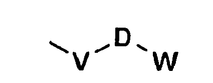
wherein V and D are as hereinbefore defined and W may represent a group of the formula 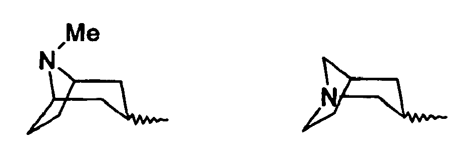 or W is a C-linked 5-or 6-membered nitrogen heterocycle which may optionally be substituted by methyl;
S¹ denotes a group of formula
V-W
wherein V and W are as hereinbefore defined;
Q denotes a fused-on, mono- or polyunsaturated 5-membered heterocyclic ring which contains oxygen as heteroatom;
S⁵ denotes a group of the formula
-D-R⁴
wherein D is as hereinbefore defined;
R¹ denotes phenyl which may be mono- or polysubstituted by one or more of the groups fluorine, chlorine, bromine, methyl, -CF₃ , hydroxy, methyloxy or ethyloxy,
R¹ denotes furan, thiophene or pyridine;
R² denotes hydrogen, fluorine, chlorine or methyl;
R³ denotes hydrogen;
R⁴ denotes NR⁵R⁶,
R⁴ denotes N-morpholinyl, N-pyrrolidinyl, N-piperidinyl or 4-methyl-piperazin-1-yl;
R⁵ denotes hydrogen, methyl, ethyl, n-propyl, isopropyl, benzyl or phenyl;
R⁶ denotes hydrogen, methyl, ethyl, n-propyl, isopropyl, benzyl or phenyl;
optionally in the form of their racemates, enantiomers, in the form of their diastereomers and mixtures thereof and optionally the pharmacologically acceptable acid addition salts thereof.

6. Compounds of general formula (I) according to clam 1, wherein
X and Y denote oxygen or nitrogen, but X and Y do not both simultaneously represent oxygen or nitrogen,
Z denotes a group of formula 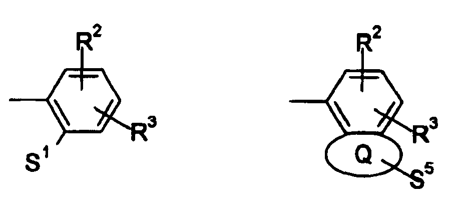 wherein
S¹ may be one of the groups -O-CH₂-CH₂-R⁴, O-CH₂-C(CH₃)H-R⁴, -O-C(CH₃)H-CH₂-R⁴ or -CH₂-CH₂-CO-R⁴;
S¹ may be 4-benzyl-piperazin-1-yl;
S¹ may be one of the groups
-O-CH₂ W or -O-W
wherein
W is a C-linked 5-or 6-membered nitrogen heterocycle which may optionally be substituted by methyl;
Q denotes a fused-on, mono- or polyunsaturated 5-membered heterocyclic ring which contains oxygen as heteroatom;
S⁵ denotes a group of the formula -CH₂-R⁴;
R¹ denotes phenyl which may be mono- or polysubstituted by one or more of the groups fluorine, chlorine, bromine, methyl, -CF₃ , hydroxy, methyloxy or ethyloxy,
R¹ denotes thiophene;
R² denotes hydrogen, fluorine, chlorine or methyl;
R3 denotes hydrogen;
R⁴ denotes NR⁵R⁶ ,
R⁴ denotes N-pyrrolidinyl or N-piperidinyl;
R⁵ denotes hydrogen, methyl, ethyl, isopropyl, benzyl or phenyl;
R⁶ denotes hydrogen, methyl, ethyl, isopropyl, benzyl or phenyl;
optionally in the form of their racemates, enantiomers, in the form of their diastereomers and mixtures thereof and optionally the pharmacologically acceptable acid addition salts thereof.

7. Use of a compound of general formula (I) according to claims 1 to 6 for preparing a pharmaceutical composition for the treatment of neurodegenerative disorders and cerebral ischaemia of various origins.

8. Pharmaceutical preparations containing as active substance one or more compounds of general formula (I) according to claims 1 to 6 or the physiologically acceptable acid addition salts thereof in conjunction with conventional excipients and/or carriers.

9. Process for preparing oxadiazole derivatives of general formula (I) 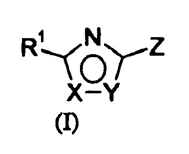 wherein
a) X denotes nitrogen and Y denotes oxygen and R¹ and Z have the meanings given in claims 1 to 6,
**characterised in that** an oxadiazole derivative of general formula (II) 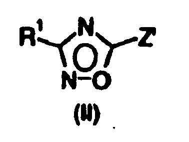
wherein R¹ is defined as in claims 1 to 6 and
Z' represents a group of formula 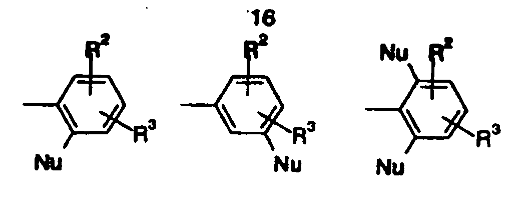
Nu represents a nucleophilic group of formula VH or B-VH wherein V, B, R² and R³ are defined as in claims 1 to 6,
is reacted under basic reaction conditions with electrophilic compounds of general formula
L-D-R⁴
wherein L denotes a leaving group such as chlorine, bromine, iodine, methanesulphonyl, trifluoromethanesulphonyl or p-toluenesulphonyl and D and R⁴ have one of the meanings given in claims 1 to 6;
b) X denotes oxygen and Y denotes nitrogen and R¹ and Z are defined as in claims 1 to 6,
**characterised in that** an aromatic hydroxylamine of general formula (III) 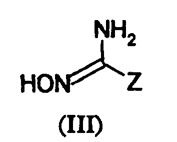 wherein
Z has the meaning given in claims 1 to 6,
is reacted with carboxylic acid derivatives of general formula 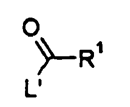
wherein L' represents chlorine, bromine or alkyloxy, for example, and R¹ is as hereinbefore defined.

10. Process for preparing oxadiazole derivatives of general formula (I), 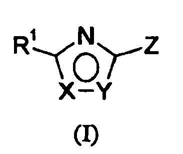 wherein X, Y, R¹ and Z are defined as in claims 1 to 6,
**characterised in that** an oxadiazole derivative of general formula (IV) 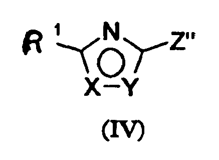 wherein Z" denotes a group of formula 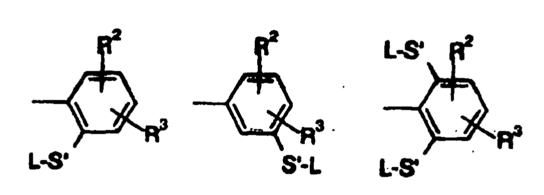 wherein S' represents a group of formula
-B-V-D- or -V-D-
L denotes a leaving group such as chlorine, bromine, iodine, methanesulphonyl, trifluoromethanesulphonyl or p-toluenemethanesulphonyl and B, V, D, R¹, R² and R³ are defined as in claims 1 to 6,
is reacted with a nucleophilic compound of formula
H- R^{**4**}
wherein R⁴ may have the meanings given in claims 1 to 6.

## Revendications

1. Dérivés d'oxadiazole de formule générale (I) 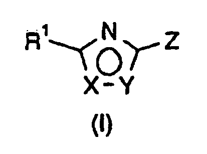
où
X et Y représentent l'oxygène ou l'azote, où X et Y ne sont pas l'un et l'autre simultanément l'oxygène ou l'azote,
Z représente un groupement de formule 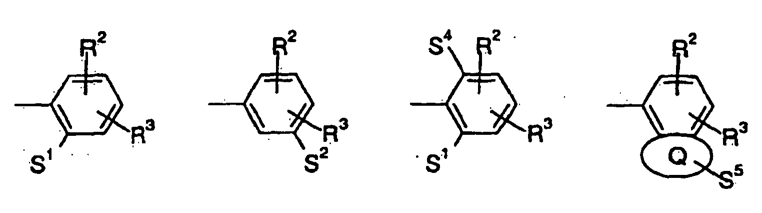
où
S¹ représente un groupement de formule 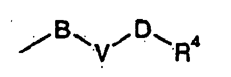
où V représente l'oxygène, le soufre ou NR⁷ et
B et D, identiques ou différents, représentent un pont C₁-C₄-alkylène, C₂-C₄-alcénylène ou C₂-C₄-alcynylène, qui peut être substitué par =O, -OR⁷, phényle ou halogène,
S¹ représente un groupement de formule 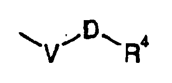
où V et D présentent la signification citée ci-dessus,
S¹ représente un groupement de formule 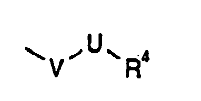
où V peut présenter la signification citée précédemment et U représente un groupe C₃-C₆-cycloalkyle ou phényle, qui peut être substitué par C₁-C₄-alkyle, -OR⁷, C₆-C₁₀-aryle ou halogène,
S¹ représente un groupement de formule 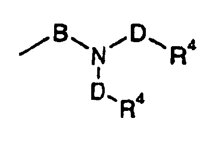
où B et D présentent la signification citée ci-dessus et les deux groupes D ainsi que les deux groupements R⁴ sont identiques ou différents,
S¹ représente un groupement de formule 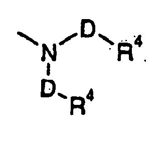
où D présente la signification citée ci-dessus et les deux groupes D ainsi que les deux groupements R⁴ sont identiques ou différents,
S¹ représente un groupement de formule 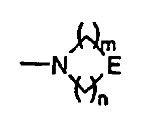
où E représente l'oxygène, le soufre ou NR⁷ (avec n, m = 1, 2 ou 3 et n+m > 2) et le groupement est éventuellement substitué par halogène, =O, -OR⁷ ou un ou plusieurs groupements C₁-C₄-alkyle ;
S¹ représente un groupement de formule 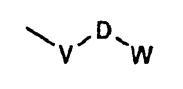
où V et D présentent la signification citée ci-dessus et W représente un groupement de formule 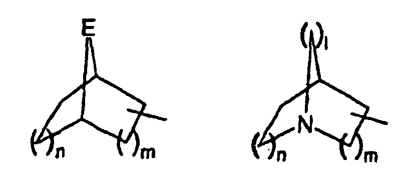
éventuellement substitué par halogène, =O, -OR⁷, -OCOR⁷, C₁-C₄-alkyle, C₂-C₆-alcényle ou C₂-C₆-alcynyle, où E représente l'oxygène ou NR⁷, n, m, 1 peuvent être = 0, 1 ou 2, ou
W est un hétérocycle à 5 ou 6 chaînons lié par C, qui contient un ou plusieurs hétéroatomes du groupe azote, oxygène ou soufre et qui peut éventuellement être substitué par benzyle ou C₁-C₄-alkyle ;
S¹ représente un groupement de formule
-V-W
où V et W présentent la signification citée ci-dessus ;
S² représente un groupement de formule 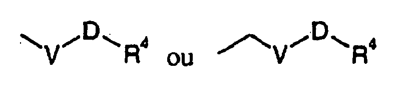
où V et D présentent la signification citée ci-dessus,
S⁴ représente un groupement de formule 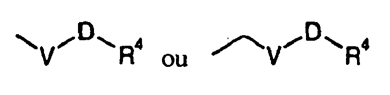
où V et D présentent la signification citée ci-dessus,
Q représente un cycle hétérocyclique à 5 ou 6 chaînons une ou plusieurs fois insaturé condensé, qui peut contenir un ou plusieurs hétéroatomes du groupe oxygène, azote ou soufre et qui peut éventuellement être substitué par OR⁷, NR⁵R⁶, halogène, CN, nitro, CF₃, COOR⁷, C₁-C₄-alkyle, C₂-C₄-alcényle ou C₂-C₄-alcynyle ;
S⁵ représente un groupement de formule
-D-R⁴
où D présente la signification citée ci-dessus ;
R¹ représente benzyle ou phényle, où le cycle phényle peut être substitué une ou plusieurs fois par un ou plusieurs des groupements fluor, chlore ou brome, -C₁-C₄-alkyle, -CF₃, -CR⁷=NOR⁷ (où les groupements R⁷ peuvent être identiques ou différents), -NMe₂, -NEt₂, -NO₂ ou -OR⁷,
R¹ représente phényle qui est substitué par un groupement de formule 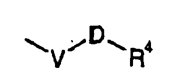
avec la condition que V soit l'oxygène ou NR⁷ et que D représente un pont C₁-C₄-alkyle,
R¹ représente un hétérocycle à 5 ou 6 chaînons lié par C ou N, qui contient un ou plusieurs hétéroatomes du groupe azote, oxygène ou soufre et qui peut éventuellement être substitué une ou plusieurs fois par benzyle, méthyle, le fluor, le chlore, le brome ou hydroxy,
R¹ représente cyclopropyle, cyclopentyle ou cyclohexyle, qui sont éventuellement substitués par =O ou -OR⁷,
R¹ représente norbornane, norbornène, dicyclopropylméthyle, adamantane ou noradamantane, qui sont éventuellement substitués par méthyle,
R¹ représente un groupement de formule 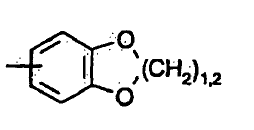
R¹ représente -CH=CH-phényle, où le cycle phényle peut être substitué par méthoxy ou hydroxy ;
R² représente l'hydrogène, le fluor, le chlore, le brome, C₁-C₄-alkyloxy, C₁-C₄-alkyle ou hydroxy ;
R³ représente l'hydrogène ;
R⁴ représente hydroxy, CN ou NR⁵R⁶ ;
R⁴ représente un N-oxyde de formule 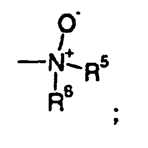
R⁵ représente l'hydrogène, C₁-C₃-alkyle, benzyle ou phényle ;
R⁶ représente l'hydrogène, C₁-C₃-alkyle, benzyle ou phényle ;
ou
R⁵ et R⁶ forment avec l'atome d'azote un cycle à 5 ou 6 chaînons saturé ou insaturé, qui peut contenir comme autres hétéroatomes de l'azote ou de l'oxygène,
où l'hétérocycle peut être substitué une ou plusieurs fois par méthyle ;
R⁷ représente l'hydrogène, C₁-C₄-alkyle, un groupement benzyle ou phényle, où le cycle phényle est éventuellement substitué une ou plusieurs fois par OH ou OCH₃, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

2. Composés de formule générale (I) selon la revendication 1, où
X et Y représentent l'oxygène ou l'azote, où X et Y ne sont pas l'un et l'autre simultanément l'oxygène ou l'azote,
Z représente un groupement de formule 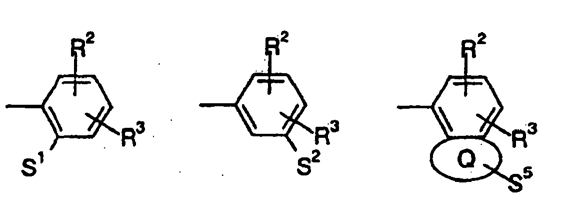
où
S¹ représente un groupement de formule 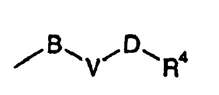
où V représente l'oxygène, le soufre ou NR⁷, B est -CH₂- et D peut être l'un des groupes -CH₂-, -CH₂-CH₂-, -CH₂-CH₂-CH₂-, -CH₂-C(CH₃)H-, -CH₂-CO-, -CH₂-CH₂-CO- ;
S¹ représente un groupement de formule 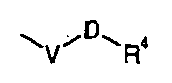
où V et D présentent la signification citée précédemment,
S¹ représente pipérazin-1-yle, 4-méthyl-pipérazin-1-yle ou 4-benzyl-pipérazin-1-yle ;
S¹ représente un groupement de formule 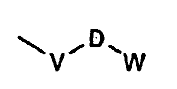
où V et D présentent la signification citée ci-dessus et W peut être un groupement de formule 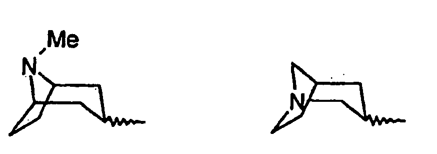
éventuellement substitué par C₁-C₄-alkyle,
ou
W est un hétérocycle azoté à 5 ou 6 chaînons lié par C, qui peut éventuellement être substitué par benzyle ou C₁-C₄-alkyle ;
S¹ représente un groupement de formule
-V-W
où V et W présentent la signification citée ci-dessus ;
S² représente un groupement de formule
-(CH₂)_{0,1}-O-(CH₂)_{2,3}-R⁴
Q représente un cycle à 5 ou 6 chaînons une ou plusieurs fois insaturé condensé, qui peut contenir un hétéroatome du groupe oxygène, azote ou soufre ;
S⁵ représente un groupement de formule
-D-R⁴
où D présente la signification citée ci-dessus ;
R¹ représente cyclopropyle, cyclopentyle, benzyle ou phényle, où le cycle phényle peut être substitué une ou plusieurs fois par un ou plusieurs des groupements fluor, chlore, brome, C₁-C₄-alkyle, -CF₃, -CMe=NOH, -NMe₂, -NO₂ ou -OR⁷,
R¹ représente phényle qui est substitué par un groupement de formule
-O-(CH₂)_{2,3}-R⁴
R¹ représente furane, thiophène, pyridine ou pyrrole, qui peuvent éventuellement être substitués une ou plusieurs fois par méthyle,
R¹ représente norbomane, norbornène, adamantane ou noradamantane,
R¹ représente -CH=CH-phényle, où le cycle phényle peut être substitué par hydroxy ;
R² représente l'hydrogène, le fluor, le chlore, le brome, C₁-C₄-alkyloxy, C₁-C₄-alkyle ou hydroxy ;
R³ représente l'hydrogène ;
R⁴ représente N-morpholinyle, N-pyrrolidinyle, N-pipéridinyle, N-pipérazinyle, 4-méthyl-pipérazin-1-yle ou 4-benzyl-pipérazin-1-yle ;
R⁴ représente CN, NR⁵R⁶ ou un N-oxyde de formule 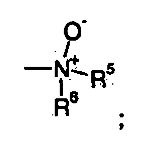
R⁵ représente l'hydrogène, C₁-C₃-alkyle, benzyle ou phényle ;
R⁶ représente l'hydrogène, C₁-C₃-alkyle, benzyle ou phényle ;
R⁷ représente l'hydrogène, méthyle, éthyle, propyle, iso-propyle, butyle, tert.-butyle, un groupement benzyle ou phényle, où le cycle phényle est éventuellement substitué une ou plusieurs fois par OH ou OCH₃,
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

3. Composés de formule générale (I) selon la revendication 1, où
X et Y représentent l'oxygène ou l'azote, où X et Y ne sont pas l'un et l'autre simultanément l'oxygène ou l'azote,
Z représente un groupement de formule 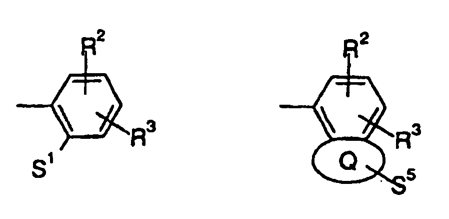
où
S¹ représente un groupement de formule 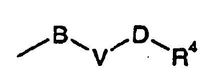
où V représente l'oxygène, le soufre ou NR⁷, B est -CH₂- et D peut être l'un des groupes -CH₂-, -CH₂-CH₂-, -CH₂-CH₂-CH₂-, -CH₂-C(CH₃)H-, -CH₂-CO-, -CH₂-CH₂-CO- ;
S¹ représente un groupement de formule 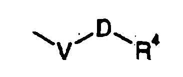
où V et D présentent la signification citée précédemment,
S¹ représente pipérazin-1-yle, 4-méthyl-pipérazin-1-yle ou 4-benzyl-pipérazin-1-yle ;
S¹ représente un groupement de formule 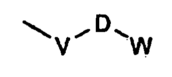
où V et D présentent la signification citée ci-dessus et W peut être un groupement de formule 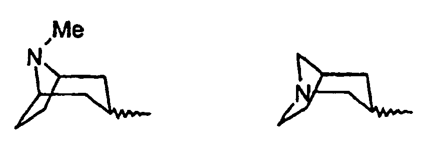
éventuellement substitué par C₁-C₄-alkyle,
ou
W est un hétérocycle azoté à 5 ou 6 chaînons lié par C, qui peut éventuellement être substitué par benzyle ou C₁-C₄-alkyle ;
S¹ représente un groupement de formule
-V-W
où V et W présentent les significations citées ci-dessus ;
Q représente un cycle hétérocyclique à 5 ou 6 chaînons une ou plusieurs fois insaturé condensé, qui peut contenir un hétéroatome du groupe oxygène, azote ou soufre ;
S⁵ représente un groupement de formule
-D-R⁴
où D présente la signification citée ci-dessus ;
R¹ représente cyclopropyle, cyclopentyle ou phényle, où le cycle phényle peut être substitué une ou plusieurs fois par un ou plusieurs des groupements fluor, chlore, brome, C₁-C₄-alkyle, -CF₃, -CMe=NOH, -NMe₂, -NO₂ ou -OR⁷,
R¹ représente furane, thiophène, pyridine ou pyrrole, qui peuvent éventuellement être substitués une ou plusieurs fois par méthyle,
R¹ représente norbomane, norbornène, adamantane ou noradamantane,
R¹ représente -CH=CH-phényle, où le cycle phényle peut être substitué par hydroxy ;
R² représente l'hydrogène, le fluor, le chlore, le brome, C₁-C₄-alkyloxy, C₁-C₄-alkyle ou hydroxy ;
R³ représente l'hydrogène ;
R⁴ représente CN, NR⁵R⁶ ou un N-oxyde de formule 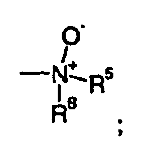
R⁴ représente N-morpholinyle, N-pyrrolidinyle, N-pipéridinyle, N-pipérazinyle, 4-méthyl-pipérazin-1-yle ou 4-benzyl-pipérazin-1-yle ;
R⁵ représente l'hydrogène, C₁-C₃-alkyle, benzyle ou phényle ;
R⁶ représente l'hydrogène, C₁-C₃-alkyle, benzyle ou phényle ;
R⁷ représente l'hydrogène, méthyle, éthyle, propyle, iso-propyle, butyle, tert.-butyle, un groupement benzyle ou phényle, où le cycle phényle est éventuellement substitué une ou plusieurs fois par OH ou OCH₃,
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

4. Composés de formule générale (I) selon la revendication 1, où
X et Y représentent l'oxygène ou l'azote, où X et Y ne sont pas l'un et l'autre simultanément l'oxygène ou l'azote,
Z représente un groupement de formule 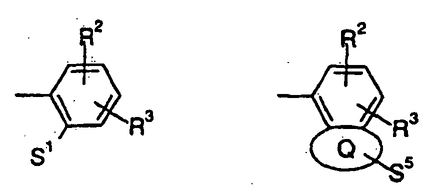
où
S¹ représente un groupement de formule 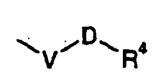
où V représente l'oxygène, le soufre ou NR⁷ et D peut être l'un des groupes -CH₂-, -CH₂-CH₂-, -CH₂-CH₂-CH₂-, -CH₂-C(CH₃)H-, -CH₂-CO-, -CH₂-CH₂-CO- ;
S¹ représente pipérazin-1-yle, 4-méthyl-pipérazin-1-yle ou 4-benzyl-pipérazin-1-yle ;
S¹ représente un groupement de formule 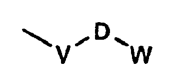
où V et D présentent la signification citée ci-dessus et W est un groupement de formule 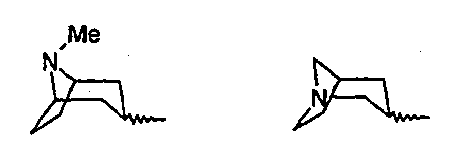
ou
W est un hétérocycle azoté à 5 ou 6 chaînons lié par C, qui peut éventuellement être substitué par méthyle ;
S¹ représente un groupement de formule
-V-W
où V et W présentent les significations citées ci-dessus ;
Q représente un cycle hétérocyclique à 5 chaînons une ou plusieurs fois insaturé condensé, qui peut contenir un hétéroatome du groupe oxygène ou azote ;
S⁵ représente un groupement de formule
-D-R⁴
où D présente la signification citée ci-dessus ;
R¹ représente phényle, qui peut éventuellement être substitué une ou plusieurs fois par un ou plusieurs des groupements fluor, chlore, brome, méthyle, éthyle, n-propyle, i-propyle, n-butyle, tert.-butyle, -CF₃ ou -OR⁷,
R¹ représente furane, thiophène ou pyridine, qui peuvent éventuellement être substitués une ou plusieurs fois par méthyle,
R² représente l'hydrogène, le fluor, le chlore, le brome, méthyle, méthyloxy ou hydroxy ;
R³ représente l'hydrogène ;
R⁴ représente NR⁵R⁶ ou un N-oxyde de formule 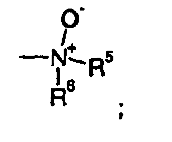
R⁴ représente N-morpholinyle, N-pyrrolidinyle, N-pipéridinyle, N-pipérazinyle, 4-méthyl-pipérazin-1-yle ou 4-benzyl-pipérazin-1-yle ;
R⁵ représente l'hydrogène, méthyle, éthyle, n-propyle, i-propyle, benzyle ou phényle ;
R⁶ représente l'hydrogène, méthyle, éthyle, n-propyle, i-propyle, benzyle ou phényle ;
R⁷ représente l'hydrogène, méthyle ou éthyle,
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

5. Composés de formule générale (I) selon la revendication 1, où
X et Y représentent l'oxygène ou l'azote, où X et Y ne sont pas l'un et l'autre simultanément l'oxygène ou l'azote,
Z représente un groupement de formule 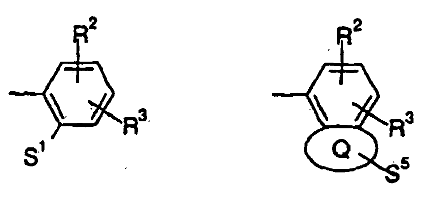
où
S¹ représente un groupement de formule 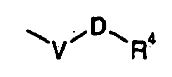
où V représente l'oxygène et D peut être l'un des groupes -CH₂-, -CH₂-CH₂-, -CH₂-C(CH₃)H-, -CH₂-CH₂-CO- ;
S¹ représente N-pipérazinyle, 4-benzyl-pipérazin-1-yle ;
S¹ représente un groupement de formule 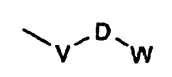
où V et D présentent la signification citée ci-dessus et W est un groupement de formule 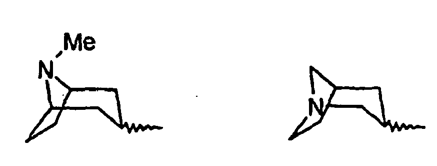
ou
W est un hétérocycle azoté à 5 ou 6 chaînons lié par C, qui peut éventuellement être substitué par méthyle ;
S¹ représente un groupement de formule
-V-W
où V et W présentent les significations citées ci-dessus ;
Q représente un cycle hétérocyclique à 5 chaînons une ou plusieurs fois insaturé condensé, qui contient l'oxygène comme hétéroatome ;
S⁵ représente un groupement de formule
-D-R⁴
où D présente la signification citée ci-dessus ;
R¹ représente phényle, qui peut être substitué une ou plusieurs fois par un ou plusieurs des groupements fluor, chlore, brome, méthyle, -CF₃, hydroxy, méthyloxy ou éthyloxy,
R¹ représente furane, thiophène ou pyridine ;
R² représente l'hydrogène, le fluor, le chlore ou méthyle ;
R³ représente l'hydrogène ;
R⁴ représente NR⁵R⁶,
R⁴ représente N-morpholinyle, N-pyrrolidinyle, N-pipéridinyle ou 4-méthyl-pipérazin-1-yle ;
R⁵ représente l'hydrogène, méthyle, éthyle, n-propyle, i-propyle, benzyle ou phényle ;
R⁶ représente l'hydrogène, méthyle, éthyle, n-propyle, i-propyle, benzyle ou phényle ;
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

6. Composés de formule générale (I) selon la revendication 1, où
X et Y représentent l'oxygène ou l'azote, où X et Y ne sont pas l'un et l'autre simultanément l'oxygène ou l'azote,
Z représente un groupement de formule 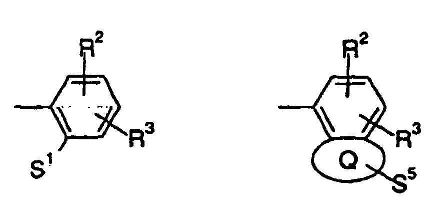
où
S¹ peut être l'un des groupements
-O-CH₂-CH₂-R⁴, -O-CH₂-C(CH₃)H-R⁴, -O-C(CH₃)H-CH₂-R⁴ ou -CH₂-CH₂-CO-R⁴;
S¹ représente 4-benzyl-pipérazin-1-yle ;
S¹ peut être l'un des groupements
-O-CH₂-W ou -O-W, où
W est un hétérocycle azoté à 5 ou 6 chaînons lié par C, qui peut éventuellement être substitué par méthyle ;
Q représente un cycle hétérocyclique à 5 chaînons une ou plusieurs fois insaturé condensé, qui contient l'oxygène comme hétéroatome ;
S⁵ représente un groupement de formule -CH₂-R⁴ ;
R¹ représente phényle, qui peut être substitué une ou plusieurs fois par un ou plusieurs des groupements fluor, chlore, brome, méthyle, -CF₃, hydroxy, méthyloxy ou éthyloxy,
R¹ représente thiophène ;
R² représente l'hydrogène, le fluor, le chlore ou méthyle ;
R³ représente l' hydrogène ;
R⁴ représente NR⁵R⁶,
R⁴ représente N-pyrrolidinyle ou N-pipéridinyle ;
R⁵ représente l'hydrogène, méthyle, éthyle, i-propyle, benzyle ou phényle ;
R⁶ représente l'hydrogène, méthyle, éthyle, i-propyle, benzyle ou phényle ;
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

7. Utilisation d'un composé de formule générale (I) selon les revendications 1 à 6 pour la production d'un médicament pour le traitement des maladies neurodégénératives ainsi que des ischémies cérébrales de genèse diverse.

8. Préparations pharmaceutiques contenant comme principe actif un ou plusieurs composés de formule générale (I) selon les revendications 1 à 6 ou leurs sels d'addition d'acide physiologiquement acceptables en combinaison avec des adjuvants et/ou supports courants.

9. Procédé de production de dérivés d'oxadiazole de formule générale (I) 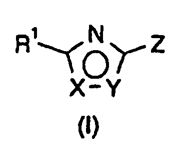
où
a) X représente l'azote et Y représente l'oxygène et R¹ et Z présentent la signification citée dans les revendications 1 à 6,
**caractérisé en ce qu'**un dérivé d'oxadiazole de formule générale (II) 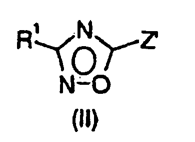
où R¹ a la signification citée dans les revendications 1 à 6 et
Z' représente un groupement de formule 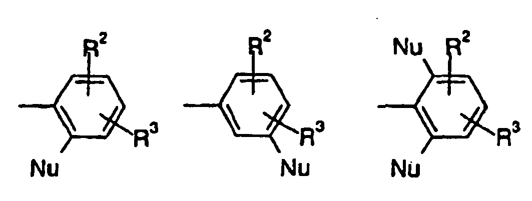
Nu représente un groupe nucléophile de formule VH ou B-VH où V, B, R² et R³ présentent la signification citée dans les revendications 1 à 6,
est mis à réagir dans des conditions réactionnelles basiques avec des électrophiles de formule générale
L-D-R⁴
où L représente un groupe partant comme par exemple le chlore, le brome, l'iode, méthanesulfonyle, trifluorométhanesulfonyle ou encore p-toluènesulfonyle et D et R⁴ présentent l'une des significations citées dans les revendications 1 à 6,
b) X représente l'oxygène et Y représente l'azote et R¹ et Z présentent la signification citée dans les revendications 1 à 6,
**caractérisé en ce qu'**une hydroxylamine aromatique de formule générale (III) 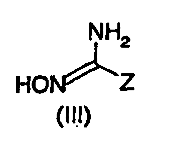
où
Z présente la signification exposée dans les revendications 1 à 6,
est mise à réagir avec des dérivés d'acide carboxylique de formule générale 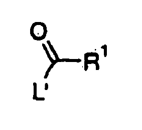
où L' représente par exemple le chlore, le brome ou alkyloxy et R¹ présente les significations citées précédemment.

10. Procédé de production de dérivés d'oxadiazole de formule générale (I) 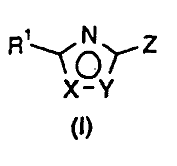
où X, Y, R¹ et Z présentent la signification citée dans les revendications 1 à 6,
**caractérisé en ce qu'**un dérivé d'oxadiazole de formule générale (IV) 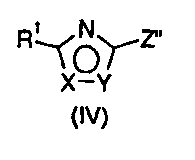
où Z" représente un groupement de formule 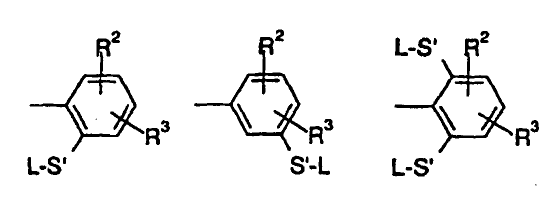
où S' représente un groupement de formule
-B-V-D- ou -V-D-,
L représente un groupe partant comme par exemple le chlore, le brome, l'iode, méthanesulfonyle, trifluorométhanesulfonyle ou p-toluèneméthanesulfonyle et B, V, D, R¹, R² et R³ présentent la signification citée dans les revendications 1 à 6, est mis à réagir avec un nucléophile de formule
H-R⁴
où R⁴ peut présenter les significations indiquées dans les revendications 1 à 6.
